# EUROPEAN PATENT SPECIFICATION

(11) **EP 3 779 921 B1**
(45) Date of publication and mention of the grant of the patent: **30.04.2025**
(21) Application number: 19776339.4
(22) Date of filing: 15.03.2019
(51) Int. Cl.: G08G 1/16, A61B 5/18, B60W 40/08, G06T 3/00, G06T 7/00, B60W 60/00, B60W 50/00

(54) **INFORMATION PROCESSING DEVICE, MOVING APPARATUS, METHOD, AND PROGRAM**
INFORMATIONSVERARBEITUNGSVORRICHTUNG, BEWEGLICHE EINRICHTUNG, VERFAHREN UND PROGRAMM
DISPOSITIF DE TRAITEMENT D'INFORMATIONS, APPAREIL MOBILE, PROCÉDÉ ET PROGRAMME

(30) Priority: 30.03.2018 JP 2018066914
(43) Date of publication of application: 17.02.2021
(73) Proprietor: Sony Semiconductor Solutions Corporation, Atsugi-shi, Kanagawa 243-0014 (JP)
(72) Inventor: OBA, Eiji, Atugi-shi, Kanagawa 243-0014 (JP); KADOSHITA, Kohei, Atugi-shi, Kanagawa 243-0014 (JP)
(74) Representative: Witte, Weller & Partner Patentanwälte mbB
(86) International application number: PCT/JP2019/010776
(87) International publication number: WO 2019/188398

(56) References cited:
- WO-A1-2017/195405
- JP-A- 2008 123 092
- JP-A- 2008 213 595
- JP-A- 2017 030 555
- US-A- 5 422 690
- US-A1- 2004 044 293
- US-A1- 2013 116 856
- US-A1- 2017 028 995
- US-A1- 2017 355 377

## Description

### TECHNICAL FIELD

The present invention relates to an information processing apparatus, a moving device, a method, and a program. More specifically, the present invention relates to an information processing apparatus, a moving device, a method, and a program that acquire driver's state information of an automobile and perform optimal control depending on the driver's state.

### BACKGROUND ART

In recent years, a large number of accidents have been caused by deterioration in attention and sleepiness of the driver, sleepiness caused by the apnea syndrome, sudden diseases such as heart attack, cerebral infarction, or the like. According to this situation, efforts to prevent these accidents by monitoring the driver's state are made. In particular, it is examined to install a monitoring system to a large vehicle that has a large possibility of causing a serious accident.

As related art disclosing a system that monitors the driver's state, for example, includes the following documents.

Patent Document 1 (Japanese Patent Application Laid-Open No. 2005-168908) discloses a system that regularly observes a vital signal of the driver, transmits an observation result to an analysis device, determines whether or not an abnormality occurs by the analysis device, and displays warning information on a display unit in a driver's seat at the time when the abnormality is detected.

Furthermore, Patent Document 2 (Japanese Patent Application Laid-Open No. 2008-234009) discloses a configuration that uses body information such as a body temperature, a blood pressure, a heart rate, brain wave information, a weight, a blood sugar level, body fat, and a height for health management of the driver.

However, there is a problem in that the configurations disclosed in the related arts is not able to cope with a sudden physical abnormality that occurs at the time of driving even though the configurations can be used to regularly recognize a health state of the driver.

Patent Document 3 (United States Patent Application Publication, Pub. No.: US 2004/0044293 A1) discloses a system and method of monitoring the alertness or wakefulness of a driver. The monitored parameters include cardiac, respiratory and movement parameters. Sensors are located in various locations of the driver side section to detect the vigilance of a driver. These sensors include pressure sensors embedded in the seat and pedals, and a head band for monitoring EEG, EMG and EOG signals.

Patent Document 4 (United States Patent, Patent No. 5,422,690) discloses a fitness impairment tester implementing a self-àdministered screening test to determine whether a subject is physically impaired.

US 2017/0355377 A1 discloses a system for analyzing driver fitness to drive with consideration given to any of various conditions such as driver drowsiness, fatigue, and anxiety. The system includes a processing hardware unit, and a non-transitory computer-readable storage device comprising various modules to determine if the driver is impaired. The modules include an input interface module that, when executed by the processing unit, receives sensor data indicating a present driver health factor. A database module includes pre-established driver-profile data particular to a driver. And the modules include an activity module that, when executed by the processing unit, obtains the driver-profile data and the sensor data, and determines based on the present health factor and the pre-established driver-profile data, fitness of the driver to drive.

### CITATION LIST

### PATENT DOCUMENT

Patent Document 1: Japanese Patent Application Laid-Open No. 2005-168908
Patent Document 2: Japanese Patent Application Laid-Open No. 2008-234009
Patent Document 3: United States Patent Application Publication, Pub. No.: US 2004/0044293 A1
Patent Document 4: United States Patent, Patent No. 5,422,690

### SUMMARY OF THE INVENTION

### PROBLEMS TO BE SOLVED BY THE INVENTION

The present invention has been made, for example, in view of the above problems. An object of the present invention is to provide an information processing apparatus, a moving device, a method, and a program that can acquire a state of a driver of an automobile, immediately determine occurrence of an abnormality, and perform optimum determination, control, and procedure.

### SOLUTIONS TO PROBLEMS

A first aspect of the present invention is an information processing apparatus as claimed in claim 1.

Moreover, a second aspect of the present invention is
a moving device as claimed in claim 8.

Moreover, a third aspect of the present invention is
an information processing method executed by an information processing apparatus as claimed in claim

Moreover, a fourth aspect of the present invention is
an information processing method executed by a moving device as claimed in claim 12.

Moreover, a fifth aspect of the present invention is
a computer program as claimed in claim 13.

Note that, for example, a program according to the present invention can be provided by a storage medium and a communication medium which provide the program in a computer-readable format to an information processing apparatus and a computer system which can execute various program codes. The information processing apparatus and the computer system can realize processing according to the program by providing such programs in the computer-readable format.

Other purpose, characteristics, and advantages of the present invention would be obvious by the detailed description based on the embodiments of the present invention as described later and the attached drawings. Note that, the system herein is a logical group configuration of a plurality of devices, and the devices of the configuration are not limited to being housed in the same casing.

### EFFECTS OF THE INVENTION

According to a configuration of one embodiment of the present invention, a configuration that receives driver's biological information and evaluates a wakefulness degree of the driver is realized.

Specifically, for example, a data processing unit that receives the driver's biological information and evaluates the wakefulness degree of the driver is included. The data processing unit analyzes a behavior of at least one of eyeballs or pupils of the driver and evaluates the driver's wakefulness degree by applying the behavior analysis result and a wakefulness state evaluation dictionary, which has been generated in advance, specific for the driver. The data processing unit evaluates the wakefulness degree of the driver by using the wakefulness state evaluation dictionary specific for the driver generated as a result of learning processing based on log data of the driver's biological information. The data processing unit further executes processing for estimating a return time until the driver can start safety manual driving.

With this configuration, the configuration that receives the driver's biological information and evaluates the wakefulness degree of the driver is realized. Moreover, it is possible to estimate an activity amount in the brain and monitor a temporal change of the activity amount.

Note that the effects described herein are only exemplary and not limited to these. Furthermore, there may be an additional effect.

### BRIEF DESCRIPTION OF DRAWINGS

Fig. 1 is a diagram illustrating an exemplary configuration of a moving device according to the present invention.
Fig. 2 is a diagram for explaining an example of data displayed on a display unit of the moving device according to the present invention.
Fig. 3 is a diagram for explaining an exemplary configuration of the moving device according to the present invention.
Fig. 4 is a diagram for explaining an exemplary configuration of the moving device according to the present invention.
Fig. 5 is a diagram for explaining an exemplary sensor configuration of the moving device according to the present invention.
Fig. 6 is a diagram illustrating a flowchart for explaining a generation sequence of a wakefulness state evaluation dictionary.
Fig. 7 is a diagram illustrating a flowchart for explaining the generation sequence of the wakefulness state evaluation dictionary.
Fig. 8 is a diagram illustrating an example of analysis data of a line-of-sight behavior of a driver.
Fig. 9 is a diagram illustrating an example of the analysis data of the line-of-sight behavior of the driver.
Fig. 10 is a diagram for explaining an exemplary data structure of the wakefulness state evaluation dictionary.
Fig. 11 is a diagram for explaining an exemplary data structure of the wakefulness state evaluation dictionary.
Fig. 12 is a diagram for explaining an exemplary data structure of the wakefulness state evaluation dictionary.
Fig. 13 is a diagram for explaining an exemplary data structure of the wakefulness state evaluation dictionary.
Fig. 14 is a diagram illustrating a flowchart for explaining a control sequence based on a driver's wakefulness state evaluation.
Fig. 15 is a diagram illustrating a flowchart for explaining a learning processing sequence performed by an information processing apparatus according to the present invention.
Fig. 16 is a diagram illustrating a flowchart for explaining the control sequence based on the driver's wakefulness state evaluation.
Fig. 17 is a diagram for explaining a distribution example of a plurality of pieces of relationship information (observation plot) of an observable evaluation value corresponding to an observation value and a return delay time (= manual driving returnable time) and a return success rate.
Fig. 18 is a diagram for explaining the manual driving returnable time in accordance with a type of processing (secondary task) executed by the driver in an automatic driving mode.
Fig. 19 is a diagram illustrating a flowchart for explaining the learning processing sequence performed by the information processing apparatus according to the present invention.
Fig. 20 is a diagram for explaining an exemplary hardware configuration of the information processing apparatus.

### MODE FOR CARRYING OUT THE INVENTION

Hereinafter, an information processing apparatus, a moving device, a method, and a program according to the present invention will be described in detail with reference to the drawings. Note that the description will be made according to the following items.
1. Outline of Configuration And Processing of Moving Device And Information Processing Apparatus
2. Specific Configuration and Processing Example of Moving Device
3. Outline of Generation Processing and Usage Processing of Wakefulness State Evaluation Dictionary And Exemplary Data Structure of Dictionary
4. (First Embodiment) Embodiment for Performing Control Based on Driver Monitoring (Control Processing Example in Case of SAE Definition Levels 1 and 2)
5. (Second Embodiment) Embodiment for Performing Control Based on Driver Monitoring (Control Processing Example in Case of SAE Definition Level 3 or Higher)
6. Exemplary Configuration of Information Processing Apparatus
7. Summary of Configuration According to Present Invention

### [1. Outline of Configuration And Processing of Moving Device And Information Processing Apparatus]

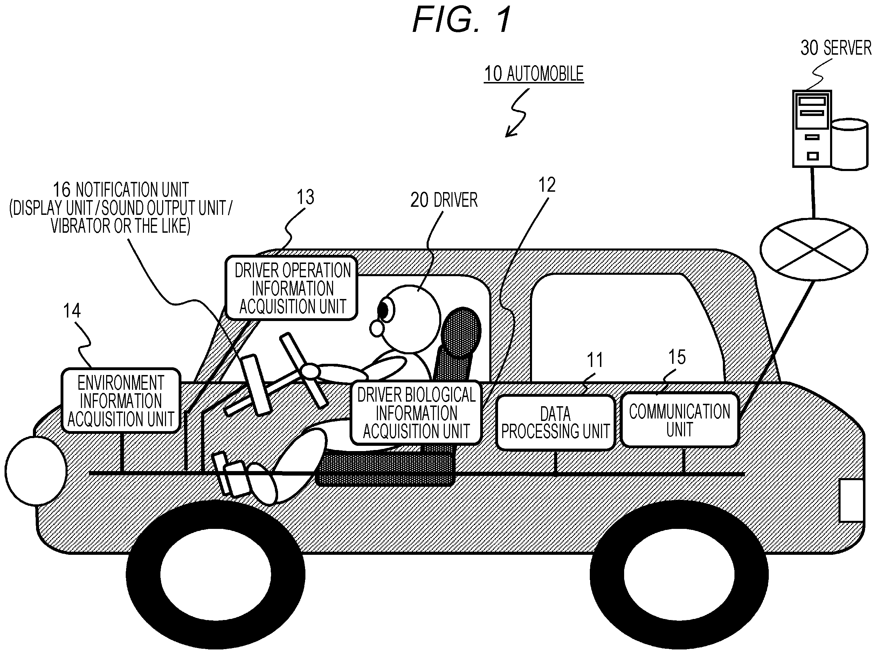

First, an outline of configurations and processing of a moving device and an information processing apparatus will be described with reference to Fig. 1 and subsequent drawings.

The moving device according to the present invention is, for example, an automobile that can travel while switching automatic driving and manual driving.

In a case where it is necessary to switch an automatic driving mode to a manual driving mode in such an automobile, it is requested to make a driver (driver) start the manual driving.

However, there are various states of the driver during the automatic driving. For example, there is a case where the driver is watching ahead of the automobile, as at the time of driving, only as releasing hands from a steering wheel, a case where the driver is reading a book, or a case where the driver is falling asleep. Moreover, there is a possibility that sudden diseases such as sleepiness due to apnea syndrome, heart attack, cerebral infarction, or the like occurs.

A wakefulness degree (wakefulness degree (consciousness level)) of the driver differs on the basis of the difference in the states.

For example, when the driver falls asleep, the wakefulness degree of the driver is deteriorated. That is, the wakefulness degree (consciousness level) is deteriorated. In such a state where the wakefulness degree is deteriorated, it is not possible to perform normal manual driving. If the driving mode is switched to the manual driving mode in such a state, there is a possibility that an accident occurs at the worst.

In order to ensure driving safety, it is necessary to cause the driver to start manual driving with clear awareness. The moving device or the information processing apparatus that can be mounted on the moving device according to the present invention acquires biological information of the driver and operation information of the driver, determines whether or not manual driving can be safely started on the basis of the acquired information, and performs control to start the manual driving on the basis of the determination result.

Configurations and processing of the moving device according to the present invention and the information processing apparatus attachable to the moving device will be described with reference to Fig. 1 and subsequent drawings.

Fig. 1 is a diagram illustrating an exemplary configuration of an automobile 10 that is an example of the moving device according to the present invention.

The information processing apparatus according to the present invention is attached to the automobile 10 illustrated in Fig. 1.

The automobile 10 illustrated in Fig. 1 is an automobile that can be driven in two driving modes including a manual driving mode and an automatic driving mode.

In the manual driving mode, traveling is performed on the basis of an operation by a driver (driver) 20, that is, a steering wheel (steering) operation, an operation on an accelerator, a brake, or the like.

On the other hand, in the automatic driving mode, the operation by the driver (driver) 20 is unnecessary or is partially unnecessary, and for example, driving based on sensor information such as a position sensor, other surrounding information detection sensor, or the like is performed.

The position sensor is, for example, a GPS receiver or the like, and the surrounding information detection sensor is, for example, a camera, an ultrasonic wave sensor, a radar, Light Detection and Ranging and Laser Imaging Detection and Ranging (LiDAR), a sonar, or the like.

Note that Fig. 1 is a diagram for explaining an outline of the present invention and schematically illustrates main components. The detailed configuration will be described later.

As illustrated in Fig. 1, the automobile 10 includes a data processing unit 11, a driver biological information acquisition unit 12, a driver operation information acquisition unit 13, an environment information acquisition unit 14, a communication unit 15, and a notification unit 16.

The driver biological information acquisition unit 12 acquires biological information of the driver as information used to determine the driver's state. The biological information to be acquired is, for example, at least any one of pieces of biological information such as a Percent of Eyelid Closure (PERCLOS) related index, a heart rate, a pulse rate, a blood flow, breathing, psychosomatic correlation, visual stimulation, a brain wave, a sweating state, a head posture and behavior, eyes, watch, blink, saccade, microsaccade, visual fixation, drift, gaze, pupil response of iris, sleep depth estimated from the heart rate and the breathing, an accumulated cumulative fatigue level, a sleepiness index, a fatigue index, an eyeball search frequency of visual events, visual fixation delay characteristics, visual fixation maintenance time, or the like.

The driver operation information acquisition unit 13 acquires, for example, the operation information of the driver that is information from another aspect used to determine the driver's state. Specifically, for example, the operation information regarding each operation unit (steering wheel, accelerator, brake, or the like) that can be operated by the driver is acquired.

The environment information acquisition unit 14 acquires traveling environment information of the automobile 10. For example, image information regarding the front, rear, left, and right sides of the automobile, position information by a GPS, surrounding obstacle information from the Light Detection and Ranging and Laser Imaging Detection and Ranging (LiDAR), the sonar, or the like.

The data processing unit 11 inputs driver's information acquired by the driver biological information acquisition unit 12 and the driver operation information acquisition unit 13 and environment information acquired by the environment information acquisition unit 14 and calculates a safety index value indicating whether or not a driver in an automobile during the automatic driving can perform safety manual driving or whether or not the driver during the manual driving is performing the safety driving.

Moreover, for example, in a case where it is necessary to switch the automatic driving mode to the manual driving mode, processing for issuing a notification to switch to the manual driving mode via the notification unit 16 is executed.

Furthermore, the data processing unit 11 analyzes a behavior of at least one of eyeballs or pupils of the driver as the biological information of the driver and evaluates a driver's wakefulness degree by applying the behavior analysis result and a wakefulness state evaluation dictionary, which has been generated in advance, specific for the driver. Details of the wakefulness state evaluation dictionary will be described later.

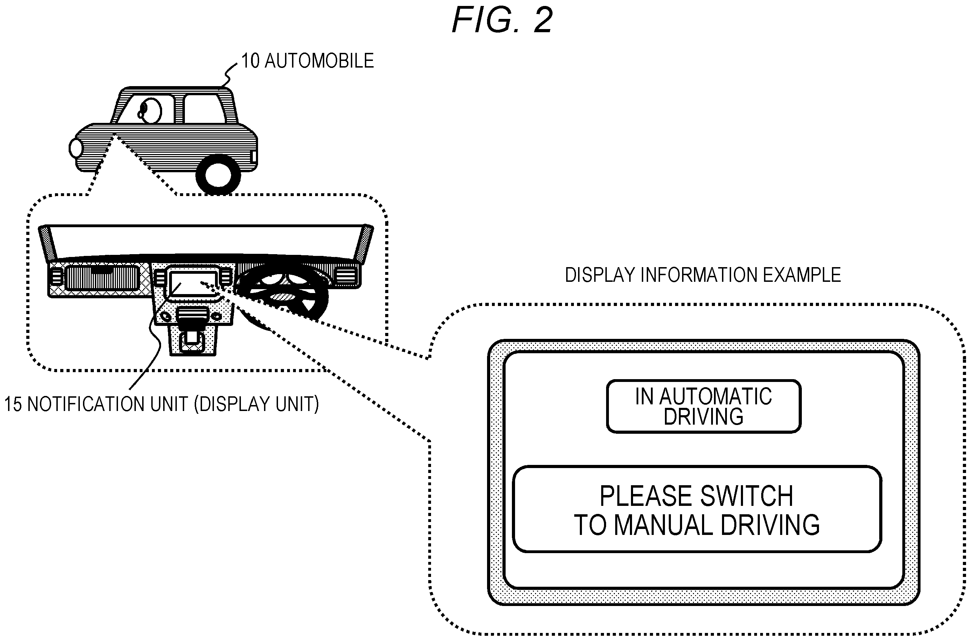

The notification unit 16 includes a display unit, a sound output unit, or a vibrator in a steering wheel or a seat that issues this notification. An example of warning display on the display unit included in the notification unit 16 is illustrated in Fig. 2.

As illustrated in Fig. 2, a display unit 30 makes displays as follows.
Driving mode information = "in automatic driving"
warning display = "Please switch to manual driving"

In a display region of the driving mode information, "during automatic driving" is displayed at the time of the automatic driving mode, and "during manual driving" is displayed at the time of the manual driving mode.

A display region of the warning display information is a display region that makes displays as follows while the automatic driving is performed in the automatic driving mode.
"Please switch to manual driving"

Note that, as illustrated in Fig. 1, the automobile 10 has a configuration that can communicate with a server 30 via the communication unit 15. The server 30 can execute a part of processing of the data processing unit 11, for example, learning processing or the like.

### [2. Specific Configuration And Processing Example of Moving Device]

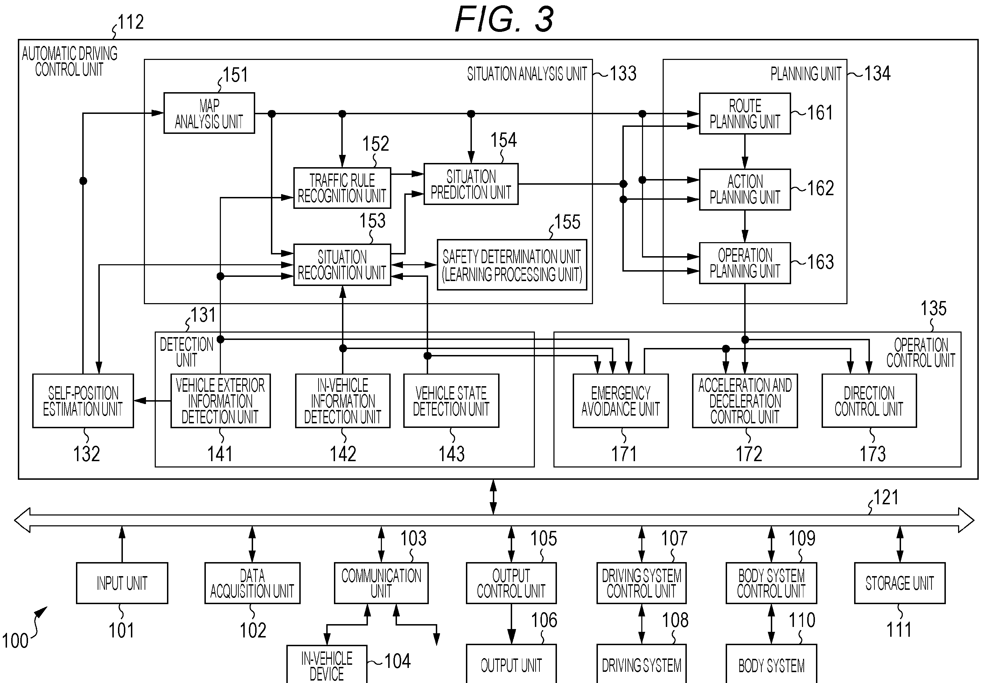

Next, a specific configuration and a processing example of a moving device 10 according to the present invention will be described with reference to Fig. 3 and subsequent drawings.

Fig. 3 is an exemplary configuration of the moving device 100. Note that, hereinafter, in a case where a vehicle in which the moving device 100 is provided is distinguished from other vehicle, the moving device 100 is referred to as own vehicle.

The moving device 100 includes an input unit 101, a data acquisition unit 102, a communication unit 103, an in-vehicle device 104, an output control unit 105, an output unit 106, a driving system control unit 107, a driving system 108, a body system control unit 109, a body system 110, a storage unit 111, and an automatic driving control unit 112.

The input unit 101, the data acquisition unit 102, the communication unit 103, the output control unit 105, the driving system control unit 107, the body system control unit 109, the storage unit 111, and the automatic driving control unit 112 are mutually connected via a communication network 121. The communication network 121 includes, for example, an in-vehicle communication network compliant with an optional standard, for example, a Controller Area Network (CAN), a Local Interconnect Network (LIN), a Local Area Network (LAN), or the FlexRay (registered trademark), a bus, or the like. Note that each unit of the moving device 100 may be directly connected without the communication network 121.

Note that, hereinafter, in a case where each unit of the moving device 100 performs communication via the communication network 121, description of the communication network 121 is omitted. For example, in a case where the input unit 101 and the automatic driving control unit 112 communicate with each other via the communication network 121, it is simply described that the input unit 101 and the automatic driving control unit 112 communicate with each other.

The input unit 101 includes a device used by an occupant to input various data, instructions, or the like. For example, the input unit 101 includes an operation device such as a touch panel, a button, a microphone, a switch, or a lever and an operation device that can perform input by a method other than a manual operation using sounds, gestures, or the like. Furthermore, for example, the input unit 101 may be an external connection device such as a remote control device that uses infrared rays and other radio waves or a mobile device or a wearable device that is compatible with the operation of the moving device 100. The input unit 101 generates an input signal on the basis of data, instructions, or the like input by the occupant and supplies the input signal to each unit of the moving device 100.

The data acquisition unit 102 includes various sensors or the like that acquire data used for the processing of the moving device 100 and supplies the acquired data to each unit of the moving device 100.

For example, the data acquisition unit 102 includes various sensors that detect a state of the own vehicle or the like. Specifically, for example, the data acquisition unit 102 includes a gyro sensor, an acceleration sensor, an inertial measurement device (IMU), sensors that detect an operation amount of an acceleration pedal, an operation amount of a brake pedal, a steering angle of a steering wheel, an engine speed, a motor speed, a wheel rotation speed, or the like.

Furthermore, for example, the data acquisition unit 102 includes various sensors that detect information outside the own vehicle. Specifically, for example, the data acquisition unit 102 includes an imaging device such as a Time Of Flight (ToF) camera, a stereo camera, a monocular camera, an infrared camera, other camera, or the like. Furthermore, for example, the data acquisition unit 102 includes an environmental sensor that detects the weather, the meteorological phenomenon, or the like and a surrounding information detection sensor that detects an object around the own vehicle. The environmental sensor includes, for example, a raindrop sensor, a fog sensor, a sunshine sensor, a snow sensor, or the like. The surrounding information detection sensor includes, for example, an ultrasonic wave sensor, a radar, a Light Detection and Ranging and Laser Imaging Detection and Ranging (LiDAR), a sonar, or the like.

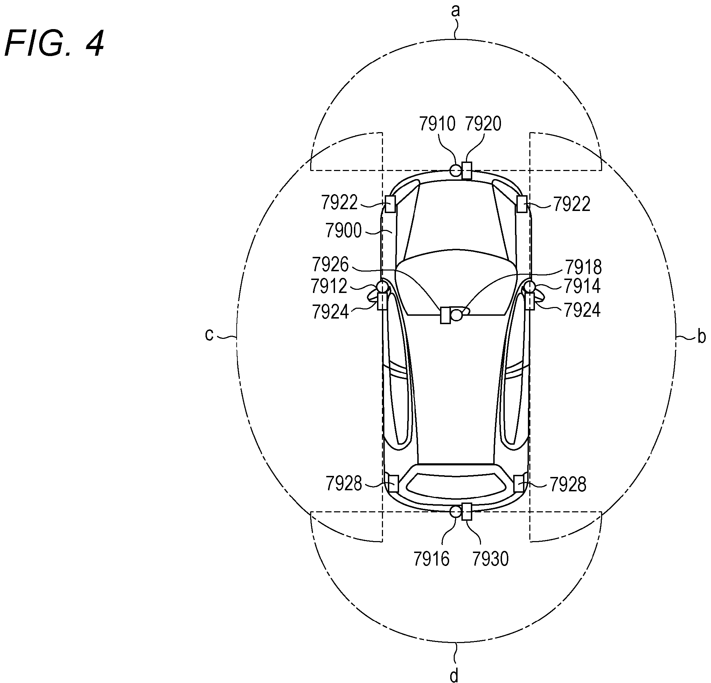

For example, Fig. 4 illustrates an installation example of various sensors to detect the information outside the own vehicle. Each of imaging devices 7910, 7912, 7914, 7916, and 7918 is provided in at least one position of, for example, a front nose, a side mirror, a rear bumper, a back door, or an upper side of a windshield in the interior of a vehicle 7900.

The imaging device 7910 provided in the front nose and the imaging device 7918 provided on the upper side of the windshield in the vehicle interior mainly obtain images on the front side of the vehicle 7900. The imaging devices 7912 and 7914 provided in the side mirrors mainly obtain images on the sides of the vehicle 7900. The imaging device 7916 provided in the rear bumper or the back door mainly obtains an image on the back side of the vehicle 7900. The imaging device 7918 provided on the upper side of the windshield in the vehicle interior is mainly used to detect a preceding vehicle, a pedestrian, an obstacle, a traffic light, a traffic sign, a traffic lane, or the like. Furthermore, in the future and in the automatic driving, when the vehicle turns right, the use of the imaging device may be extended to a pedestrian on a right-turn or left-turn destination road in a wider range and further to a crossing road approaching object range.

Note that, in Fig. 4, exemplary photographing ranges of the respective imaging devices 7910, 7912, 7914, and 7916 are illustrated. An imaging range a indicates an imaging range of the imaging device 7910 provided in the front nose, and imaging ranges b and c respectively indicate imaging ranges of the imaging devices 7912 and 7914 provided in the side mirrors. An imaging range d indicates an imaging range of the imaging device 7916 provided in the rear bumper or the back door. For example, by superposing image data imaged by the imaging devices 7910, 7912, 7914, and 7916, a bird's eye image of the vehicle 7900 viewed from above, and in addition, an all-around stereoscopic display image of a vehicle periphery surrounded by a curved plane, or the like can be obtained.

Sensors 7920, 7922, 7924, 7926, 7928, and 7930 provided on the front, the rear, the sides, the corner, and the upper side of the windshield in the vehicle interior of the vehicle 7900 may be, for example, ultrasonic wave sensors or radars. The sensors 7920, 7926, and 7930 provided on the front nose, the rear bumper, the back door, and the upper side of the windshield in the vehicle interior of the vehicle 7900 may be, for example, LiDARs. These sensors 7920 to 7930 are mainly used to detect a preceding vehicle, a pedestrian, an obstacle, or the like. These detection results may be further applied to improve a stereoscopic display in the bird's eye display and the all-around stereoscopic display.

Returning to Fig. 3, each component will be described. The data acquisition unit 102 includes various sensors that detect a current position of the own vehicle. Specifically, for example, the data acquisition unit 102 includes a Global Navigation Satellite System (GNSS) receiver or the like that receives a GNSS signal from a GNSS satellite.

Furthermore, for example, the data acquisition unit 102 includes various sensors that detect in-vehicle information. Specifically, for example, the data acquisition unit 102 includes an imaging device that images a driver, a biometric sensor that detects the biological information of the driver, a microphone that collects sounds in the vehicle interior, or the like. The biometric sensor is provided, for example, on a seat surface, a steering wheel, or the like and detects a sitting state of the occupant who sits on the seat or the biological information of the driver who holds the steering wheel. As the vital signal, various observable data can be used such as a heart rate, a pulse rate, a blood flow, breathing, psychosomatic correlation, visual stimulation, a brain wave, a sweating state, a head posture and behavior, eyes, watch, blink, saccade, microsaccade, visual fixation, drift, gaze, pupil response of iris, sleep depth estimated from the heart rate and the breathing, an accumulated cumulative fatigue level, a sleepiness index, a fatigue index, an eyeball search frequency of visual events, visual fixation delay characteristics, visual fixation maintenance time, or the like. The biological activity observable information reflecting the observable driving state is used to calculate a return notification timing by a safety determination unit 155 to be described later as characteristics specific for a case where the return of the driver is delayed from the return delay time characteristics that are aggregated as an observable evaluation value estimated by the observation and are associated with a log of an evaluation value.

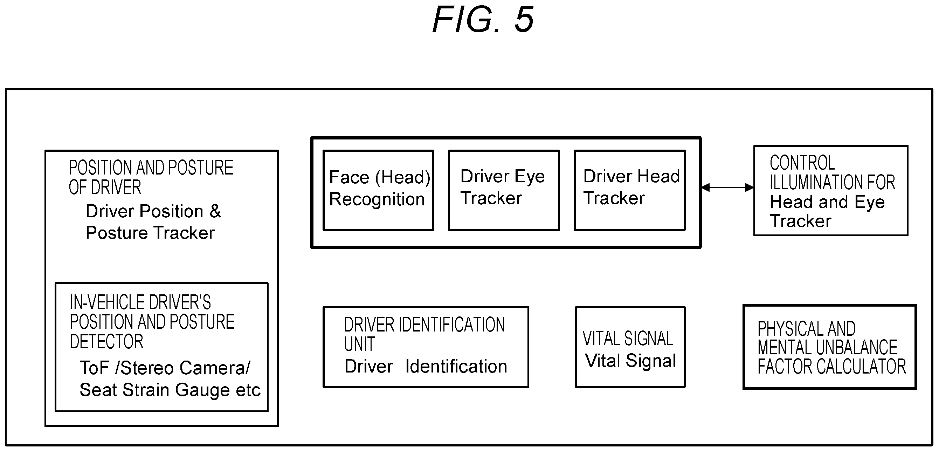

Fig. 5 illustrates an example of various sensors used to obtain information regarding the driver in the vehicle included in the data acquisition unit 102. For example, the data acquisition unit 102 includes a ToF camera, a stereo camera, a Seat Strain Gauge, or the like as a detector that detects a position and a posture of the driver. Furthermore, the data acquisition unit 102 includes a face recognition device (Face (Head) Recognition), a driver eye tracker (Driver Eye Tracker), a driver head tracker (Driver Head Tracker), or the like as a detector that obtains biological activity observable information of the driver.

Furthermore, the data acquisition unit 102 includes a vital signal (Vital Signal) detector as a detector that obtains the biological activity observable information of the driver. Furthermore, the data acquisition unit 102 includes a driver identification (Driver Identification) unit. Note that, as an identification method, biometric identification by using the face, the fingerprint, the iris of the pupil, the voiceprint, or the like is considered in addition to knowledge identification by using a password, a personal identification number, or the like.

Moreover, the data acquisition unit 102 includes a physical and mental unbalance factor calculator that detects eyeball behavior characteristics and pupil behavior characteristics of the driver and calculates an unbalance evaluation value of the sympathetic nerve and the parasympathetic nerve of the driver.

The communication unit 103 communicates with the in-vehicle device 104, various devices outside the vehicle, a server, a base station, or the like. The communication unit 103 transmits data supplied from each unit of the moving device 100 and supplies the received data to each unit of the moving device 100. Note that a communication protocol supported by the communication unit 103 is not particularly limited. Furthermore, the communication unit 103 can support a plurality of types of communication protocols.

For example, the communication unit 103 performs wireless communication with the in-vehicle device 104 by using a wireless LAN, the Bluetooth (registered trademark), Near Field Communication (NFC), a Wireless USB (WUSB), or the like. Furthermore, for example, the communication unit 103 performs wired communication with the in-vehicle device 104 by using a Universal Serial Bus (USB), the High-Definition Multimedia Interface (HDMI) (registered trademark), the Mobile High-definition Link (MHL), or the like via a connection terminal which is not illustrated (and cable as necessary).

Moreover, for example, the communication unit 103 communicates with a device (for example, application server or control server) that exists on an external network (for example, the Internet, cloud network, or company-specific network) via the base station or an access point. Furthermore, for example, the communication unit 103 communicates with a terminal near the own vehicle (for example, terminal of pedestrian or shop or Machine Type Communication (MTC) terminal) by using the Peer To Peer (P2P) technology.

Moreover, for example, the communication unit 103 performs V2X communication such as Vehicle to Vehicle (intervehicle) communication, Vehicle to Infrastructure (between vehicle and infrastructure) communication, Vehicle to Home (between own vehicle and home) communication, and Vehicle to Pedestrian (between vehicle and pedestrian) communication. Furthermore, for example, the communication unit 103 includes a beacon reception unit, receives radio waves or electromagnetic waves transmitted from a wireless station installed on a road or the like, and acquires information including the current position, congestion, traffic regulations, a required time, or the like. Note that the communication unit may perform pairing with a preceding traveling vehicle that is traveling in a section and may be a leading vehicle, acquire information acquired by a data acquisition unit mounted in the preceding vehicle as previous traveling information, and complementally use the information with the data of the data acquisition unit 102 of the own vehicle. In particular, this may be a unit that ensures the safety of a subsequent rank when a leading vehicle leads traveling ranks.

The in-vehicle device 104 includes, for example, a mobile device (tablet, smartphone, or the like) or a wearable device of the occupant, or an information device carried in or attached to the own vehicle, and a navigation device that searches for a route to an optional destination or the like. Note that, in consideration of the fact that the occupant is not necessarily fixed to a seat fixed position in accordance with widespread use of automatic driving, a video player, a game machine, and other device that is detachable from the vehicle may be extendedly used in the future. In the present embodiment, an example is described in which a person to whom information regarding a point where the driver's intervention is needed is limited to the driver. However, the information may be provided to a subsequent vehicle in the traveling rank or the like. In addition, by constantly providing the information to an operation management center of a passenger transport share-ride bus or a long-distance logistics commercial vehicle, the information may be appropriately used in combination with remote traveling support.

The output control unit 105 controls an output of various information to the occupant of the own vehicle or the outside of the own vehicle. For example, the output control unit 105 generates an output signal including at least one of visual information (for example, image data) or auditory information (for example, audio data) and supplies the generated signal to the output unit 106 so as to control the outputs of the visual information and the auditory information from the output unit 106. Specifically, for example, the output control unit 105 synthesizes pieces of imaging data imaged by different imaging devices of the data acquisition unit 102, generates a bird's eye image, a panoramic image, or the like, and supplies the output signal including the generated image to the output unit 106. Furthermore, for example, the output control unit 105 generates audio data including warning sound, a warning message, or the like for danger such as collision, contact, entry to a dangerous zone, or the like, and supplies the output signal including the generated audio data to the output unit 106.

The output unit 106 includes a device that can output the visual information or the auditory information to the occupant of the own vehicle or the outside of the vehicle. For example, the output unit 106 includes a display device, an instrument panel, an audio speaker, a headphone, a wearable device such as a glass-shaped display worn by the occupant or the like, a projector, a lamp, or the like. The display device included in the output unit 106 may be a device that displays the visual information in a field of view of the driver, for example, a head-up display, a transmissive display, a device having an Augmented Reality (AR) display function, or the like, in addition to a display having a normal display.

The driving system control unit 107 generates various control signals and supplies the generated signals to the driving system 108 so as to control the driving system 108. Furthermore, the driving system control unit 107 supplies the control signal to each unit other than the driving system 108 as necessary and issues a notification of a control state of the driving system 108 or the like.

The driving system 108 includes various devices related to the driving system of the own vehicle. For example, the driving system 108 includes a driving force generation device that generates a driving force such as an internal combustion engine, a driving motor, or the like, a driving force transmission mechanism that transmits the driving force to the wheels, a steering mechanism that adjusts the steering angle, a braking device that generates a braking force, an Antilock Brake System (ABS), an Electronic Stability Control (ESC), an electronic power steering device, or the like.

The body system control unit 109 generates various control signals and supplies the generated signals to the body system 110 so as to control the body system 110. Furthermore, the body system control unit 109 supplies the control signal to each unit other than the body system 110 as necessary and issues a notification of a control state of the body system 110 or the like.

The body system 110 includes various body-system devices mounted on the vehicle body. For example, the body system 110 includes a keyless entry system, a smart key system, a power window device, a power seat, a steering wheel, an air conditioner, various lamps (for example, headlights, backlights, indicators, fog lights, or the like), or the like.

The storage unit 111 includes, for example, a magnetic storage device such as a Read Only Memory (ROM), a Random Access Memory (RAM), or a Hard Disc Drive (HDD), a semiconductor storage device, an optical storage device, a magneto-optical storage device, or the like. The storage unit 111 stores various programs, data, or the like used by each unit of the moving device 100. For example, the storage unit 111 stores map data such as a three-dimensional high-accuracy map such as a dynamic map, a global map that covers a wide area and has lower accuracy than the high-accuracy map, a local map including information around the own vehicle, or the like.

The automatic driving control unit 112 controls the automatic driving such as autonomous traveling, driving assistance, or the like. Specifically, for example, the automatic driving control unit 112 performs cooperative control to realize a function of an Advanced Driver Assistance System (ADAS) including collision avoidance or impact relaxation of the own vehicle, following traveling based on a distance between vehicles, a vehicle speed maintaining travel, an own vehicle collision warning, a lane deviation warning of the own vehicle, or the like. Furthermore, for example, the automatic driving control unit 112 performs cooperative control for the automatic driving for autonomously traveling without depending on the operation by the driver. The automatic driving control unit 112 includes a detection unit 131, a self-position estimation unit 132, a situation analysis unit 133, a planning unit 134, and an operation control unit 135.

The detection unit 131 detects various information necessary for controlling the automatic driving. The detection unit 131 includes a vehicle exterior information detection unit 141, an in-vehicle information detection unit 142, and a vehicle state detection unit 143.

The vehicle exterior information detection unit 141 executes processing for detecting information outside the own vehicle on the basis of the data or the signal from each unit of the moving device 100. For example, the vehicle exterior information detection unit 141 executes detection processing, recognition processing, and tracking processing on an object around the own vehicle and processing for detecting a distance to the object and a relative speed. The object to be detected includes, for example, a vehicle, a person, an obstacle, a structure, a road, a traffic light, a traffic sign, a road marking, or the like.

Furthermore, for example, the vehicle exterior information detection unit 141 executes processing for detecting environment around the own vehicle. The surrounding environment to be detected includes, for example, the weather, the temperature, the humidity, the brightness, the state of the road surface, or the like. The vehicle exterior information detection unit 141 supplies data indicating the result of the detection processing to the self-position estimation unit 132, a map analysis unit 151, a traffic rule recognition unit 152, and a situation recognition unit 153 of the situation analysis unit 133, and an emergency avoidance unit 171 of the operation control unit 135, or the like.

When a traveling section is a section in which a local dynamic map that is constantly updated by setting the traveling section as a section in which traveling using the automatic driving can be intensively performed is supplied from the infrastructure, the information acquired by the vehicle exterior information detection unit 141 can be received mainly from the infrastructure. Alternatively, traveling may be performed by receiving constantly updated information from a vehicle or a vehicle group that precedingly travels in the section prior to the entry to the section. Furthermore, in a case where the local dynamic map is not constantly updated to the latest map by the infrastructure, in order to obtain road information immediately before a safer entry section particularly in the traveling ranks, road environment information obtained by a leading vehicle that enters the section may be complementally used. In many cases, whether or not the section is a section in which the automatic driving can be performed is determined depending on whether or not information has been provided from the infrastructures in advance. Information indicating whether or not the automatic driving travel can be performed on a route provided by the infrastructure is equivalent to provision of an invisible track as so-called "information". Note that, for convenience, the vehicle exterior information detection unit 141 is illustrated as assuming that the vehicle exterior information detection unit 141 is mounted on the own vehicle. However, predictability at the time of traveling can be further enhanced by using the information that is recognized as the "information" by the preceding vehicle.

The in-vehicle information detection unit 142 executes processing for detecting the in-vehicle information on the basis of the data or the signal from each unit of the moving device 100. For example, the in-vehicle information detection unit 142 executes processing for authenticating and recognizing the driver, processing for detecting the driver's state, processing for detecting the occupant, processing for detecting in-vehicle environment, or the like. The driver's state to be detected includes, for example, a physical condition, a wakefulness degree, a concentration level, a fatigue level, a line-of-sight direction, a detailed eyeball behavior, or the like.

Moreover, the use of the automatic driving by the driver who is completely separated from a driving steering operation is assumed in the future. Because the driver temporary falls asleep or starts other works, it is necessary for the system to recognize how the wakefulness of the consciousness necessary for returning to the driving is restored. That is, a driver monitoring system that has been conventionally considered has mainly included a detection unit that detects deterioration in the consciousness such as sleepiness. However, since the driver does not intervene driving steering in the future, the system does not include a unit that directly observes a driving intervention degree of the driver from steering stability or the like of the steering device, and it is necessary to transfer intervention to the steering from the automatic driving to the manual driving after observing consciousness restoration transition necessary for driving from a state where the accurate consciousness state of the driver is unknown and recognizing the accurate internal wakefulness state of the driver.

Therefore, the in-vehicle information detection unit 142 mainly has two major roles. One role is passive monitoring of the driver's state during the automatic driving, and the other role is the surrounding recognition, perception, determination of the driver and detection and determination of an operation ability of a steering device to a level at which the manual driving can be performed after the system requests to return the wakefulness and before the vehicle reaches a section in which driving is performed with attention. As in a case where a failure self-diagnosis of the entire vehicle is further performed as control and a function of the automatic driving is deteriorated due to a partial functional failure of the automatic driving, it may be prompted to return to the manual driving by the driver at an early stage. The passive monitoring here indicates a type of a detection unit that does not require a conscious response reaction from the driver and does not exclude a detection unit that emits physical radio wave, light, or the like from the device and detects a response signal. That is, the passive monitoring that indicates monitoring of a driver's unconscious state such as when the driver takes a nap, and monitoring that is not a recognition response reaction of the driver is classified as a passive method. An active response device that analyzes and evaluates reflection of irradiated radio waves, infrared rays, or the like and diffused signals is not excluded. Conversely, a device that requires a conscious response for requesting a response reaction to the driver is assumed to be active.

The in-vehicle environment to be detected includes, for example, the temperature, the humidity, the brightness, the odor, or the like. The in-vehicle information detection unit 142 supplies the data indicating the result of the detection processing to the situation recognition unit 153 of the situation analysis unit 133 and the operation control unit 135. Note that it is found that the automatic driving is not able to be achieved by the driver within an appropriate period after the system has issued a driving return instruction to the driver and it is determined that switch from the automatic driving to the manual driving is too late even if deceleration control is performed while performing the automatic driving and a time extension is generated, an instruction is issued to the emergency avoidance unit 171 of the system or the like, and deceleration and evacuation and stop procedures are started to evacuate the vehicle. That is, even in a situation where the initial states are the same and the switching is too late, it is possible to earn a reach time before reaching a switching limit by stating the deceleration of the vehicle early. Since the deceleration procedure due to the delay of the switching procedure can be performed by the single vehicle, this does not directly cause a problem. However, in a case of improper deceleration, there are a large number of adverse effects such as route obstruction, a congestion inducing factor, a collision risk factor, or the like to the other following vehicle that travels in the road section. Therefore, this is an event that should be avoided as an abnormal event, not as normal control. Therefore, it is desirable to define the deceleration due to the delay of the switching procedure as a penalty target to be described later in order to prevent abnormal use by the driver.

The vehicle state detection unit 143 executes processing for detecting the state of the own vehicle on the basis of the data or the signal from each unit of the moving device 100. The state of the own vehicle to be detected includes, for example, the speed, the acceleration, the steering angle, whether or not an abnormality occurs, content of the abnormality, a driving operation state, a position and inclination of a power seat, a door lock state, a state of other in-vehicle devices, or the like. The vehicle state detection unit 143 supplies the data indicating the result of the detection processing to the situation recognition unit 153 of the situation analysis unit 133, the emergency avoidance unit 171 of the operation control unit 135, or the like.

The self-position estimation unit 132 executes processing for estimating the position, the posture, or the like of the own vehicle on the basis of the data or the signal from each unit of the moving device 100 such as the vehicle exterior information detection unit 141, the situation recognition unit 153 of the situation analysis unit 133, or the like. Furthermore, the self-position estimation unit 132 generates a local map used to estimate the self-position (hereinafter, referred to as self-position estimation map) as necessary.

The self-position estimation map is, for example, a map with high accuracy using a technology such as Simultaneous Localization and Mapping (SLAM). The self-position estimation unit 132 supplies the data indicating the result of the estimation processing to the map analysis unit 151, the traffic rule recognition unit 152, the situation recognition unit 153, or the like of the situation analysis unit 133. Furthermore, the self-position estimation unit 132 makes the storage unit 111 store the self-position estimation map.

The situation analysis unit 133 executes processing for analyzing the situations of the own vehicle and surroundings. The situation analysis unit 133 includes the map analysis unit 151, the traffic rule recognition unit 152, the situation recognition unit 153, a situation prediction unit 154, and the safety determination unit 155.

While using the data or the signal from each unit of the moving device 100 such as the self-position estimation unit 132, the vehicle exterior information detection unit 141, or the like as necessary, the map analysis unit 151 executes processing for analyzing various maps stored in the storage unit 111 and constructs a map including information necessary for the automatic driving processing. The map analysis unit 151 supplies the constructed map to the traffic rule recognition unit 152, the situation recognition unit 153, the situation prediction unit 154, and a route planning unit 161, an action planning unit 162, an operation planning unit 163, or the like of the planning unit 134.

The traffic rule recognition unit 152 executes processing for recognizing traffic rules around the own vehicle on the basis of the data or the signal from each unit of the moving device 100 such as the self-position estimation unit 132, the vehicle exterior information detection unit 141, the map analysis unit 151, or the like. According to this recognition processing, for example, a position and a state of a traffic light around the own vehicle, content of traffic regulations around the own vehicle, a traffic lane on which the own vehicle can travel, or the like are recognized. The traffic rule recognition unit 152 supplies the data indicating the result of the recognition processing to the situation prediction unit 154 or the like.

The situation recognition unit 153 executes processing for recognizing a situation of the own vehicle on the basis of the data or the signal from each unit of the moving device 100 such as the self-position estimation unit 132, the vehicle exterior information detection unit 141, the in-vehicle information detection unit 142, the vehicle state detection unit 143, the map analysis unit 151, or the like. For example, the situation recognition unit 153 executes processing for recognizing a situation of the own vehicle, a situation around the own vehicle, a situation of the driver of the own vehicle, or the like. Furthermore, the situation recognition unit 153 generates a local map used to recognize the situation around the own vehicle (hereinafter, referred to as situation recognition map) as necessary. The situation recognition map is, for example, an Occupancy Grid Map (Occupancy Grid Map).

The situations of the own vehicle to be recognized include, for example, vehicle specific or load specific conditions such as the position, the posture, the movement (for example, speed, acceleration, moving direction, or the like) of the own vehicle, a loading movement determining motion characteristics of the own vehicle and gravity movement of the vehicle body caused by the loading cargo, the tire pressure, braking distance movement depending on a brake pad wearing situation, maximum allowable speed reduction braking to prevent cargo movement causing the load movement, a centrifugal relaxation limit speed at the time of curve traveling caused by loading liquid, or the like, and in addition, a friction coefficient of a road surface, a road curve, a gradient, or the like. Even in the completely same road environment, the return start timing requested for control differs depending on the characteristics of the vehicle and the load or the like. Therefore, it is necessary to collect and learn various conditions and reflect the learned conditions to an optimal timing at which the control is performed. The content of the situation is not content with which it is not sufficient whether or not an abnormality occurs, content, or the like of the own vehicle be simply observed and monitored in determining a control timing depending of a type and a load of the vehicle. In the transportation industry, in order to ensure certain safety according to characteristics specific for the load, a parameter that determines to add a desirable return grace time may be set as a fixed value in advance, and it is not necessary to use a method for uniformly determining all the notification timing determination conditions by self-accumulation learning.

The situation around the own vehicle to be recognized includes, for example, a type and a position of a stationary object around the own vehicle, a type of a moving object around the own vehicle, a position and a movement (for example, speed, acceleration, moving direction, or the like), a configuration of a road around the own vehicle and a state of a road surface, and the weather, the temperature, the humidity, the brightness, or the like around the own vehicle. The driver's state to be detected includes, for example, a physical condition, a wakefulness degree, a concentration level, a fatigue level, a line-of-sight movement, a driving operation, or the like. In order to safely travel the vehicle, a control state point when a counter-measure is required largely differs according to a loading amount mounted on the vehicle in a specific state and a chassis fixing state of a mounting unit, deviation of the center of gravity, a maximally deceleratable acceleration value, a maximal loadable centrifugal force, a return response delay amount in accordance with the driver's state, or the like.

The situation recognition unit 153 supplies the data indicating the result of the recognition processing (including situation recognition map as necessary) to the self-position estimation unit 132, the situation prediction unit 154, or the like. Furthermore, the situation recognition unit 153 makes the storage unit 111 store the situation recognition map.

The situation prediction unit 154 executes processing for predicting the situation of the own vehicle on the basis of the data or the signal from each unit of the moving device 100 such as the map analysis unit 151, the traffic rule recognition unit 152, the situation recognition unit 153, or the like. For example, the situation prediction unit 154 executes the processing for predicting the situation of the own vehicle, the situation around the own vehicle, the situation of the driver, or the like.

The situation of the own vehicle to be predicted includes, for example, a behavior of the own vehicle, occurrence of an abnormality, a travelable distance, or the like. The situation around the vehicle to be predicted includes, for example, a behavior of a moving object around the own vehicle, a change in a state of the traffic light, a change in the environment such as the weather, or the like. The situation of the driver to be predicted includes, for example, a behavior, a physical condition, or the like of the driver.

The situation prediction unit 154 supplies the data indicating the result of the prediction processing to the route planning unit 161, the action planning unit 162, the operation planning unit 163, or the like of the planning unit 134 together with the data from the traffic rule recognition unit 152 and the situation recognition unit 153.

The safety determination unit 155 has a function as a learning processing unit that learns an optimal return timing depending on a return action pattern of the driver, the vehicle characteristics, or the like and provides learned information to the situation recognition unit 153 or the like. As a result, for example, it is possible to present, to the driver, an optimal timing that is statistically obtained and is required for the driver to normally return from the automatic driving to the manual driving at a ratio equal to or more than a predetermined fixed ratio.

The route planning unit 161 plans a route to a destination on the basis of the data or the signal from each unit of the moving device 100 such as the map analysis unit 151, the situation prediction unit 154, or the like. For example, the route planning unit 161 sets a route from the current position to a designated destination on the basis of a global map. Furthermore, for example, the route planning unit 161 appropriately changes the route on the basis of a situation such as congestions, accidents, traffic regulation, constructions, or the like, the physical condition of the driver, or the like. The route planning unit 161 supplies data indicating the planned route to the action planning unit 162 or the like.

The action planning unit 162 plans an action of the own vehicle to safely travel the route planned by the route planning unit 161 within a planned time on the basis of the data or the signal from each unit of the moving device 100 such as the map analysis unit 151, the situation prediction unit 154, or the like. For example, the action planning unit 162 makes a plan such as starting, stopping, a traveling direction (for example, forward, backward, turning left, turning right, turning, or the like), a traveling lane, a traveling speed, overtaking, or the like. The action planning unit 162 supplies data indicating the planned action of the own vehicle to the operation planning unit 163 or the like.

The operation planning unit 163 plans an operation of the own vehicle to realize the action planned by the action planning unit 162 on the basis of the data or the signal from each unit of the moving device 100 such as the map analysis unit 151, the situation prediction unit 154, or the like. For example, the operation planning unit 163 plans acceleration, deceleration, a traveling track, or the like. The operation planning unit 163 supplies data indicating the planned operation of the own vehicle to an acceleration and deceleration control unit 172, a direction control unit 173, or the like of the operation control unit 135.

The operation control unit 135 controls the operation of the own vehicle. The operation control unit 135 includes the emergency avoidance unit 171, the acceleration and deceleration control unit 172, and the direction control unit 173.

The emergency avoidance unit 171 executes processing for detecting an emergency such as collisions, contacts, entry to the dangerous zone, an abnormality of the driver, an abnormality of the vehicle, or the like on the basis of the detection results of the vehicle exterior information detection unit 141, the in-vehicle information detection unit 142, and the vehicle state detection unit 143. In a case where the occurrence of the emergency is detected, the emergency avoidance unit 171 plans an operation of the own vehicle to avoid an emergency such as sudden stop, sudden turn, or the like. The emergency avoidance unit 171 supplies data indicating the planned operation of the own vehicle to the acceleration and deceleration control unit 172, the direction control unit 173, or the like.

The acceleration and deceleration control unit 172 controls acceleration and deceleration to realize the operation of the own vehicle planned by the operation planning unit 163 or the emergency avoidance unit 171. For example, the acceleration and deceleration control unit 172 calculates a control target value of the driving force generation device or the braking device used to realize the planned acceleration, deceleration, or sudden stop and supplies a control instruction indicating the calculated control target value to the driving system control unit 107. Note that there are two main cases in which an emergency may occur. That is, there are a case where an unexpected accident occurs due to a sudden reason when the automatic driving is performed on a road that is a traveling route during the automatic driving and is assumed to be originally safe in the local dynamic map or the like acquired from the infrastructures and emergency return is too late and a case where it is difficult for the driver to accurately return from the automatic driving to the manual driving.

The direction control unit 173 controls a direction to realize the operation of the own vehicle planned by the operation planning unit 163 or the emergency avoidance unit 171. For example, the direction control unit 173 calculates a control target value of the steering mechanism to realize a traveling track or a sudden turn planned by the operation planning unit 163 or the emergency avoidance unit 171 and supplies a control instruction indicating the calculated control target value to the driving system control unit 107.

### [3. Outline of Generation Processing and Usage Processing of Wakefulness State Evaluation Dictionary And Exemplary Data Structure of Dictionary]

Next, an outline of generation processing and usage processing of a wakefulness state evaluation dictionary executed by any one of the moving device according to the present invention, the information processing apparatus included in the moving device, or a server that communicates with these devices and an exemplary data structure of the dictionary will be described. Note that the generated wakefulness state evaluation dictionary is used for processing for evaluating a wakefulness state of the driver who is driving the moving device.

The wakefulness state evaluation dictionary is a dictionary that has data indicating a lowering risk (= wakefulness lowering risk) of the wakefulness degree (consciousness level) specific for the driver and is a dictionary that is specific for the driver and is associated with each driver.

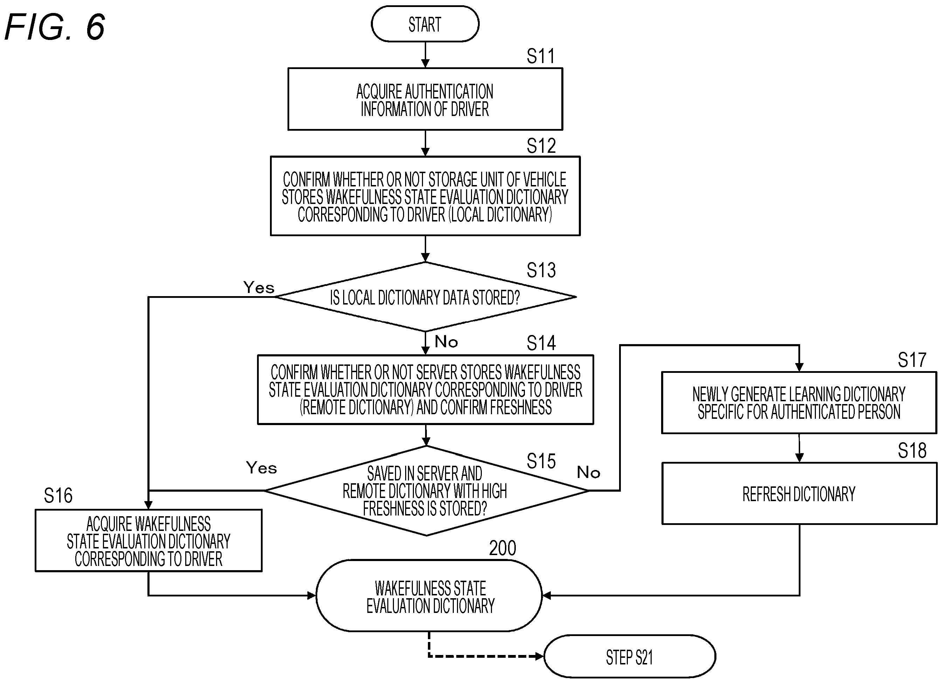

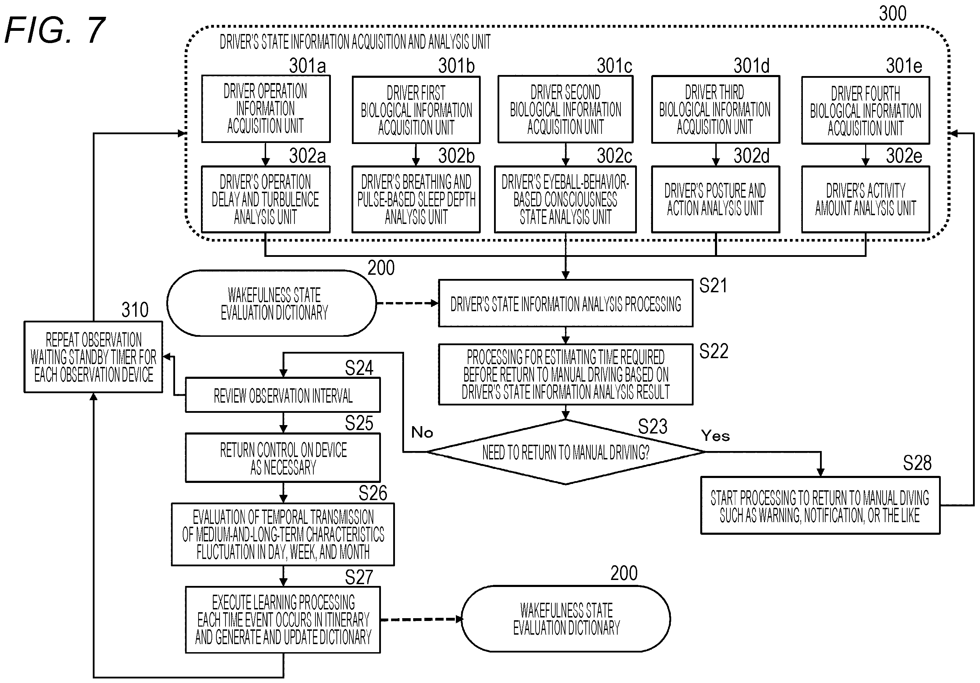

A generation sequence of the wakefulness state evaluation dictionary will be described with reference to the flowcharts illustrated in Figs. 6 and 7.

Note that, in the description of the flow in Fig. 6 and the subsequent drawings, processing in each step in the flow is executed by the moving device according to the present invention, the information processing apparatus included in the moving device, or the server that communicates with these devices. However, in the following description, for simplification of the description, an example of setting will be described in which the information processing apparatus executes the processing in each step.

### (Step S11)

First, the information processing apparatus executes processing for authenticating the driver in step S11. In this authentication processing, the information processing apparatus executes collation processing with user information (driver information) that has been registered in the storage unit in advance, identifies the driver, and acquires personal data of the driver that has been stored in the storage unit.

### (Step S12)

Next, in step S12, it is confirmed whether or not a dictionary (wakefulness state evaluation dictionary corresponding to driver) used to evaluate the wakefulness state of the authenticated driver is saved in the storage unit of the information processing apparatus (storage unit in vehicle) as a local dictionary of the vehicle.

In a case where the authenticated driver uses the vehicle on a daily basis, the storage unit in the vehicle saves the local dictionary (wakefulness state evaluation dictionary) corresponding to the driver. The local dictionary (wakefulness state evaluation dictionary) saves learning data or the like such as a driver's state observation value that is observable for each driver, behavior characteristics when the driver returns from the automatic driving to the manual driving, or the like.

By using dictionary data including the return behavior characteristics specific for the driver, it is possible for the system to estimate the wakefulness degree and the delay time until the driver returns from the detected state to the manual driving from the observed driver's state observation value.

For example, the return delay characteristics of the driver are calculated on the basis of observable evaluation values of a series of transitional behaviors such as pulse wave analysis, eyeball behaviors, or the like of the driver that is monitored during driving.

Note that the same driver does not necessarily use the same vehicle repeatedly. For example, in a case of car sharing, there is a case where a single driver uses a plurality of vehicles. In this case, there is a possibility that the local dictionary (wakefulness state evaluation dictionary) corresponding to the driver is not stored in the storage unit in the vehicle driven by the driver. In order to make it possible to use the wakefulness state evaluation dictionary corresponding to the driver in such a case, the dictionary corresponding to each driver is stored in an external server that can communicate with the automobile as a remote dictionary, and each vehicle has a configuration that can acquire the remote dictionary from the server as necessary.

Note that the remote dictionary includes the wakefulness state evaluation dictionary corresponding to a large number of drivers.

### (Step S13)

The information processing apparatus confirms in step S13 whether or not the wakefulness state evaluation dictionary corresponding to the driver is stored in the storage unit of the vehicle that is currently driven as the local dictionary.

In a case where the wakefulness state evaluation dictionary is stored, the procedure proceeds to step S16.

In a case where the wakefulness state evaluation dictionary is not stored, the procedure proceeds to step S14.

### (Steps S14 and S15)

In a case where the information processing apparatus determines in step S13 that the wakefulness state evaluation dictionary corresponding to the driver is not stored in the storage unit of the vehicle that is currently driven as the local dictionary, the information processing apparatus confirms in steps S14 and S15 whether or not the wakefulness state evaluation dictionary (remote dictionary) corresponding to the driver is stored in the server, and further confirms freshness of the wakefulness state evaluation dictionary in a case where the wakefulness state evaluation dictionary is stored.

In a case where the wakefulness state evaluation dictionary (remote dictionary) corresponding to the driver with high freshness is stored in the server, the procedure proceeds to step S16.

In a case where the wakefulness state evaluation dictionary is not stored, the procedure proceeds to step S14.

Note that, at the time of searching the server for the dictionary, the driver may manually specify a save destination of the dictionary or the system may execute personal identification processing on the driver on the basis of the authentication information in step S11 and automatically search the save destination of the dictionary from a remote server or the like. Moreover, a search destination may be registered in advance as member log data of a lease, a shared car, a rental car, or the like, a boarding card, and preset selection saving information.

In a case where the driver repeatedly uses the same vehicle on a daily basis, the self-completed use is assumed such that a dictionary that is updated for each use in the local dictionary in the vehicle. On the other hand, regarding the commercial vehicles such as a taxi, a share-ride bus, and a logistics delivery vehicle, there is a use form in which a plurality of drivers alternately uses the same vehicle in order to efficiently use the vehicles. In a use form in which a combination of a vehicle and a driver assigned depending on an operation plan is determined each time, the dictionary is remotely stored in a remote server or the like and is appropriately downloaded to the vehicle in use and used each time the drive uses the vehicle, without associating the dictionary with the vehicle.

In applications in which the driver switches a plurality of vehicles as in a case of a commercial vehicle such as a taxi, it is preferable to use a form in which the learning data of the driving behavior characteristics is saved in the remote server and locally downloaded according to the vehicle when the vehicle is used and additionally learned. In particular, in the industry in which a bus, a taxi, and a truck of which drivers are changed are operated at high efficiency, the driving is not performed by necessarily being fixed to a specific vehicle. This will be an important use form in car sharing, rental cars, or the like in the future.

When the data becomes obsolete due to a long non-use period or the like, it is difficult to appropriately determine the wakefulness state of the driver. Therefore, in a case where valid dictionary data with high freshness is confirmed, the dictionary is taken into a local reference dictionary of the vehicle before using the dictionary data, and the dictionary is used to determine the state of the specific driver. Specific information of the driver may be saved in a recording medium in the vehicle, and may be data that is additionally learned, updated, and saved each travel in the past as a learning history in a remote system that manages operations including the user as described later. Note that the dictionary data with high freshness indicates that the vehicle is normally used on a daily basis until few days immediately before the determination date. For example, in a case where the driver who does not drive the vehicle for several months or several years drives the vehicle, the freshness of the dictionary is low, and the dictionary data is not suitable to directly use for the estimation of the wakefulness state.

In step S15, in a case where the wakefulness state evaluation dictionary (remote dictionary) corresponding to the driver is stored in the server, the freshness of the dictionary is confirmed.

In a case where a user who does not have a dictionary newly uses the vehicle and a case where a blank period when the dictionary is not used is long, there is a possibility that the driving characteristics of the driver change. Therefore, the procedure proceeds to step S17, a new dictionary is generated or an existing dictionary is refreshed, and it is determined whether or not to start to newly learn the characteristics of the driver.

For example, a system that can recognize the driver's state does not constantly function in all the vehicles to be used by the driver. Furthermore, in a case where a blank period when the driver does not use the vehicle is long, there is a possibility that the characteristics of the driver are different from the information registered in the learning dictionary, that is, the characteristics are fluctuated. If such an old dictionary is used, it is difficult to accurately determine the driver's state on the basis of the current observable wakefulness related information of the driver.

Therefore, in a case where the existing authenticated person continuously reuses the vehicle, it is necessary to confirm the freshness of the saved dictionary. In steps S14 and S15, the freshness determination processing is executed.

In a case where the dictionary (wakefulness state evaluation dictionary (remote dictionary)) corresponding to the driver is detected from the remote server in the determination processing in step S15, the procedure proceeds to step S16.

### (Step S16)

In a case where the dictionary (wakefulness state evaluation dictionary (remote dictionary)) corresponding to the driver is detected from the vehicle in step S13 or in a case where the dictionary (wakefulness state evaluation dictionary (remote dictionary)) corresponding to the driver with high freshness is detected from the server in step S15, the procedure proceeds to step S16.

In step S16, the information processing apparatus acquires the wakefulness state evaluation dictionary corresponding to the driver from the vehicle or the server.

### (Steps S17 and S18)

In a case where the dictionary (wakefulness state evaluation dictionary (remote dictionary)) corresponding to the driver is not detected from the vehicle in step S13 and in a case where the dictionary (wakefulness state evaluation dictionary (remote dictionary)) corresponding to the driver with high freshness is not detected from the server in step S15, the procedure proceeds to step S17.

In steps S17 and S18, the information processing apparatus generates a new dictionary corresponding to the driver. Note that, in a case where the dictionary becomes obsolete due to a long non-use period although there is the dictionary corresponding to the driver, the dictionary is refreshed. In a case where the driver's state is estimated from the existing dictionary, it is confirmed whether or not observable wakefulness related biological information of the driver in the unused period and a delay time necessary for actual wakefulness return are fluctuated, and calibration is performed.

By executing the processing according to the flow, acquisition of a wakefulness state evaluation dictionary 200 corresponding to a driver that is described at the end of the flow is completed. In the wakefulness state evaluation dictionary 200 corresponding to the driver, a data processing unit of the information processing apparatus, specifically, a data processing unit that executes driver's wakefulness state determination processing is stored in an accessible memory.

The wakefulness state evaluation dictionary 200 corresponding to the driver is used for driver's wakefulness state determination processing based on driver's state information (observation value), processing for estimating time needed before the return to the manual driving (delay time), or the like.

The information processing apparatus regularly monitors the driver, acquires the driver's state information (biological information and operation information) as monitoring information, appropriately optimizes an observation device while predicting a change in the state, acquires the optimized observation result, and executes the driver's wakefulness state determination processing, the processing for estimating the time needed before the return to the manual driving (delay time), or the like by using the wakefulness state evaluation dictionary 200 corresponding to the driver.

Note that, in a case where necessity for switching the automatic driving to the manual driving suddenly occurs, it is necessary for the information processing apparatus to determine whether or not the driver can immediately start the manual driving. In that case, the driver who drives the event is observed for early determination. By using the dictionary obtained as the result of the learning history of the driver, the state of the driver is monitored, and the change in the state is observed at a plurality of different intervals including at least a change in a short range and a change in a medium range.

The reason why it is necessary to observe the driver's state at the different time intervals including the change in the short range and the change in the medium range is as follows. That is, the driver can completely rely on the automatic driving in a fully automatic driving possible section such as a motorway in which switching to the manual driving is not needed. In a case where the fully automatic driving possible section continues, the necessity of the manual driving is low, and the driver can engage in tasks that are largely separated from a driving steering task. An example of the above is a case where it is expected that the driver has sufficient time before the return, such as a resting task in which the driver leaves the seat and enters a deep sleep while lying down or a sorting work for moving to the back and a sorting packages for each delivery destination. In that case, it is not necessary for the driver to return to the manual driving in a short period of time. Therefore, in that case, the necessary for frequently observing the driver's state is low, and it is considered that a long observation interval does not cause a great problem.

Note that information indicating whether or not the road on which the automobile is traveling is the fully automatic driving possible section or the section in which the manual driving is needed can be acquired from a local dynamic map (LDM) that is received from the external server by the automobile. The local dynamic map (LDM) is travel map information regarding the road on which the vehicle travels and includes section information indicating that roads on the map is in the fully automatic driving possible section, a manual driving required section, or the like. The section information is sequentially changed and updated, for example, on the basis of a situation change such as a congestion.

On the other hand, in a case where a switching point (switching point from automatic driving section to manual driving section) is close or a section in which careful travel is requested is approaching or in a case where the vehicle travels in a section having an unexpected switching risk, there is an increasing possibility that a time needed before the driver's return (return to manual driving) is short and is limited. Therefore, it is necessary for the system to accurately recognize the time necessary for return in order to reduce the risk. That is, it is necessary to obtain highly accurate information for predicting the time necessary for the driver to return by the system on the basis of the current state of the driver. In such a situation, the driver needs to be prepared to return to some extent, and is quickly ready to prepare for switching in response to a notification. Observation of a state of rapid changes is assumed, and the return work with rapid change is started from a point when the driver receives a notification or a warning. Therefore, it is desirable to start high-frequency observation at the timing of notification or a certain period before the notification timing. Furthermore, recognition of the return situation of the driver and the return ability are reflected on a transition state of a return start procedure at that time. Then, a quick operation such as visual information acquisition is subsequently performed to recognize the situation at the final stage when the active steering is started. In order to calculate the notification timing in advance before the notification is issued, the system starts log recording before the notification. That is, active reaction characteristics and transition characteristics of the driver before and after the notification in response to the notification are observed at this initial step.

It is necessary to appropriately perform monitoring in stepwise to estimate the return prediction time. That is, regarding a monitoring cycle of the observation of the driver's state, it is necessary to appropriately review a timing for confirmation of a next driver's state by adding section approach information of the road.

Next, sequences of the driver's wakefulness state determination processing and the processing for estimating the time needed before the return to the manual driving (delay time) by the information processing apparatus will be described with reference to the flowchart illustrated in Fig. 7. This processing is executed by using the wakefulness state evaluation dictionary 200 corresponding to the driver generated in the sequence that has been previously described with reference to Fig. 6.

Processing in each step in the flow illustrated in Fig. 7 will be described.

Fig. 7 illustrates a driver's state information acquisition and analysis unit 300 attached to an automobile.

The driver's state information acquisition and analysis unit 300 has the following configuration.

As information acquisition units, a driver operation information acquisition unit 301a, a driver first biological information acquisition unit 301b, a driver second biological information acquisition unit 301c, a driver third biological information acquisition unit 301d, and a driver fourth biological information acquisition unit 301e are included.

Moreover, as information analysis units, a driver's operation delay and turbulence analysis unit 302a, a driver's breathing and pulse-based sleep depth analysis unit 302b, a driver's eyeball-behavior-based consciousness state analysis unit 302c, a driver's posture and action analysis unit 302d, and a driver's activity amount analysis unit 302e are included. Furthermore, a driver's action history analysis device may be a watch-like device that is daily worn. In that case, partial action transition information before the driver gets on the vehicle can be used as input determination information.

The driver operation information acquisition unit 301a acquires operation information of a steering wheel, an accelerator, a brake, or the like by the driver, and the driver's operation delay and turbulence analysis unit 302a inputs these pieces of the driver operation information and generates analysis data regarding a delay and disturbance of the driver's operation.

The driver first biological information acquisition unit 301b acquires information regarding breathing and pulse that is the biological information of the driver, and the driver's breathing and pulse-based sleep depth analysis unit 302b analyzes the sleep depth of the driver on the basis of the acquired information.

The driver second biological information acquisition unit 301c acquires eyeball behavior information of the driver that is the biological information of the driver, and the driver's eyeball-behavior-based consciousness state analysis unit 302c analyzes a consciousness state of the driver on the basis of the acquired information.

The driver third biological information acquisition unit 301d acquires posture and action information of the driver that is the biological information of the driver, and the driver's posture and action analysis unit 302d analyzes the posture and the action of the driver on the basis of the acquired information.

The driver fourth biological information acquisition unit 301e acquires the biological information of the driver, and the driver's activity amount analysis unit 302e analyzes an activity amount of the driver on the basis of the acquired information.

The analysis units including the driver's operation delay and turbulence analysis unit 302a, the driver's breathing and pulse-based sleep depth analysis unit 302b, the driver's eyeball-behavior-based consciousness state analysis unit 302c, the driver's posture and action analysis unit 302d, and the driver's activity amount analysis unit 302e configured as the information analysis units generate state parameters necessary for driver's state total determination processing executed in next step S21. Examples of the state parameters vary depending on the device. The examples include a numerical value of stability of the operation of a steering, an accelerator, a brake, or the like from which the state can be determined, a Percent of Eyelid Closure (PERCLOS) related index, a sleep depth estimated from the heart rate and the breathing, an accumulated cumulative fatigue level, a sleepiness index, a fatigue level index, a frequency at which eyeballs search for a visual event, visual fixation delay characteristics, visual fixation maintenance time, or the like.

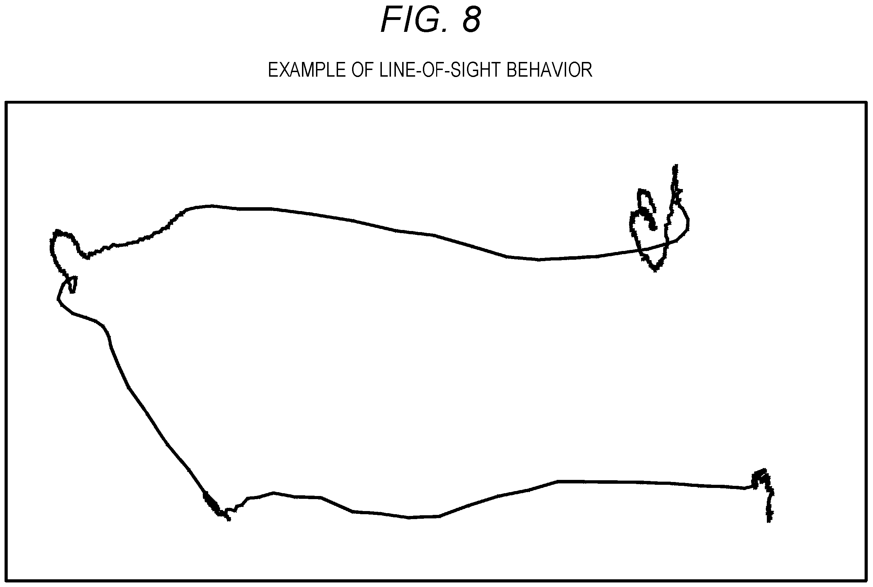

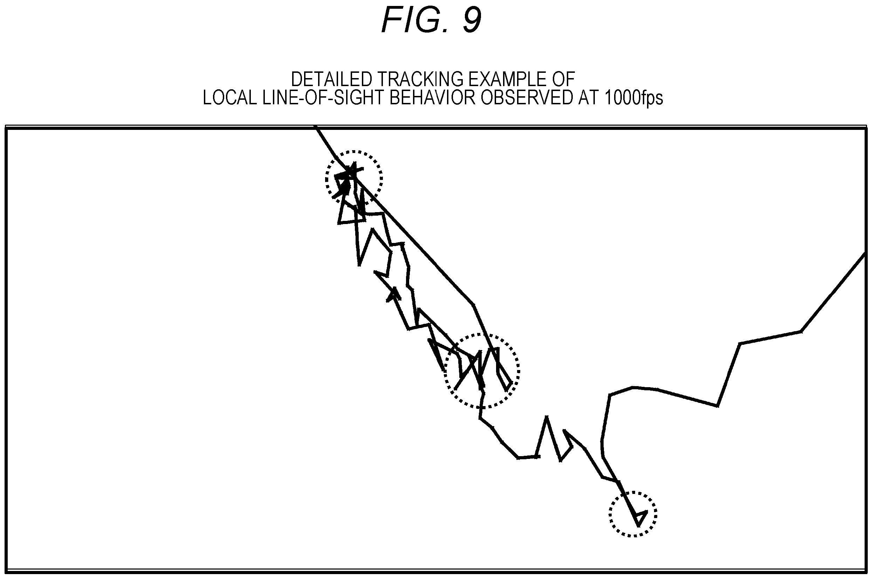

As an example of processing for analyzing the driver's state, an analysis example based on the driver's eyeball behavior information acquired by the biological information acquisition unit 301c, that is, processing for analyzing the consciousness state of the driver executed by the driver's eyeball-behavior-based consciousness state analysis unit 302c will be described with reference to Figs. 8 and 9.

Fig. 8 is an example of data obtained by analyzing the movement of the driver's eyes, that is, analyzing the behavior of the eyeballs at high speed and drawing a transition of a line-of-sight direction in one stroke. The line of sight of the driver is directed to a direction of information related to driving. At this time, the eyeball is rotated at high speed by a so-called saccade operation, visual and optical information in the direction in which the line of sight is directed is taken in the retina as light, and the visual cortex in the brain proceeds understanding of the information. Fixational eye movement in the line-of-sight direction plays a role for supplementing retinal stimulation and understanding determination. The fixational eye movement is a small movement of the eyeball that occurs in a focused direction by the eyeball behavior analysis. When recognition is completed by referring to information supplemented in a process of the fixational eye movement and the memory, processing for keeping the line of sight in the same direction is unnecessary. Then, next, a so-called saccade operation appears that moves the line-of-sight direction to surroundings and moves the line-of-sight direction to another priority confirmation event. Fig. 8 illustrates an example of a series of eyeball behaviors from the fixational eye movement and microsaccade to the movements of the line of sight to a focused direction next in a process for advancing the information recognition.

A neighborhood search range in the visual fixation of the eyeball, a search drift range at the time of fixational eye movement, a neighborhood staying time before the recognition, or the like are ranges and times defined by recognition in the brain and the series of sequences of the (reflection) action. These ranges and times vary depending on the wakefulness state of the driver. For example, when the wakefulness state of the driver is insufficient, a time delay occurs before the recognition.

Then, in this visual fixation search, a range of a search behavior such as the width and the range of the fluctuation of the eyeball before the recognition is disturbed. Moreover, even if the fixational eye movement is performed, a delay before the recognition occurs. The movement and the range at the time when the wakefulness is deteriorated or the activity in the brain is lowered change from those at the time when the wakefulness is stable.

The acquisition of the optical and visual information is not completed by taking in physical information that has been purely captured as light. Feedback with a series of stored information is repeatedly performed, for example, memory reference recognition is started on the basis of initial visual information, and information insufficient for the determination is additionally acquired. Furthermore, in a case where the visual information does not cause the reference recognition with the memory, this causes an effect such that eyes shift. Therefore, if a stable behavior state at the time of normal wakefulness of the driver is found, it is possible to estimate the wakefulness state of the driver by comparing the found state with each wakefulness level specific characteristics of the driver's eyeball behavior and performing analysis.

Fig. 9 is an example of tracking data of a local line-of-sight behavior of the driver that occurs in a short time of about 0.1 seconds observed at 1000 f/s. A range surrounded by a circle illustrated in Fig. 9 is a behavior range of the eyeball performing the visual fixation and the search. However, in a case where the number of small behaviors is small and the line-of-sight is immediately moved to a different target, an example of an eyeball behavior is indicated from which it is estimated that the determination on the individual target is made in a short time on the basis of the stored information. For example, the same portion is observed over time, the line of sight is returned to the direction when the situation determination is insufficient, and the detailed visual fixation observation is performed again.

The information recognition ability using the line of sight is largely affected by the experience and the memory of the driver. In one example, a taxi driver who drives an empty taxi shows an eyeball behavior that pays more attention to a behavior of a pedestrian on the side of the road because the taxi driver load a passenger by finding a user on the road. Regarding the staying time of the line-of-sight visual fixation when the driver looks at the traffic light, it tends to take more time to confirm the direction of the traffic light and the direction of the light of the green light depending on the state of the driver whose visual acuity is deteriorated due to fatigue or eye fatigue. Furthermore, as an experienced rally driver, in a situation in which the driver instantly determines various surrounding environment information and visually recognize the surrounding situations that change one after another, even in a case where a line of sight of a general driver is accurately directed to a specific object on the road and the visual fixation is performed, the eyeball direction is changed from the surrounding peripheral visual field to the target by the saccade in a short time, an early risk is determined, and the line of sight is frequently moved to various visual information of the next target.

As described above, in the eyeball behavior characteristics of the driver, a behavior that is directly affected by the reference recognition cycle with the memory in the brain in particular is reflected and indicated, and the driver's personal characteristics are strongly expressed. Therefore, the determination on the wakefulness is largely affected by the change in the behavior specific for the driver. One of the behaviors of the eyeballs is the movement of the central visual field. The central visual field is moved to visually determine the details of a luminance differential portion in the peripheral visual field. In the luminance differential portion, weighting for what to pay attention is unconsciously performed according to the memory and the importance of the memory. The luminance differential portion is a portion related to an event in which a gain is increased by the weighting. When the visual information having a change in the peripheral visual field is taken, it is considered that firing of neural transmission that instructs an oculomotor muscle (extraocular muscle) to cause the saccade operation for moving the line of sight occurs in the brain. The eyeball behavior response is not a unique response to the input information. The eyeball behavior response occurs as a result of suppression and promotion of intracerebral substances that cause the firing of the neural transmission.

Information regarding the wakefulness state of the driver caused in conjunction with the use of the automatic driving and the normality or abnormality according to the result can be acquired by associating the switching quality to the manual driving with the eyeball behavior characteristics that has been acquired in advance. Teacher data according to the driver's state can be learned on the basis of the acquired information. The normal switching from the automatic driving to the manual driving is considered as the result of the accurate and quick recognition behavior in the brain. On the other hand, in a case where the normal switching is delayed or failed, the response is delayed because the driver's mind is absent, the wakefulness return is insufficient, or the consciousness is lowered due to drug intake or the like. By using these pieces of information, various teacher data can be automatically generated.

Conventionally, a correlation between the eyeball behavior and the determination recognition behavior in the brain is not able to be grasped by the limited number of subjects who had a medical examination or a clinical experiment. That is, the above number is limited as the number of sample data. By using the eyeball behavior affected by the activity state in the brain as an input value of externally observable information, behavior correlation learning and long-term fluctuation tracking by using artificial intelligence under variety of conditions in a wide range can be performed through a learning device.

The wakefulness state evaluation dictionary of the automatic driving supports an important function such as the switching determination of the vehicle by the system and can secondarily calculate an activity index in the user's brain. Therefore, the wakefulness state evaluation dictionary can be used as a precursor index of the autonomic ataxia or other diseases of which symptoms appear in connection with the activity in the brain.

In the configuration according to the present invention, the wakefulness state of the driver is determined at each time point on the basis of the dictionary (wakefulness state evaluation dictionary) corresponding to each authenticated driver. Moreover, behavior transition information of each driver in the medium and long term is acquired, the evaluation value of the wakefulness state is calculated. Even if each eyeball behavior for viewing each event is observed, the eyeball behavior is strongly affected by the visual information necessary for the visual fixation depending on the target to be viewed. Therefore, it is difficult to directly estimate the wakefulness degree. However, by multi-dimensionally classifying the situations of a target to be visually recognized and analyzing the behavior by a statistical method, a change in the behavior appears as a change in extension of the visual fixation staying time, expansion of a search range, a saccade frequency, visual fixation drift, or the like with respect to those at a fixed time. By analyzing the behavior characteristics in time series, it is possible to monitor the transition of the return to the wakefulness state of the driver.

The characteristics of the eyeball behavior are determined on the basis of functional and structural anatomical elements of a human body. Therefore, the behavior is determined on the basis of the experience and the memory related to the recognition for each individual and risk memory, rather than being derived from the individual differences. However, the searches performed to understand the target by using the visual acuity or the like is added to the delay and insufficiency until the eyes are focused due to the conditions regarding the brightness, eye fatigue, or the like. Therefore, in order to determine the level of the wakefulness degree with higher accuracy, it is better to determine the wakefulness level by using a multi-dimensional dictionary in which the characteristics of the individuals are classified for each condition. Because the medium-and-long-term observation data directly received effects related to the visual recognition activity in the brain, a fluctuation specific for the driver is observed in the medium-and-long-term observation data. For example, a transition observed record such as a warning symptom of autonomic ataxia or the like is generated as a personal characteristics amount by learning to be described later as a part of dictionary creation.

In this case, as one of the procedures to monitoring the wakefulness state return temporal transition until the return of the driver to the manual driving, the eyeball behavior analysis for observing the direct effects caused by the neural transmission delay in the perceptual determination sequence in the brain has been described in detail. However, by hierarchically applying various other methods such as evaluation on an electrocardiogram by the Lorentz plot and evaluation on the breathing, evaluation on functional steering stability of the steering device, the PERCLOS evaluation, or the like, it is possible to monitor the wakefulness state transition with high accuracy in the return sequence.

### (Steps S21 and S22)

Returning to the description of the flow in Fig. 7, processing in steps S21 and S22 will be described.

In steps S21 and S22, the information processing apparatus executes the driver's state total determination processing on the basis of the state parameters input from the analysis units configured as the information analysis units including the driver's operation delay and turbulence analysis unit 302a, the driver's breathing and pulse-based sleep depth analysis unit 302b, the driver's eyeball-behavior-based consciousness state analysis unit 302c, the driver's posture and action analysis unit 302d, and the driver's activity amount analysis unit 302e.

In this processing, the wakefulness state evaluation dictionary corresponding to the driver generated according to the flow described with reference to Fig. 6 is used.

In steps S21 and S22, the driver's wakefulness state determination processing and the processing for estimating the time needed before the return to the manual driving (delay time) are executed on the basis of the observation value at that time.

### (Steps S23 and S28)

In step S23, necessity of the return to the manual driving is determined. In a case where the return to the manual driving is not needed, processing in and after step S24 is executed. In a case where the return to the manual driving is needed, processing in step S28 is executed.

In step S23, on the basis of the latest data of the local dynamic map (LDM) including the information regarding the road where the vehicle is currently traveling, prior to the situation in which the driver enters a next section of which a driving return intervention level is different, in a case where a time when a manual driving return success rate reaches a predetermined success rate approaches on the basis of the return time obtained in step S22, it is determine that the return to the manual driving is needed, and the procedure proceeds to step S28. When the time reaches the return start time, a warning and a notification are issued, and a return sequence to the manual driving is started.

### (Step S24)

In a case where it is determined in step S23 that the return to the manual driving is not needed at the current time, the observation interval is reviewed in step S24.

That is, on the basis of the acquired wakefulness observation information of the driver, a grace time before the return and a grace time before the state is reconfirmed are confirmed in total, and regular observation intervals are adjusted as necessary. A new observation interval is set by adjusting a repeat observation waiting standby timer 310 for each observation device.

The monitoring frequency is reset for each device, for example, at a timing when the driver takes a nap in a nap space in a long-distance section of the maintained highway on which automatic driving can be continuously performed.

The roads are, for example, divided into a region where the automatic driving can be performed and a region where the manual driving is needed. However, the states of these sections are not fixed, and are sequentially changed due to the weather, the disasters, the occurrence of the congestions and accidents, or the like. Therefore, when an unexpected early return point (= switching point from automatic driving to manual driving) is generated due to an environmental change or the like, the driver needs to start the manual driving in a shorter time than a plan in which the driver does not need to drive at the moment. For example, in such a case, it is necessary to observe the return transition, and it is necessary to observe the series of state transitions, for example, awakening, standing up, seating on the driver's seat, visually recognizing the situation, and determining the situation necessary for steering.

In other words, the state with rapid change is observed, it is necessary to shift a time interval of a normal monitoring at the time of nap to a short interval of monitoring, and the depth of the sleep is estimated on the basis of the passive heart rate and breathing evaluation for more accurate estimation. Therefore, by analyzing the details of the eyeball behavior of the driver at the stage close to the completion of the switching, the recognition determination action ability is estimated at sub-millisecond intervals, and it is necessary for the system to determine whether or not the driver can cope with the event by performing the manual driving on the basis of the observable information.

In step S24, the interval of the observation by each observation device is set according to the state of the driver, and the driver's state information acquisition and analysis unit 300 performs monitoring observation at the optimized frequency.

### (Step S25)

In a case where there is a grace time before the next observation standby time, in step S25, the interval of the observation of the driver's state and the observation device are reviewed, and an observable vital signal is continuously observed.

### (Step S26)

In step S26, the fluctuation in the observation value indicating the behavior characteristics and the wakefulness degree of each driver in the medium and long term is relatively compared with recorded reference values that are stored and saved in the past and evaluated.

There is a case where the behavior characteristics of the driver change with time. For example, in a case where the driver is accustomed to driving and the tension is released, the behavior characteristics of the driver may change due to occurrence of an accident, event experience that does not cause an accident, or the like. By grasping the change from the behavior characteristics of the driver in the past, for example, a baseline behavior change and using the medium-and-long-term analysis results, it is possible to correct the accuracy when the return delay time is estimated on the basis of the observation value.

Note that, by using the medium-and-long-term analysis results, it is possible to detect a delay of the neural transmission or the like due to an unbalance between the sympathetic nerve and the parasympathetic nerve that is a precursor symptom of autonomic ataxia of the driver, and it is possible to grasp occurrence of an abnormality such as recognition determination delay or the like at an early point.

### (Step S27)

Step S27 is processing for learning the behavior characteristics of the driver that is obtained at each travel to be baseline information of the medium-and-long-term characteristics fluctuation and processing for generating and updating the dictionary on the basis of the learning result.

The learning processing and the dictionary may be saved each time the event occurs, and for example, setting may be used in which the learning data and the dictionary are saved in a remote server in association with the driver by summarizing the results saved in a local memory of the automobile for each itinerary.

Note that the frequency for observing the vital signal of the driver varies depending on the situation, and it is desirable to comprehensively evaluate the frequency depending on the situation. For example, a user who tends to have Sleep Apnea Syndrome has a high Apnea Hypopnea Index (AHI) coefficient and a high risk of feeling sleepiness. Even in such a case, the driver has a high risk of suddenly feeling sleepiness. Therefore, detection is performed on the basis of the monitoring of the change in a short term and event driven detection. On the other hand, a change caused by the deterioration in the recognition determination such as gradually progressing fatigue, dysfunction, or the like hardly appears in the vital signal and the observed behavior of the driver as a short-term change. It is necessary to observe the change in a long term in order to detect and determine the fatigue or the like. The short-term change triggers emergency event measures executed by the AEBS on the system against sudden steerability loss such as myocardial infarction or stroke. The medium-term change detection is used as a trigger signal for the system to perform a function for reducing a risk caused by the deterioration in the recognition determination ability due to fatigue, sleepiness, or the like.

By mainly monitoring a long-term change in characteristics of a driving steering response each time the vehicle is used, it is possible to grasp fluctuations in optic nerve reaction and a physical functional ability of the driver. In particular, a delay amount of a reflection operation in a shorter time than a normal use at the usual time and a sudden correction operation to compensate the delay of speed of a repeating operation or a steering correction operation with respect to a steering operation necessary for the driving of the vehicle are detected. Then, by comparing changes in different use periods (learning history values), it is possible to capture a long-term change in a neural transmission path of any one of a current recognition, determination, and prevention actions of the driver.

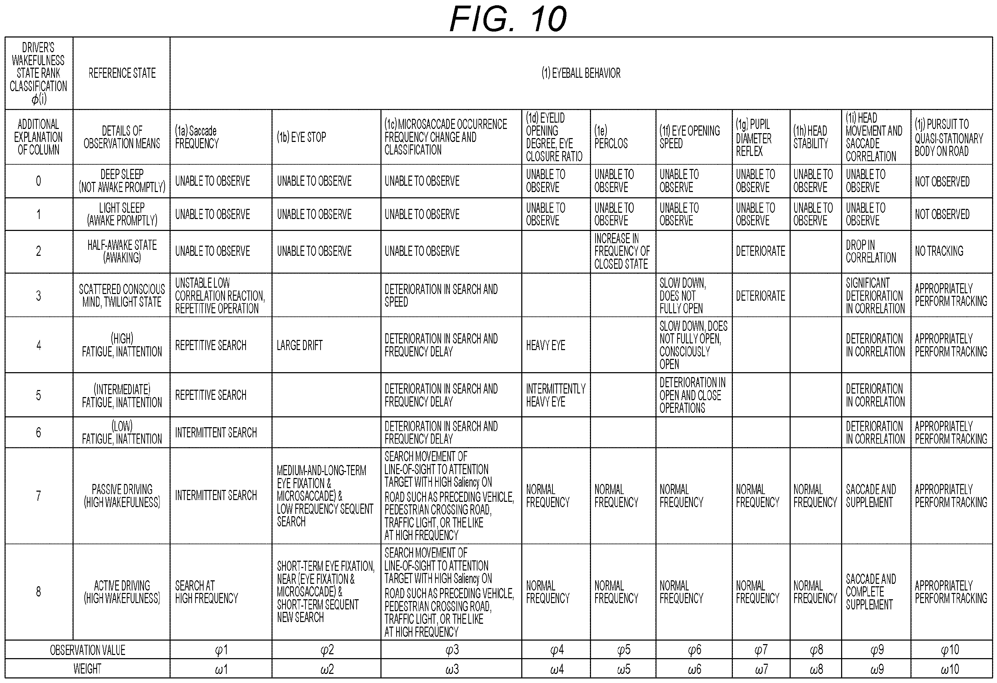

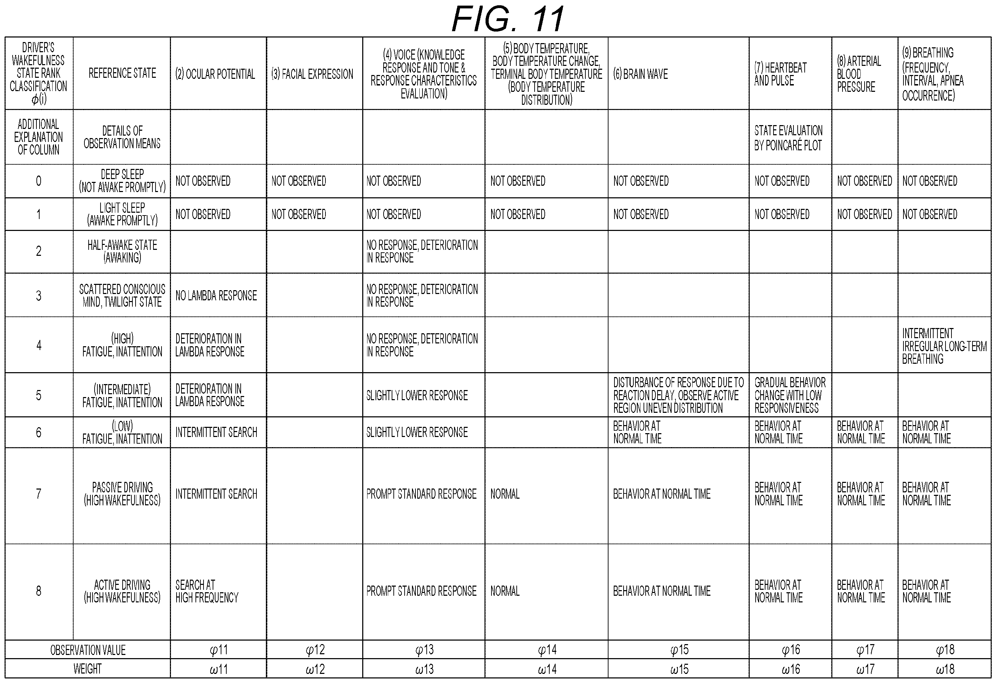

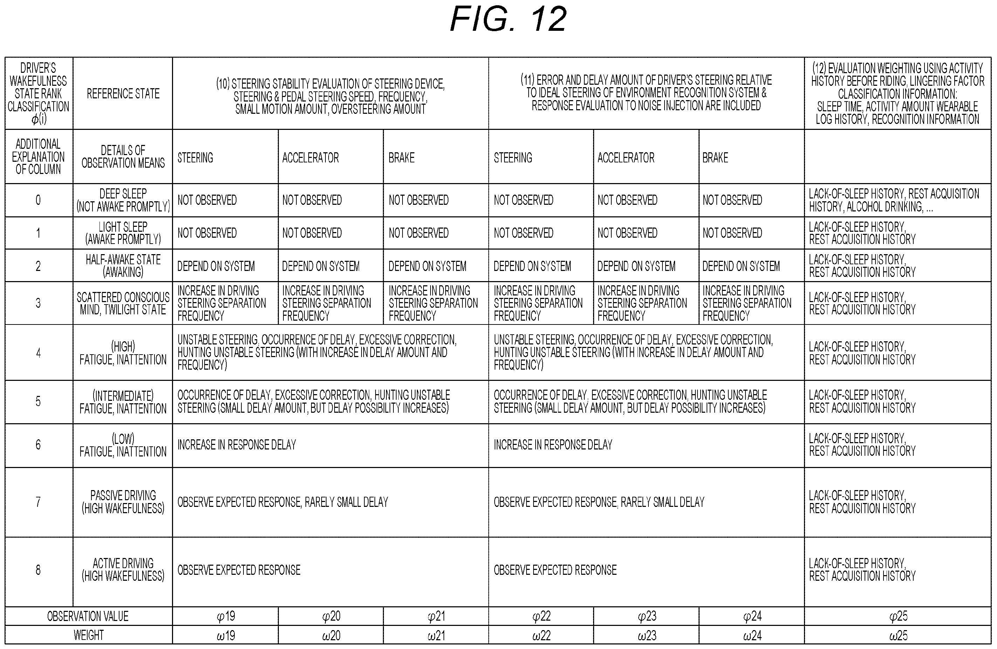

Figs. 10 to 12 illustrate exemplary configurations of the wakefulness state evaluation dictionary corresponding to the driver to be generated and updated according to the processing described with reference to Figs. 6 and 7.

All data illustrated in Figs. 10 to 12 indicates a part of configuration data of the wakefulness state evaluation dictionary corresponding to the driver. As illustrated in the leftmost columns in Figs. 10 to 12, a wakefulness state rank of the driver is set to nine categories of zero to eight.

The wakefulness state rank 0 is the lowest wakefulness state and is a level corresponding to deep sleep.

The wakefulness state rank 9 is the highest wakefulness state and is a level corresponding to a wakefulness state in which active driving, that is, normal manual driving can be performed.

In Figs. 10 to 12, (1) to (12) that are driver's information (biological information and operation information) that can be obtained from the driver are individually illustrated. These information is the information (observable value) acquired by the above-described driver's state information acquisition and analysis unit 300 illustrated in Fig. 7.

Figs. 10 to 12 illustrate following observable information.
(1) Eyeball behavior
(2) Ocular potential
(3) Facial expression
(4) Voice (knowledge response and tone & response characteristics evaluation)
(5) Body temperature, body temperature change, terminal body temperature (body temperature distribution)
(6) Brain wave
(7) Heartbeat and pulse
(8) Arterial blood pressure
(9) Breathing (frequency, interval, apnea occurrence)
(10) Steering stability evaluation of steering device, steering & pedal steering speed, frequency, small motion amount, oversteering amount
(11) Error and delay amount of driver's steering relative to ideal steering of environment recognition system & response evaluation to noise injection are included
(12) Evaluation weighting using action history before riding, lingering factor classification information: sleep time, activity amount wearable log history, recognition information

Note that (1) Eyeball behavior is subdivided into observation information including:
(1a) Saccade frequency
(1b) Eye stop
(1c) Microsaccade occurrence frequency change and classification
(1d) Eyelid opening degree, eye opening ratio
(1e)PERCLOS
(1f) Eye opening speed
(1g) Pupil diameter reflex
(1h) Head stability
(1i) Head movement and saccade correlation
(1j) Pursuit to quasi-stationary body on road

These pieces of information (1a) to (1j) to (12) are information (observable value) acquired by the above-described driver's state information acquisition and analysis unit 300 illustrated in Fig. 7.

The information processing apparatus generates a dictionary (wakefulness state evaluation dictionary) specific for the driver on the basis of the acquired information and sequentially updates the dictionary. Furthermore, the wakefulness degree of the driver is evaluated by using the dictionary. That is, it is determined which one of the levels zero to nine corresponds to the wakefulness degree level of the driver on the basis of the observed value of the driver.

Note that the driver's state information acquisition and analysis unit 300 acquires the observation values of (1a) to (12) illustrated in Figs. 10 to 12 as observation values φ1 to φ25 illustrated in the lower portions of Figs. 10 to 12.

At the time of wakefulness degree determination processing, a wakefulness degree evaluation value is calculated according to, for example, the following arithmetic expression (Expression 1) by using the observation values φ1 to φ25.Wakefulness degree evaluation value = Σφ (i) ·ω (i)

Note that the reference i is an index, and i = 1 to 25 are respectively satisfied for (1a) to (12) as illustrated in the lower portions of Figs. 10 to 12.

The value ω (i) is a weight value corresponding to each observation value φ (i).

Note that, by setting the weight value to a value specific for each driver, a wakefulness degree level specific for each driver can be calculated. As the weight value, for example, a value calculated from the result of monitoring the driver is used.

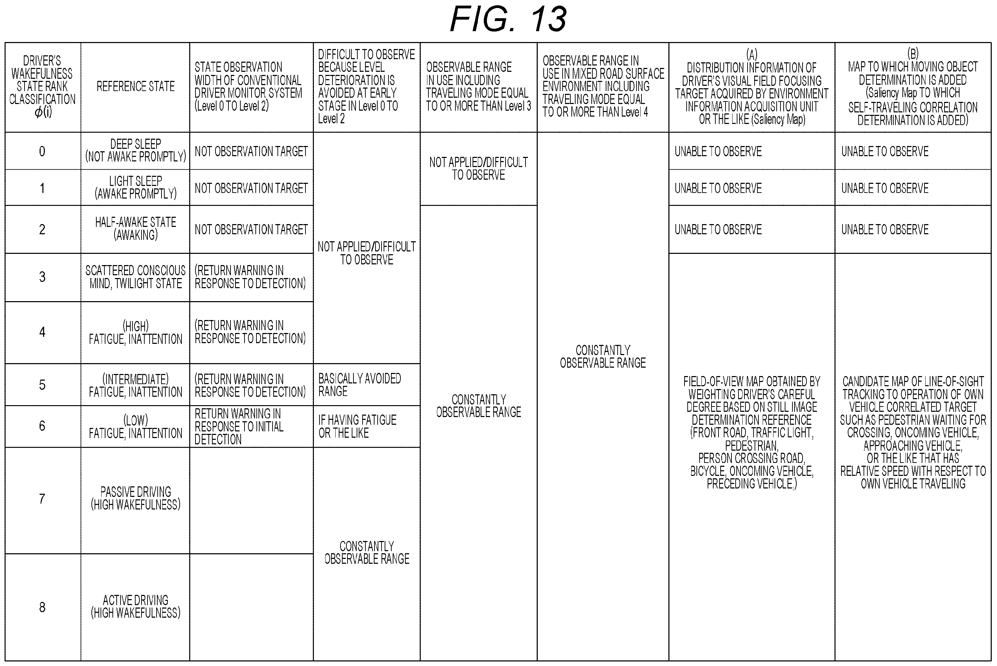

Note that the wakefulness state evaluation dictionary may set to include data illustrated in Fig. 13 in addition to the generation information and the operation information of the driver described with reference to Figs. 10 to 12. That is, as illustrated in Fig. 13,
(A) Distribution information of driver's visual field focusing target acquired by environment information acquisition unit or the like (Saliency Map)
(B) Map to which moving object determination is added (Saliency Map to which self-traveling correlation determination is added)

It is possible to have a configuration including these pieces of data.

Moreover, as illustrated in Fig. 13, a configuration may include definition of observable data according to an automatic driving level in automatic driving definition of the Society of Automotive Engineers (SAE) .

A dictionary configuration in Figs. 10 to 12 illustrated as an example of the wakefulness state evaluation dictionary is an example of a data structure. A multivariable correlation relationship is automatically or semi-automatically generated by self-learning in association with driving switching quality by using the artificial intelligence, and autonomous learning is performed within a range allowed for self-learning calculation of the system, thereby it is possible to improve composite factor determination performance. It is possible to perform learning while self-selecting the teacher data because the switching quality is evaluated at each switching event in the automatic driving. The dictionary for each driver is practically and widely learned without data collection by a third party and labeling only by performing a learning function at the time of switching during the automatic driving. Details will be described later.

### [4. (First Embodiment) Embodiment for Performing Control Based on Driver Monitoring (Control Processing Example in a Case of SAE Definition Levels 1 and 2)]

Next, an embodiment for performing control based on driver monitoring will be described as a first embodiment of processing executed by a moving device according to the present invention, an information processing apparatus included in the moving device, or a server that communicates with these devices.

The first embodiment described below is a control processing example in a case where automatic driving at about automatic driving levels 1 and 2 in the automatic driving definition of the Society of Automotive Engineers (SAE) is performed.

In the automatic driving at about levels 1 and 2, a driver is not allowed to be completely separated from a driving steering loop, and only partial separation is allowed. Specifically, it is not necessary for the driver to operate an accelerator, a brake, or a wheel steering, and it is only required for the driver to operate the steering wheel at the time when a sudden event occurs without releasing hands from the steering wheel. However, even in such a state, there is a possibility that the driver is suddenly unable to operate the steering wheel. In such a case, it is possible to prevent an accident by applying the configuration according to the present invention.

As described above, in recent years, a large number of accidents have been caused by the deterioration in attention and sleepiness of the driver, sleepiness caused by the apnea syndrome, or sudden disease such as heart attack, cerebral infarction, or the like. According to this situation, efforts to prevent these accidents by monitoring the driver's state are made. In particular, it is considered to install a driver monitoring system to a large vehicle that has a large possibility of causing a serious accident. The configuration of the present invention makes it possible to acquire the state of the driver of the automobile in real time and immediately perform optimum control at the time when the state is abnormal. Specifically, for example, in a case where a risk such that the driver is separated from normal steering control is detected on the basis of detection information (monitoring information) of the driver's state, control for emergently stopping the vehicle is performed, and a serious accident is prevented.

The driver's state is determined on the basis of the vital signal that can be acquired from the driver. For example, PERCLOS (eye opening ratio) evaluation that analyzes an orientation of the face of the driver and the state where the eyes are closed, processing for determining a state from heartbeat characteristics and a breathing signal, or the like of the driver, or the like is used.

Note that, regarding the acquired vital signal, an abnormality is appropriately determined on the basis of a predetermined threshold, and analysis to which a steering operation characteristics change in a time range such as several minutes to several tens minutes in terms of time is added is executed in parallel. Then, an accident is prevented by detecting physical and functional abnormalities of the driver and calling for attention and emergently stopping the vehicle.

In the present embodiment, steering stability information of the steering, the accelerator, the brake, or the like of the vehicle by the driver is acquired in addition to the observable biological information of the driver, transition and correlation of these pieces of information are analyzed, and the driver's state is analyzed in an immediate and long-term span. The long-term analysis information is used to estimate the mental state of the driver.

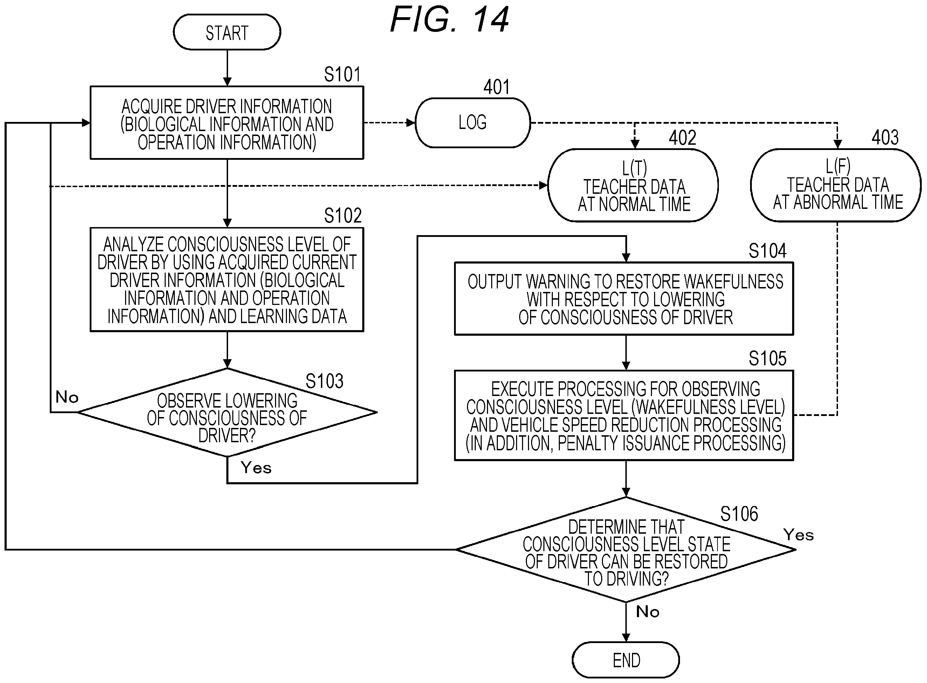

A control sequence according to the first embodiment will be described with reference to the flowchart illustrated in Fig. 14. As described above, when the automatic driving levels 1 and 2 are used, the driver is not allowed to be completely separated from the driving steering loop. When the automatic driving level 2 is used, for example, a situation, such as an emergency, in which the driver needs to start the manual driving may occur. However, there is a case where the driver is not in a state where normal manual driving can be performed For safe driving, it is necessary to switch the automatic driving mode to the manual driving mode after it is confirmed that the driver is in a state where normal manual driving can be performed. For driver's state determination processing for determining whether or not the driver is in a state where normal manual driving can be performed, for example, observation information of the deterioration of a consciousness state that causes the driver to separate from a steering work can be used.

Note that when the automatic driving levels 1 and 2 that are not the complete automatic driving are used or when a vehicle having a driving assistance system is used, it is necessary for the driver to at least operate any one of the steering wheel, the brake, or the like. Therefore, the system can continuously monitor steering validity of a device to be operated by the driver and can continuously determine the consciousness state of the driver. For example, when the driver is using a lane keep assist system that makes a vehicle automatically travel in a specific lane of a road, the driver needs to continuously control at least the accelerator and the brake. Furthermore, while an auto-cruise control (ACC) is used, the driver needs to control the steering wheel. By monitoring these operations of the driver, the consciousness state of the driver can be continuously determined.

The flowchart illustrated in Fig. 14 is a control processing sequence, for example, in a case where driving using the automatic driving levels 1 and 2 of the SAE definition or the vehicle having the driving assistance system is performed. That is, a flow used to describe a control sequence in a case where a part of travel control of the automobile is automatically controlled and a part of the travel control is performed by the driver, specifically, for example, in a case of driving in which the accelerator and the brake are automatically controlled and the automatic control and the control by the driver are used to control the steering wheel that is a control sequence in a state where the driver is performing some driving operation.

Processing in each step in the flow illustrated in Fig. 14 will be sequentially described.

Note that, in the description of the flow in Fig. 14 and the subsequent drawings, processing in each step in the flow is executed by the moving device according to the present invention, the information processing apparatus included in the moving device, or the server that communicates with these devices. However, in the following description, for simplification of the description, an example will be described in which the information processing apparatus executes the processing in each step.

### (Step S101)

The information processing apparatus according to the present invention monitors the driver's state in step S101. The driver's state to be monitored includes the biological information of the driver, the operation information of the automobile by the driver, for example, operation information of a steering wheel or the like.

These pieces of monitoring driver information are sequentially stored in the storage unit as a log 401.

### (Step S102)

Next, in step S102, the information processing apparatus analyzes current driver information (biological information and operation information) based on the acquired log and the wakefulness degree (consciousness level) of the driver using learning data that has been acquired in the past. The information processing apparatus includes a learning processing unit (learning device) that performs learning processing based on the log of the driver's state information, and a storage unit of the information processing apparatus stores a learning data dictionary generated as a learning result.

In step S102, the information processing apparatus observes whether or not response characteristics of the driver fluctuate in a long term on the basis of the learning data dictionary corresponding to the driver that has been generated by the learning processing in the past.

Note that, when a driver starts to drive an automobile, the information processing apparatus executes authentication processing and personal identification. The information processing apparatus stores the learning data such as the log corresponding to the identified driver or the like in the storage unit and acquires the learning data dictionary corresponding to the driver from the storage unit.

In step S102, whether or not the response characteristics of the driver fluctuate in a long term is observed on the basis of the learning data dictionary generated on the basis of the learning data such as the log in the past corresponding to the driver, that is, the wakefulness state evaluation dictionary corresponding to the driver. Specifically, for example, whether or not the response characteristics of the driver fluctuate in the long term is observed on the basis of a learning data dictionary of cumulative actions created by an action learning device specific for the identified driver. Note that, although the cumulative action learning is described above, cumulative integration may be simply evaluated. By performing the cumulative classification according to the situation, it is possible to determine the state more accurately depending on the classified action. For example, action characteristics of the driver's environmental recognition largely change according to the situation, such as action characteristics in sunset hours, action characteristics in midnight traveling, action characteristics according to accumulated fatigue due to continuous traveling, action characteristics in daytime traveling in rainy weather, action characteristics in backlight traveling in urban area in the nighttime, action characteristics in traveling on winding roads, or the like.

In these classification action learning, it is possible to perform learning by using existing classification. However, there are actually individual drivers, various traveling environments, and combinations thereof. By performing self-classification learning by artificial intelligence on the basis of the input information of the system, classification that can be applied to various situations is performed so that optimization according to the situation can be performed, such as characteristics specific for the driver, motion characteristics of the vehicle or the loaded cargo.

It is difficult to make a determination by using the learning device and the dictionary because the learning device and the dictionary do not have sufficient classified actual values in the initial use. However, determination accuracy improves as a use period extends. These classifications are made because it is possible to express and reflect a difference in the visual acuity due to the age of the driver, a difference in focus adjustment ability according to the brightness environment, a difference in mind in carefulness in traveling according to the load or in careful traveling depending on traveling experience in the past. Therefore, the action appears as individual characteristics. For example, in a case where driving of the private vehicle on commuting by the same driver is compared with driving of a large freight vehicle by the specific driver, the line-of-sight and the operation characteristics are changed after shifting to steering of a large tractor that is driven for business. A surrounding confirmation operation, operation and braking start point, a distance, or the like of the driving after the cargo are loaded, or in addition, a tracking vehicle is coupled are largely different from traveling steering characteristics at the time when no cargo is loaded. It is possible to determine the state estimation of the driver with high accuracy from an observable evaluation value of the driver by performing situation-classification-type learning including these environments. When the classification is not completed or return characteristics under different conditions from observable biological information of the driver are uniformly added without considering the vehicle information and the environmental conditions, the return characteristics are widely distributed, and accuracy of the determination is damaged.

### (Step S103)

Next, in step S103, it is determined whether or not the wakefulness degree (consciousness level) of the driver is deteriorated. When the deterioration in the wakefulness degree (consciousness level) is not observed, the procedure returns to step S101 to continue the observation of the driver's state, and the processing in and after step S101 is repeated. That is, a driver's state log with time is continuously taken and recorded in the storage unit.

The processing for determining whether or not the wakefulness degree (consciousness level) of the driver is deteriorated is executed by using analysis results of the wakefulness degree (consciousness level) acquired in step S102. Next, in step S102, processing is executed for analyzing the current driver information (biological information and operation information) based on the acquired log and the wakefulness degree (consciousness level) of the driver using learning data that has been acquired in the past. The learning processing unit (learning device) executes processing for generating a dictionary that registers a threshold with which the deterioration in the consciousness of the driver can be determined (wakefulness state evaluation dictionary used to determine wakefulness degree (consciousness level)) according to the analysis result and for storing the dictionary in the storage unit. Note that this dictionary is specific for the identified driver. In a case where the dictionary that registers the threshold with which the deterioration in the consciousness corresponding to the current driver (wakefulness state evaluation dictionary) exists in the storage unit, the determination is made on the basis of the threshold registered in this dictionary. However, in a case where the dictionary corresponding to the driver does not exist in the storage unit, the determination is made by using thresholds for determination that are registered in a generalized dictionary generated by statistical processing based on the wakefulness state evaluation dictionary corresponding to a large number of drivers.

Note that, in a case where the deterioration in the wakefulness degree (consciousness level) of the driver is not observed in step S103, the processing in and after step S101 is continued, and the learning processing unit (learning device) records a driver's state log at this point as teacher data at the normal time L(T)402 indicating a normal state in the storage unit.

On the other hand, in a case where it is determined in step S103 that the wakefulness degree (consciousness level) of the driver is deteriorated, the procedure proceeds to step S104.

### (Step S104)

In a case where it is determined in step S103 that the wakefulness degree (consciousness level) of the driver is deteriorated, the information processing apparatus issues a notification to the driver, for example, by issuing attention warnings, applying haptics vibration to a seat, applying a signal to a steering wheel and prompts the driver to return to driving steering so as not to further deteriorate the consciousness deterioration level from that at the current time in next step S104.

When the deterioration in the consciousness of the driver advances and temporary traveling depending on the Advanced Driver Assistance System (ADAS) function including a lane separation warning or the like of the own vehicle is continued, it is not possible for the driver to return to driving during the temporary traveling, and an automatic driving system of the ADAS causes a situation that directly causes an accident in a case where the automatic driving system exceeds limit of automatic steering in a situation where no valid operator exists. Therefore, this situation is extremely dangerous. Therefore, it is necessary to take various measures to prevent the deterioration in the consciousness of the driver in the systems up to level 2. There is no need to limit a method for preventing the deterioration in the consciousness of the driver and the intervention.

### (Step S105)

Moreover, in step S105, the information processing apparatus continuously observes the consciousness deterioration level (wakefulness level) of the driver and starts vehicle speed reduction processing and driving control forced termination processing by automatic driving. Moreover, penalty issuance processing may be executed. An original purpose of the penalty is not to impose a penalty to a user, and the penalty is a function provided to avoid the reception of the penalty by the driver in advance. Therefore, it is effective to gradually impose penalties. The system assumes that the wakefulness state of the driver is determined to permit or refuse the usage. For example, when a user sets to activate follow traveling setting at the level 2 to a driver who tends to violate rules, a risk occurs such that the driver is not able to cope with the use in a state where the wakefulness is deteriorated by a function other than the follow traveling. Therefore, it is useful to refuse the use in a state where the driver's consciousness is deteriorated to a certain level. At the time of determination, by increasing a threshold used to determine whether the wakefulness degree of the driver is sufficient or insufficient, the penalty limiting the usage may be imposed. By increasing the threshold used to determine the wakefulness state, the use of the function of which the wakefulness degree (consciousness level) of the driver is deteriorated is limited. There are various measures to cope with the penalty, the penalty may be a penalty for violation in use. Examples of the measures include to lower the maximum cruising speed, to forcibly guide to a service are, to limit use of the vehicle, or the like.

Note that the automatic driving level 2 in the SAE definition is a level at which the driver is responsible for controlling the vehicle. In a case where the driving control forced termination processing by the automatic driving is started in step S105, the driver needs to control the vehicle. That is, the driver starts the manual driving.

Moreover, in a case where the procedure reaches step S105, a driver's state log immediately before that time is a log indicating the deterioration in the wakefulness degree (consciousness level) of the driver. Therefore, the learning processing unit stores this log data in the storage unit as teacher data that is precursor data of the deterioration in the consciousness, that is, teacher data at the abnormal time L(F)403.

### (Step S106)

Next, the information processing apparatus analyzes in step S106 operation information of the manual driving started in step S105, verifies whether or not the driver performs a normal driving operation, and determines whether or not the wakefulness degree (consciousness level) state of the driver is at a level at which the driver can return to driving on the basis of the verification result.

In a case where it is determined that the wakefulness degree (consciousness level) state of the driver is at the level at which the driver can return to driving, the procedure returns to step S101, and the transition of the driver's state is continuously observed, and the processing in and after step S101 is repeated. On the other hand, in a case where it is determined that the wakefulness degree (consciousness level) state of the driver is not at the level at which the driver can return to driving, processing for reducing the vehicle speed and processing for stopping the vehicle are executed, and the processing is terminated.

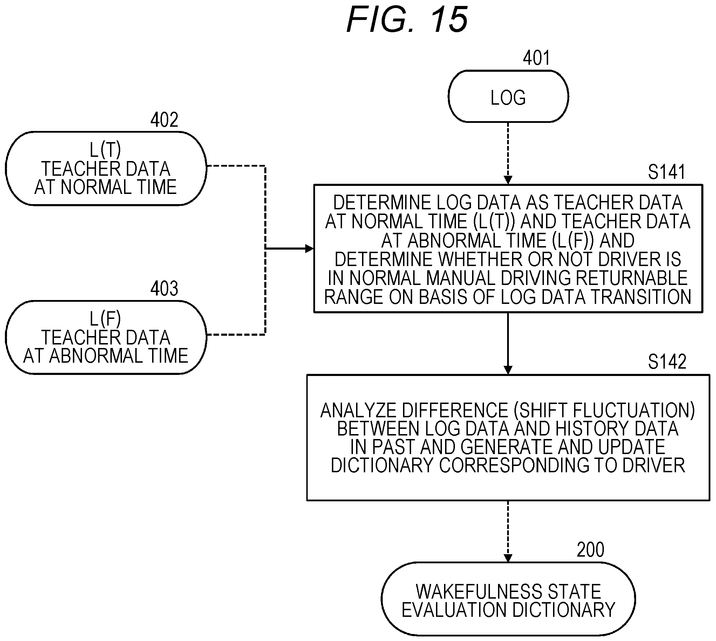

Next, a sequence of learning processing executed by the learning processing unit of the information processing apparatus will be described with reference to the flowchart in Fig. 15.

The learning processing unit of the information processing apparatus inputs or generates these pieces of data
(a) the log 401 acquired in step S101 in the flow illustrated in Fig. 14
(b) teacher data at the normal time L(T)402 acquired in a case where it is determined as No in the determination in step S103
(c) the teacher data at the abnormal time L(F)403 acquired in step S105.

The log 401 acquired in step S101 in the flow includes the biological information of the driver, the operation information of the automobile by the driver, for example, driver's state information (monitoring driver information) including steering wheel operation information or the like.

Furthermore, the teacher data at the normal time L(T)402 acquired in a case where it is determined as No in the determination in step S103 is data of the driver's state log at this time recorded by the learning processing unit in the storage unit as the teacher data at the normal time L(T)402 indicating a normal state in a case where the deterioration in the wakefulness degree (consciousness level) of the driver is not observed in step S103.

Moreover, the teacher data at the abnormal time L(F)403 acquired in step S105 is data in which the driver's state log immediately before step S105 is determined as a log indicating the deterioration in the wakefulness degree (consciousness level) of the driver and the learning processing unit stores the log data as teacher data that is the precursor data of the deterioration in the consciousness, that is, the teacher data at the abnormal time L(F)403 in the storage unit in a case where the deterioration in the wakefulness degree (consciousness level) of the driver is observed in step S103 and the procedure proceeds to step S105.

The learning processing unit executes the processing according to the flow illustrated in Fig. 15 by using these pieces of data and executes processing for updating the wakefulness state evaluation dictionary 200 indicated at the end of the flow in Fig. 15.

The wakefulness state evaluation dictionary 200 is a dictionary used to determine a degree of the deterioration in the wakefulness degree (consciousness level) of the driver.

Processing in each step in the flow in Fig. 15 will be described.

### (Step S141)

The learning processing unit (learning device) of the information processing apparatus first determines, in step S141, which one of the teacher data at the normal time (L(T)) that has been generated in the learning processing in the past and stored in the storage unit and the teacher data at the abnormal time (L(F)) the log 401 acquired in step S101 in the flow in Fig. 14 is similar to and determines whether or not the driver is in a normal manual driving returnable range on the basis of a log data transition.

The processing for determining whether or not the driver is in the normal manual driving returnable range on the basis of the log data transition is executed, for example, as follows.

When log data indicating the current driver's state is close to the teacher data at the normal time (L(T)) that has been generated in the learning processing in the past, it is determined that the driver is in the normal manual driving returnable range.

On the other hand, the log data indicating the current driver's state is close to the teacher data at the abnormal time (L(F)) that has been generated in the learning processing in the past, it is determined that the driver is not in the normal manual driving returnable range.

### (Step S142)

Next, in step S142, the learning processing unit (learning device) of the information processing apparatus analyzes a difference (shift fluctuation) between the log data and the history data in the past and updates the determination dictionary corresponding to the driver, that is, the wakefulness state evaluation dictionary 200.

Specifically, the processing in steps S141 and S142 executes, for example, the following processing.

In step S141, classification processing is executed that indicates the log data indicating the driver's state is close to the teacher data at the normal time (L(T)) indicating a previous state at the time of the wakefulness when the wakefulness degree (consciousness level) is not deteriorated or close to the teacher data at the abnormal time (L(F)) indicating a previous state at the time when the wakefulness is deteriorated that is a state where the wakefulness degree (consciousness level) is deteriorated.

In step S142, by using the classification result in step S141, processing for updating the determination dictionary having data indicating the deterioration risk (= wakefulness deterioration risk) of the wakefulness degree (consciousness level) specific for the driver, that is, the wakefulness state evaluation dictionary 200 is executed.

By executing the processing in steps S141 and S142, a dictionary reflecting the individual characteristics based on the repeat learning is created. For example, a learning dictionary reflecting a log of behavior characteristics specific for the driver at the state steady time (wakefulness state evaluation dictionary) is generated. By applying this dictionary, it is possible to perform the analysis based on the long-term fluctuation specific for the driver in step S102 described above with reference to Fig. 14. For example, in a case where the reflection characteristics delay due to a neurological disorder such as autonomic ataxia of the driver, it is possible to detect the delay as long-term behavior fluctuation by constructing a detailed behavior dictionary generated by using individual characteristics.

In this way, from determination of the state which is classified according to the condition and the observation of the long-term state change observed for each class, in the example of the present embodiment, it is possible to observe a delay in a reflection response caused by imbalance between the actual sympathetic nerve and parasympathetic nerve of the driver, and it is possible to convert the delay as a reflection index of the user.

In the present embodiment, by associating the monitoring history of the driver and return quality when the deterioration of the wakefulness degree is observed and perform section classification learning, it is possible to analyze a detailed state section on the basis of the individual characteristics of the driver. Then, the dictionary data is brushed up and updated by accumulatively learning the driver characteristics by repeatedly using the vehicle. With this update, accuracy of the determination in step S102 of the flow in Fig. 14 is increased, and it is possible to capture a long-term behavior fluctuation.

In general, before being the autonomic ataxia, the imbalance between the sympathetic nerve and the parasympathetic nerve starts to affect a perceptual determination reflection reaction. Therefore, processing before the perceived information is recognized and determined is delayed, and unstable operations such as an increase in a turning width of the steering wheel or the like increases in order to recover hesitation of steering or delay in steering. As a result, it is possible to detect modulation in steering feeling.

In the device according to the present invention, by having the function for analyzing the detailed eyeball behavior of the driver, it is possible to observe saccade, microsaccade, visual fixation, and drift, and it is possible to directly observe a perceptual reaction in the brain of the driver from the behavior transition. In particular, even in a traveling section of which a main use frequency of the vehicle is about the level 1 or 2, a vehicle that is designed as assuming that traveling at a higher level is performed on the highway in which the environment is maintained can provide a driver's mental state observation unit that is sufficiently valid in a use environment in which the level of the automatic driving is the level 1 or 2, even in the times when a driver's state observation and recognition device that assumes traveling at the level 3 or 4 is mounted and the automatic driving is performed in various environments in the society.

In this way, the data processing unit of the information processing apparatus according to the present invention analyzes the behavior of at least one of the eyeballs or the pupils of the driver, evaluates the wakefulness degree of the driver by applying the behavior analysis result and the wakefulness state evaluation dictionary that has been generated in advance and is specific for the driver, and further calculates a perceptual transmission index of the driver. As the analysis of the behavior of the eyeball, for example, analysis of saccade, microsaccade, drift, fixation, or the like is included. Here, the perceptual transmission index is an evaluation value indicating a mental state obtained from the observable evaluation value that affects visual recognition determination by the driver.

### [5. (Second Embodiment) Embodiment for Performing Control Based on Driver Monitoring (Control Processing Example in a Case of SAE Definition Level 3 or Higher)]

Next, an embodiment for performing control based on driver monitoring will be described as a first embodiment of processing executed by a moving device according to the present invention, an information processing apparatus included in the moving device, or a server that communicates with these devices.

The first embodiment described below is a control processing example in a case where automatic driving at about automatic driving levels 3 or higher in automatic driving definition of the Society of Automotive Engineers (SAE) is performed.

In the automatic driving at the automatic driving level 3 or higher, the driver can separate from almost all the driving operations. In a travel possible section at the level 4, for example, the driver can take a nap.

However, in a case where a section in which the driver is required to return to the driving work or there is a possibility of that is generated on a route due to an unexpected event that has not been predicted before entering the section, there is a case where the driver is not able to cope with the case. For example, in a case where the driver does not notice a warning (alarm) even when the warning (alarm) is output and the driver is not able to return to the manual driving due to occurrence of a sudden disease of the driver, the system is not able to cope with the case by using automatic steering, and this causes an accident. The embodiment described below is an embodiment that can prevent occurrence of such an accident in advance by constantly monitoring the driver's state.

In this way, automatic driving travel under continuous attention is permitted in the travel possible section at the level 3. Furthermore, by performing complete automatic driving travel in the travel possible section at the level 4, reduction in the attention to the driving is permitted in the travel. However, in reality, at the time of the automatic driving travel at the level 3, in consideration of mental action characteristics of a person, it is considered that it is difficult to maintain attention in a state where the person does not work (for example, state of not driving, not reading books, or the like). Therefore, it is expected that the driver's attention is reduced or the driver feels sleepiness in the automatic driving travel at the level 3. Furthermore, when the events that do not need to be coped with continue, an optic nerve reaction of the perceptual recognition learns the state in a recognition mechanism, and as a result, the reduction in the attention is prompted during the long-term use. Therefore, adverse effects such as reduction in an effect of monitoring the surrounding situation and drop in a perception level are predicted. Therefore, it can be said that the automatic driving travel at the level 3 is not an appropriate use form for continuous use by a person.

As a result, it is desirable to use the automatic driving travel at the level 3 in a short section between automatic driving travel possible sections at the level 4 in an environment in which the automatic driving travel possible sections at the level 4 are intermittently provided. The automatic driving travel possible sections at the level 4 are intermittently provided because continuous use of the automatic driving at the level 4 needs infrastructure and environmental improvements. In an environment in which the level 4 sections and the level 3 sections are sparsely connected to each other as a traveling environment, an operation form is assumed in which a section in which traveling that needs the attention to the driving is needed is generated in the middle, a driver return request is issued at the level 3 or lower and the driver appropriately returns to driving. Then, the system needs to automatically determine that the driver's wakefulness degree is appropriately returned, that is, the recognition and the determination and the coping action are returned to the wakefulness level necessary for the driver. Then, until the driver can actually perform steering on the steering seat after receiving the notification or the warning, the return delay time specific for the driver is needed. After estimating the temporal delay time before the return of the driver depending on the wakefulness level of the driver, the system needs to issue the notification or the warning before the estimated time. Moreover, the system needs to grasp the wakefulness level and the characteristics of the return sequence. Because the driver does not explicitly indicate the wakefulness level and the characteristics of the return sequence, the system needs to estimate the wakefulness level and the characteristics of the return sequence. As will be described in detail later, a strong candidate of the estimation processing is the behavior analysis of the eyeballs or the pupils.

It is difficult for the system to directly observe the perceptual determination in the brain and the coping action. However, additional visual information search necessary for information acquisition for external world recognition and determination or the like appears in the behavior of the eyeballs. Therefore, a response based on an information transmission loop from the detailed observation to the recognition and the determination, that is, an eyeball behavior response capable of acquiring information in order to understand the information in the brain (in visual cortex of brain) is observed. On the other hand, to observe the recognition and determination activity in the brain from the behavior of the eyeballs means to observe a partial reflection result of the neural transmission characteristics in the brain. As a side effect of the eyeball behavior observation, it is possible to estimate the neural transmission characteristics necessary for the perception in the brain. Although it is difficult to make absolute evaluation, it is possible to detect a transition of an activity degree of neural transmission through a temporal change in the eyeball behavior.

As a result, it is possible to acquire the reduction in the response reflection reaction caused by imbalance of the driver's autonomic nerves. In particular, since the driver is observed frequently by using the automatic driving, it is possible to continuously collect information for a long period without having a consciousness by the driver, and it is possible to detect the fluctuation in the driver's state. Therefore, use from the viewpoint of mental health monitoring is expected.

Note that, the driver's eyeball behavior will be briefly described. The eyeball behavior at the time when a person recognizes environment, in order to efficiently perform perception and recognition, for example, the line-of-sight is moved to a direction to be focused on the basis of information captured in the peripheral visual field, reference/search based on the memory of the experience in the past and the information from vision is sequentially proceeded, and fixed determination as object/situation recognition is obtained. At this time, needed next search is repeated. A partial response in the determination procedure in the brain can be observed as a response of the eyeball for searching for the visual information. In general, in a case where the driver returns from a situation in which the driver does not drive and takes a nap or watching videos to the steering after the automatic driving is widely used and the user is temporarily and completely separated from the driving steering loop, the driver needs to return to the perception and the recognition and the action determination, not to a state where the driver is dreaming. Therefore, an external observation strong unit that knows that the action determination is made in the brain includes the eyeball behavior analysis. Details will be described below.

The movement of the eyeball is not a behavior of the eyeball itself or a behavior caused by local reflection to the visual information. That is, the determination is seemingly and instantly made by hierarchically performing complementary information search necessary for completion of the determination in the brain in sequence. However, following comprehensive silhouette determination, local search is sequentially proceeded by the central visual field that makes the fixed recognition. Then, when the determination is completed with reference to the memory, the search is terminated. That is, the procedure proceeds to the saccade operation that immediately turns the central visual field to a peripheral luminance change portion from the risk determination made by the peripheral visual field that has been made prior to the behavior functional perceptual determination and the fixational eye movement that advances understanding of information captured in the central visual field next once when the central visual field captures the target at the time of turning by the saccade operation. However, the fixational eye movement that advances understanding of the target captured in the central visual field without via the recognition operation is sparse or becomes an operation that is not associated with an object, and this behavior becomes a glance sideways.

That is, by using the detailed behavior of the eyeball of the driver as an observation unit of the driver's state, it is possible to observe one aspect of the perceptual reaction in the driver's brain. In particular, the behavior of the eyeball turns the central visual field to a portion of which information is insufficient that is insufficient for situation recognition as a human recognition function, and detailed characteristics necessary for recognition of detailed image information captured by the central visual field is grasped. Then, the line-of-sight repeatedly moves to an event with higher priority to be recognized next in an order of making the determination with reference to user's experience information in the brain.

In these recognition actions, a specific behavior appears according to a step for advancing the rotational movement at high speed called saccade and recognition, and the perceptual determination in the brain of the user in Fixation time in which the line-of-sight is directed and the detailed search is proceeded. Note that the Fixation (visual fixation) that has been medically defined is a term that is defined because the eyeballs are observed as being fixed to a specific direction in the comprehensive observation. However, in reality, it has been known that the eyeballs performs slight and fine fluctuation search at high speed around that direction.

As described above with reference to Fig. 8, an unstable behavior of the eyeball, after turning the line-of-sight to new search by the saccade, observed in the peripheral direction indicated after the line-of-sight is turned to the target for information complementation is referred to as microsaccade. Furthermore, when specific information additional search is not performed, the behavior becomes a glance behavior as a simple drift. Note that, regarding the microsaccade that appears at the time of the visual fixation, when transmission through a back-side visual path to the parietal association cortex in the transmission of the visual information and a ventral visual path to the temporal association cortex is changed, the perceptual determination is affected.

In this way, the active situation in the brain directly affects the detailed behavior characteristics of the eyeball. Therefore, it is possible to know a perceptual active state by performing detailed analysis on the eyeball behavior. Note that the present embodiment is merely a description of a simplified model because the object of the present embodiment is not to describe the perceptual functions in the brain. When the user's consciousness is deteriorated, the situation determination regarding the content slightly delays even when the line-of-sight is directed to the target. As a result, the behavior characteristics of the eyeballs change according to the wakefulness state. However, as a result of advancing the determination with reference to the memory of the driver as described above, the determination largely depends on memories of the experience in the past and unconscious memory that control driver's individual memory. As a result, the detailed behavior appears as the characteristics specific for the driver. Therefore, there is an aspect that, unless learning evaluation is performed as assuming that the detailed behavior as the individual characteristics, it is not possible to make inner wakefulness determination with high accuracy. Therefore, details of the embodiment regarding a driver's manual driving return determination unit of the automatic driving will be described, and the detailed analysis on the eyeball behavior characteristics of the driver acquired in the above process is used so as to evaluate the imbalance between the sympathetic nerve and the parasympathetic nerve of the driver or the like. Accordingly, the result can be used as a screening index regarding the mental health of the driver or a screening index regarding a mental disorder.

For example, in a case where the driver needs to start the manual driving in the middle of automatic driving travel called the level 3 in the SAE definition (automatic driving travel that needs almost no driver's operation), the driver needs to return to the wakefulness state in which the driver can use operation ability which the driver can safely perform manual driving travel. If the system is not able to recognize the recovery state, safely travel on a travel route (itinerary) is not guaranteed in which a section where automatic driving can be performed and a section where manual driving and automatic driving under monitoring of the driver (so-called level 3 corresponding section) are mixed. This is not desirable.

When the automatic travel shifts to the manual travel by the driver, it is desirable to seamlessly switch the automatic travel to the manual travel with no reduction in speed and no stop. Processing for frequently stopping the vehicle and waiting for voluntary return of the driver is annoying to other vehicles that is traveling around the vehicle and causes traffic congestion and accidents. This causes inconvenience. Therefore, it can be said that the unit for determining the return ability to the manual driving during the travel, that is, a configuration that continuously evaluates the wakefulness degree is a vital technique.

In a second embodiment to be described below, learning specific for the driver conforming to the first embodiment described above is basically performed, and the driver's state is observed. However, in the vehicle that has the driving mode including use of the automatic driving level 3 or higher, a situation is caused in which the driver completely separates from the driving steering and it is not possible for the system to directly observe a device steering ability of the driver. That is, because it is not possible to observe the steering of the steering wheel and the pedal by the driver, passive observation is needed in at least a part of stages. As one of passive observations, eyeball behavior analysis of the driver is used.

Background that requires such processing will be briefly described.

Even if the automatic driving is introduced into the real world, it is difficult for a while for the vehicle to move at a speed equal to that of a conventional manual driving traveling vehicle between difficult two points with no steering by a person under various environmental conditions in which the vehicle may travel by using environment recognition by the artificial intelligence and a determination ability. Therefore, introduction of a traveling section with no human intervention proceeded by improving road infrastructures on which travel by the automatic driving system can be performed with no human intervention is considered as a realistic introduction procedure. On the other hand, the form of the owned vehicle that has been used to be used has an advantage such that the vehicle can move along roads from any starting point to the destination although the vehicle sometimes detours if road environments are continuously connected. That is, it is considered that the road environment is maintained in which the section where the vehicle can automatically travel with no human intervention and the section where the human intervention is needed are mixed and the driver travels through a traveling section by returning to the driving as necessary in this mixed section.

This idea also seems to be valid. However, a case where this idea is simply introduced has a pitfall. That is, human action patterns are not considered in this mechanism. As described above, in a case where a driver who uses the automatic driving section for a long term completely separates from the driving steering loop and a thinking activity completely separates from the driving steering, even when the driver is requested to return from the automatic driving to the manual driving or intervening driving, there is a possibility that the driver is unable to recognize the request. At the time of initial introduction of the automatic driving, it is considered that large separation of the consciousness from the driving steering loop is rare because a user unbelievingly uses the automatic driving system. However, when the driver gets used to automatic driving, there is a possibility that the driver misses a sign indicating that the driver needs to return from the automatic driving to the manual driving. Furthermore, a case may occur where the driver does not even have a consciousness of the need to return. A method is proposed that executes processing for emergently stopping the vehicle, decelerating, slowing down, and evacuating the vehicle in a case where there is a driver who misses an instruction to return to the manual driving. However, a frequency of such stopping processing increases on general road infrastructures, congestions occur, and there is a possibility to cause failures of social infrastructure functions and economic activities.

Return to the main topic, in order to take full advantage of the benefits of the automatic driving that is expected to be widely introduced in the future, while roads where the automatic driving can be performed are prepared and the environment is maintained, it is needed to return the driver to the safety manual driving in sections or at points where the road preparation and the environment maintenance are difficult to achieve.

In this case, before the vehicle enters the manual driving travel section from the automatic driving travel section, it is necessary to determine whether or not the driver can return to the manual driving. That is, it is required that the driver's state be constantly monitored and the driver's wakefulness degree reach a predetermined wakefulness degree at a manual driving start timing. In order to realize the above, the system needs to observe the driver's state. Furthermore, as a result of the actual switching work, it is possible to observe the result indicating whether or not the actual switching is succeeded and the switching quality (degree of driver's state at the time of successful switching). The observation of the observable evaluation value related to the wakefulness degree associated with the driver, whether or not the switching is succeeded, and the switching quality such as a switching return delay time, or the like can be collected as accumulated data, and it is possible for the system to estimate an accurate notification timing and a wakefulness (warning issuance) timing by performing correlation learning on data that is added each time an event occurs. Details of the timing estimation will be described later. On the other hand, it is possible to obtain a mental health index by associating the observable evaluation value obtained from the observation of the driver's state with the switching success quality of the switching work that occurs at each observation, performing self-learning on the fluctuation of the observable evaluation value in the medium and long term, and analyzing transition of the temporal change. In particular, in a case where the continuous change in the mental health index of the driver is continuously observed in comparison with the normal time, it is highly possible that imbalance between the sympathetic nerve and the parasympathetic nerve occurs. It is considered that a risk increases such that the continuance of the state causes the autonomic ataxia, depression, or the like to be serious. Subjective symptoms at the initial stage of the imbalance between the sympathetic nerve and the parasympathetic nerve are not known, and the disease condition is found after a medical examination at the time when the imbalance becomes serious, in many cases in general. Therefore, in most cases, at the time of the diagnosis, long-term treatment that imposes a burden is needed.

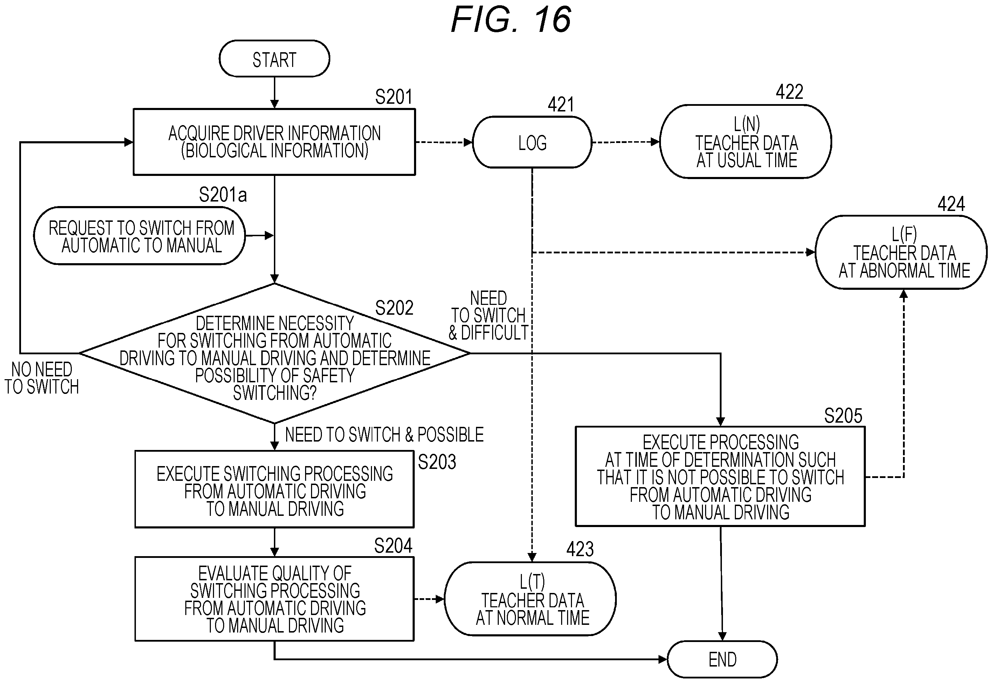

Here, when the automatic driving is used as described above, the driver is constantly observed in order to estimate the return delay time of the driver from the behavior characteristics of the optic nerve reaction that controls visual recognition determination by the driver. Then, the response characteristics affected by the imbalance between the sympathetic nerve and the parasympathetic nerve can be secondarily obtained from the observation results. This index relates to a technique to be provided. Specifically, a mechanism of the technique that can calculate the index with high accuracy is a portion having a return time estimator used at the time of switching to the manual driving when the automatic driving is used as a core. The mechanism will be described with reference to the flowchart in Fig. 16 and subsequent drawings.

The flowchart illustrated in Fig. 16 is, for example, a control processing sequence in a case where the automatic driving is performed at the level equal to or higher than the automatic driving level 3 in the SAE definition. That is, the flow is used to describe a control sequence in a case where the vehicle travels in a state where the driver can separate from almost all the driving operations, that is, a control sequence in a case where it is not possible to acquire the driving operation information from the driver.

Processing in each step in the flow illustrated in Fig. 16 will be sequentially described.

Note that, in the description of the flow in Fig. 16 and the subsequent drawings, processing in each step in the flow is executed by the moving device according to the present invention, the information processing apparatus included in the moving device, or the server that communicates with these devices. However, in the following description, for simplification of the description, an example will be described in which the information processing apparatus executes the processing in each step. When the vehicle having the automatic driving traveling mode is used, the system constantly observes the behavior characteristics of the driver, the state is recognized. The history of the behavior characteristics is learned as behavior characteristics information specific for the driver, and the behavior characteristics are taken in the dictionary as the individual characteristics.

Note that, in the flow illustrated in Fig. 16, a loop of steps S201 and S202 is continuously performed in a case where there is no need to switch from the automatic driving to the manual driving. However, for example, in a case where it is needed to switch the automatic driving to the manual driving due to an external factor change or the like, for example, in a case where an event trigger is received, it is determined whether or not to start switching in step S202 in accordance with the change. Note that, in order to recognize a sequence transition at the time of the change in the loop, it is assumed that a time when the driver's state is continuously observed be set to t (n + 1) after the driver's state information (biological information) is observed in step S201 at a time t (n) and the switching start sequence is not started in step S202, and the procedure returns to step S201.

When it is assumed that an arrival time to a time when switching from the automatic driving to the manual driving is needed be t (ToR_point) (Take over Request point) and a calculated switching budget in order to perform the switching at a predetermined switching success rate Requested Recovery Rate (RRR) back to the arrival time be Δt Minimum Transition Budget Time (MTBT), even in a case where the observation result at the time t (n + 1) does not change, it is requested to issue a switching notification or warning before {t (ToR_point) - Δt Minimum Transition Budget Time (MTBT)} prior to a predicted time t (ToR_point) to reach a ToR point. Therefore, the procedure shifts to a notification and warning issuance procedure at the time t (n) when {t (n + 1) - {t (ToR_point) - Δt Minimum Transition Budget Time (MTBT)}} < 0 is satisfied at the time t (n + 1) after the time t (n) by Δt.

In short, in a monitoring cycle period in which the driver's state is grasped, before the minimum switching delay allowable budget time that can achieve the RRR determined by the local dynamic map (LDM) data provided by the road environment is longer than the monitoring cycle period, it is required to issue the switching notification or wakefulness warning at the return delay time observed before the period.

Processing in each step in the flow illustrated in Fig. 16 will be described.

### (Step S201)

In step S201, the information processing apparatus starts to constantly observe observable biological information of the driver and estimates a delay time distribution before the switching success specific for the driver needed from the reception of the notification or the wakefulness warning predicted from the observable evaluation value of the driver from the system to the return to the manual driving by referring to a dictionary that records the history in the past.

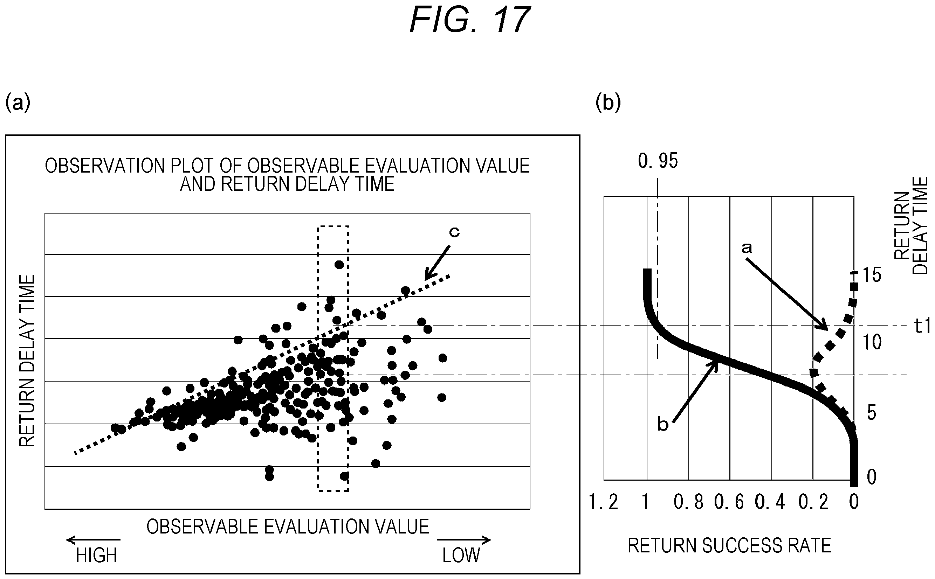

A correspondence relationship between the observable evaluation value and the return delay time (= manual driving returnable time) will be described with reference to Fig. 17. Fig. 17(a) illustrates an example of distribution of a plurality of pieces of relationship information (observation plot) between the observable evaluation value corresponding to the observation value and the return delay time (= manual driving returnable time). This example corresponds to a secondary task type of a certain driver. In order to calculate the return delay time from the plurality of pieces of relationship information (observation plot), relationship information (observation plot) in a region having a certain width in an evaluation value direction corresponding to the acquired observation value (indicated by broken-line rectangular frame) is extracted. A dotted line c in Fig. 17(a) indicates a boundary line when a return delay time at which a return success rate in Fig. 17(b) is 0.95 by using an observation value of a different driver.

By issuing the notification or the warning to return from the automatic driving to the manual driving to the driver at a time longer than the dotted line c, that is, an early extension time, the success of the return from the automatic driving to the manual driving of the driver at a rate equal to or higher than 0.95 is guaranteed in the region. Note that, a target value (Request for Recovery Ratio) at which the driver normally returns from the automatic driving to the manual driving is determined by the road side, for example, on the basis of the necessity of the infrastructure and is provided to an individual vehicle that passes though the section.

Note that, in a case where, even when the vehicle stops on the traveling road, the vehicle does not hinder the surroundings, it is only required to stop the vehicle and reduce the speed to a speed that the system can cope with. Normally, the stop on the traveling road is not preferable in many cases. Therefore, it is desirable to set a high return rate as default setting. There is a case where a significantly high return success rate is requested as a default even if update information is not given from the infrastructure in a specific route, in particular, Metropolitan Expressway or the like.

Fig. 17(b) illustrates a relationship between the return delay time obtained by using the plurality of extracted pieces of relationship information (observation plot) and the return success rate. Here, a curved line a indicates an independent success rate at each return delay time, and a curved line b indicates a cumulative success rate at each return delay time. In this case, on the basis of the curved line b, a return delay time t1 is calculated so that a success rate is a predetermined rate, that is, 0.95 in the illustrated example.

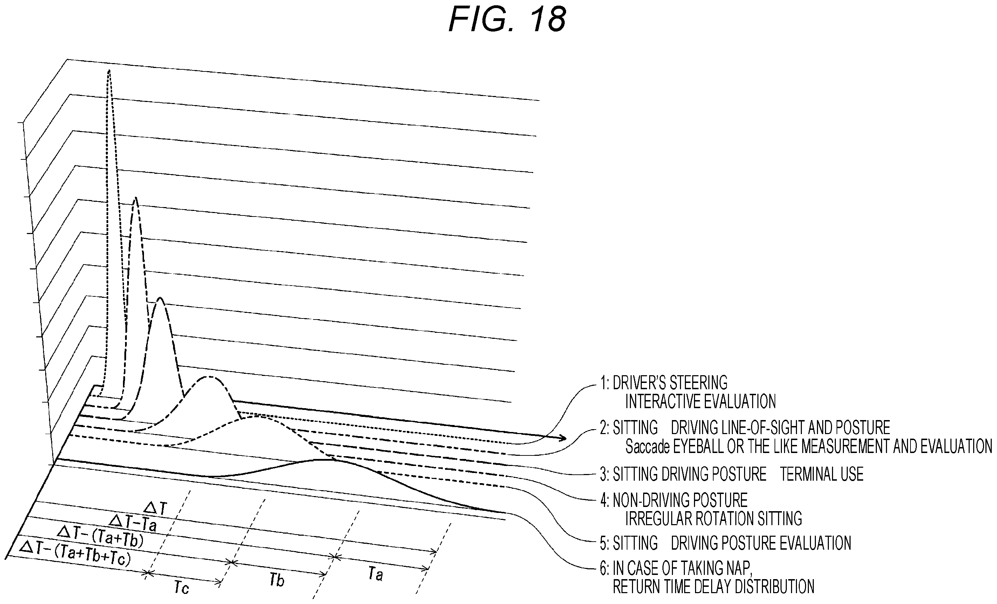

Fig. 18 is a diagram for explaining a manual driving returnable time according to a type of processing (secondary task) that is executed by the driver in the automatic driving mode in a state where the driver separates from the driving steering work.

Each distribution profile corresponds to the curved line a predicted on the basis of the observation value, that is, the driver's state illustrated in Fig. 17(b). That is, in order to complete the switching from the automatic driving to the manual driving at a necessary return rate, monitoring is performed until the switching is completed whether or not the driver's state reaches a state necessary for actual return at each return stage on the basis of the time t1 at which the profile (return success rate profile in Fig. 17(b)) is a desired value with reference to the characteristics in the past that are necessary for the driver to return from the observation value with which the driver's wakefulness degree detected at each stage can be evaluated.

For example, an initial curved line in a case of taking a nap is a cumulative average distribution of the return delay characteristics of the driver after a sleep level is estimated from observation information such as the breathing, the pulse wave, or the like that are passively monitored during a nap period in the automatic driving and the wakefulness warning is issued. Each distribution is determined according to the driver's state observed in the moving return procedure after the driver awakes. "6. in a case of taking a nap" illustrated in Fig. 18 is observed, and a right timing at which the wakefulness warning is in time is determined. The subsequent process in the middle of the procedure is a return time distribution in the return budget predicted from the observable driver's state evaluation value at the predicted intermediate point.

On the way, observation such that a remaining switching time limit that is sequentially decreased until the switching is not violated is continued, and in a case where there is a violation risk, the speed is reduced, and a time extension is generated. Note that, for example, "6. in a case of taking a nap", in a distribution at the time of the return starting from "4**.** non-driving posture irregular rotational sitting" with no step of "5. sitting", the return process starts from initial situation recognition. Therefore, even if the posture is the same as that of "4**.** non-driving posture irregular rotational sitting" that is an intermediate state of the process starting from "6. in a case of taking a nap", a thinking process is in the middle of a return consciousness process. In a case where the process starts from the situation recognition of the posture in "4**.** non-driving posture irregular rotational sitting" from the beginning, it takes time to recognize the situation.

Note that there is a case where the relationship information between the observable evaluation value and the return delay time of the driver who is currently driving is not sufficiently accumulated in the storage unit. In that case, the return delay time t1 can be calculated by using, for example, the return characteristics information generated on the basis of information collected from drivers in the same age that has been stored in the storage unit in advance as expected distribution information of the return. In the return information, the characteristics specific for the driver are not sufficiently learned yet. Therefore, the same return rate may be used on the basis of this information, or a higher return success rate may be set. Note that, because an unaccustomed user is ergonomically more careful, early return in the initial period of use is expected. As the user gets used to use, the driver adapts to an action according to the notification from the system. Note that, in a case where different vehicles are used in the logistics industry that operates a large number of vehicles, the bus and taxi industry, and in addition, sharing cars and rental bicycles, the driver is authenticated, and the observable information and the return characteristics of driving are intensively or dispersively managed and learned by a remote server or the like. An individual vehicle does not necessarily hold the data of the return characteristics, and may remotely execute learning processing or hold the data.

Furthermore, since a notification timing at which a notification of necessity of the switching from the automatic driving to the manual driving is issued is important, the return success rate is described as a time up to the uniform success. However, determination extended to the return switching quality may be further made without limiting the success to a binary success including the automatic driving and the manual driving. That is, return within an allowed time such as a delay time of the return procedure transition before the return is actually confirmed, a return start delay with respect to the notification, a stop in the middle of the return operation, or the like may be further input to the learning device as return quality evaluation values.

Note that the monitoring information (driver's state information) acquired in step S201 is the biological information of the driver, and the monitoring driver's information is sequentially stored in the storage unit as a log 421. Moreover, this log information is stored in the storage unit as teacher data at the usual time L(N)422.

It is not necessary to limit the observable evaluation value of the driver taken in step S201 to an observation value of specific biological information. There is a plurality of evaluation values that are driver's wakefulness indexes such as the heart rate, heart rate variability, blood flow, blood flow fluctuation, electrodermal activity, pupil luminance response characteristics, eye-opening time, eye-closing behavior characteristics, saccade, visual fixation, microsaccade, breathing fluctuation, blood pressure, brain wave, ocular potential, breath, facial expression evaluation, direction of the head, behavior, gesture, posture evaluation, behavior evaluation of posture fluctuation, active gesture response characteristics, sitting posture fluctuation, steering device setting stability evaluation, or the like. The driver's state is evaluated by using at least any one or more of the methods. Furthermore, at the same time, a log of the detailed behavior of the eyeball is concurrently acquired.

A major difference from a case of the use at the automatic driving levels 1 to 2 in the first embodiment described with reference to the flow in Fig. 14 is in that the operation information of the driver is not acquired. In the automatic driving at the level 3 or higher, the driver is allowed to completely separate from the steering loop. Therefore, it is not possible to acquire the operation information of the driver. In this state, it is not possible for the information processing apparatus to constantly acquire the response reaction of the driver. The information processing apparatus needs to recognize a time necessary for the return to the driving by the driver (= time before start of safety manual driving) only from the biological information. However, in a case where the driver regularly feeds back a reaction in response to the recognition of the notification from the system by using a wearable terminal, a nomadic device, or the like, there is a use form in which the driver's response reaction is used.

The system (information processing apparatus) needs a procedure for already recognizing the simple state and the status of the driver at the stage before the wakefulness degree of the return to the driving is determined. That is, a timing for calling attention to the return differs depending on the situation such as the driver is sleeping, or is not seated. The system needs to issue notifications and warnings at an appropriate return timing. This is because, in a case where the system issues a notification much earlier than a point necessary for actual switching, inevitability of the time from the notification to a time to start to perform an actual return work is deteriorated. The notification is issued at a timing when there is enough time even if the driver does not promptly start to return, and the notification is assumed as a crying-wolf notification, and the user downplays the notification (importance of early action). However, if the notification is issued immediately before the point, there is a possibility that the driver fails to cope with the notification in time. Therefore, in order to realize long-term stable use of a large number of vehicles, optimization of the notification timing from the ergonomic viewpoint according to the driver's state and the control characteristics of the vehicle is needed. To find the timing, it is necessary to constantly monitor the driver. A vital signal group effective for the medium-and-long-term observation includes the heart rate, the heart rate variability, the blood flow, the blood flow fluctuation, the electrodermal activity, the pupil luminance response characteristics, the eye-opening time, the eye-closing behavior characteristics, the breathing fluctuation, the blood pressure, the brain wave, the ocular potential, the breath, the facial expression evaluation, the direction of the head, the behavior, the gesture, the posture evaluation, the behavior evaluation of the posture fluctuation, the active gesture response characteristics, the sitting posture fluctuation, the steering device steering stability evaluation (case of performing steering), or the like. Then, since the timing optimized by constant monitoring is constantly monitored, the return notification is issued to the driver on the basis of that information, and the return by the driver from the secondary task starts at the time of the notification.

Moreover, major characteristics that are largely different from the first embodiment (automatic driving levels 1 and 2) described with reference to Fig. 14 above are that it is necessary to determine the return level of the consciousness state necessary for the driver's return from the state completely separated from the driving to the driving. In particular, the system needs to determine the situation in the brain without directly viewing the situation. The situation in the brain indicates how accurately the driver who is in a dreaming state due a nap or the like recognizes the thinking activity in the brain and the situation in the real world in front of the traveling vehicle so as to return to driving. Therefore, although it is difficult to directly observe the activity situation in the driver's brain, the detailed behavior characteristics of the eyeball reflect a part of the activity situation in the brain and are a few units for indirectly observing the activity from outside.

A person who uses the visual information as an information acquisition unit necessary for the activities moves the eyeballs, the head and the body to supplement detailed determination information to the direction captured by the peripheral visual field as a mechanism necessary for survival, and shifts to the visual fixation state in which the information is supplemented in order to understand the visual information by the central visual field. In addition, when the intelligent understanding of the content is completed, the line-of-sight shifts to acquire next information. The determination is completed by making a determination at the time when the visual information is unconsciously compared with the knowledge in the past, and the visual information and certain determination memory are comprehensively determined. The firing of the determination at this time terminates the activity of the fixational eye movement that is detailed information supplement, and the procedure shifts to the next information search.

That is, the search is intended to continue from the visual information advanced as the activity in the driver's brain, in particular, individual information accumulation of the detailed local information obtained by repeating the fixational eye movement by the central visual field to the determination with reference to experienced memory information in the brain made when the determination is fixed. As a result, the eyeball behavior for searching that is the fixational eye movement is directly affected by the imbalance between the sympathetic nerve and the parasympathetic nerve, and the determination act is advanced. Here, when the driver starts to return to the seat and to perform steering on the basis of the drive return instruction, it is assumed that instruments are checked after finally and visually confirming the front road situation at least once and acquiring the information through more intuitive visual feelings.

In other words, an information and characteristics recognition work using individual visuals in driving that is a part of the switching procedure is observation of the driver by a device, which is not binding and is separated, from the outside as the behavior of the eyeglasses. Therefore, it is possible to indirectly observe a part of the perceptual activity in the driver's brain.

According to the present embodiment, a classifier (classification unit) can recognize the observation value as the individual characteristics and perform self-completed learning by performing self-learning on a correlation between the actual observable value of the driver, the observation in each situation, a driving returnable level that is further observed as a result of the switching in the observed state. Because the learning processing is constantly executed through the use of the vehicle, the response characteristics of the driver at the normal time can be acquired, and the observation can be performed when the response characteristics change. By monitoring the behavior characteristics of the driver that can be classified in a long term, it is possible to capture the response change characteristics, and the unbalance index is secondarily calculated.

Note that, in the conventional driver monitor system, the deterioration in the wakefulness is an observation target in the driver's state observation. The observation is stopped depending on the behavior characteristics and the switching quality at the time of switching depending on the behavior, that is, at the initial time when the wakefulness is deteriorated. Therefore, detailed classification processing based on the wakefulness degree quality including a range where the wakefulness is deteriorated is not needed. Accordingly, the change in the behavior characteristics has a narrow fluctuation width of the observable value that can be classified and is only an observation variation width.

In the processing according to the present invention, the biological information acquired in step S201 is held in a memory such as a recording medium for a certain period of time. The holding time may be about several minutes at the longest if the driver is in the wakefulness state. However, in a case where the wakefulness level is deteriorated, a transition history for a longer time is held separately from short-term records in order to evaluate a long-term transition of the return delay time. When the transition history is saved, repeated records for a certain period of time are recorded in an endless manner, records for a certain period of time are continuously taken. Then, in a case where the state changes, the records before the time is extracted and saved as a series of biological observable record logs, and the learning processing associated with the switching quality thereafter is executed so that the correlation between biological observable change transition and the wakefulness and reflex level of the driver can be obtained.

### (Step S202)

Next, the information processing apparatus executes in step S202 necessity determination of switching from the automatic driving to the manual driving and safety switching possibility determination processing.

This determination processing is executed with reference to a time requested for a Take Over Request (TOR switching request information = time requested before switching from automatic driving to manual driving) generated in step S201a.

The time requested for the Take Over Request (TOR switching request information) is compared with a remaining budget in a return necessity grace time, and the determination processing in step S202 is executed.

The processing in step S202 is executed for a notification regarding switching points (switching point from automatic driving to manual driving) that appear one another as traveling advances or time elapses, a notification prior to the above notification, review of a wakefulness warning point, review of an approaching point, or the like. Note that the notification point and the wakefulness warning point are timings when a notification is issued according to the driver's observation state or the system issues the wakefulness warning.

The notification timing is {t (n + 1) - {t (ToR_point) - Δt Minimum Transition Budget Time (MTBT)}} < 0 at the time t (n + 1) determined on the basis of the above formula. The notification timing is a time when a remaining time budget of the return transition time until the driver can normally return to the manual driving can be secured with no excess or deficiency in a case where the system issues the notification or the wakefulness warning after the calculation timing on the basis of the learning result of the return characteristics of the driver.

In step S202, update information of the local dynamic map (LDM) according to the change in the driver's state and the travel along a planned traveling route is additionally considered, and whether or not the switching can be performed (switching availability state from automatic driving to manual driving) is confirmed in consideration of the update situation.

This step may seem unnecessary. However, this step is needed, for example, in the following situation. For example, in a case where it is determined that the driver sufficiently awakes and does not need to start switching in the confirmation procedure in step S202 in a state where a planned switching point does not change, the procedure returns to step S201. Even when the deterioration in the wakefulness state of the driver is advanced at that time and the return warning is issued in the changed wakefulness deteriorated state, the driver is in a low wakefulness state where the return takes more time than the return predicted time of the system, and in addition, there is a possibility that the driver is in a state where the return is not expected in actual due to a sudden attack or the like.

On the other hand, in a case where an event such as a new accident or the like occurs on a planned route, there is a possibility that the switching point and the warning issuance point change. That is, the loop including steps S201 and S202 is an effective step when the switching point is reviewed according to the situation changes.

Note that, regarding the change here, a change from a state where the driver feels sleepiness and the notification immediately before the switching point is originally sufficient to a state where the wakefulness warning is issued at an earlier timing may occur. The countermeasure to the change is taken. When the switching occurrence state does not change, the procedure returns from step S202 to step S201, and a switching standby loop is formed by observing the change and turning the loop. Note that, if the characteristics of the road infrastructure, the weather, the traveling environment, the flow of the vehicles, or the like change, there is a possibility that t (ToR_point) changes. There is a possibility that the time extension required to return, that is, Δt Minimum Transition Budget Time (MTBT) changes if secondary task work content of the driver changes.

A repetition interval at the time of monitoring, that is, an interval of Δt is a variable interval, and monitoring is continuously performed with this standby loop. The continuous state of the driver and the change in the situation are detected and updated, and step S203 is performed according to each change detection state. In that state, it is determined again whether or not the switching is needed. That is, it is determined whether or not the wakefulness returns from the automatic driving to the manual driving by referring to the series of observable evaluation values that have been detected in the time when the change occurs and are used to determine the wakefulness of the driver and the self-learning dictionary. As described above, the change in the state here is a change in a case where the driver suddenly falls asleep and the deterioration in the consciousness state is observed due to advance in the deterioration in the consciousness of the driver although the driver has been in a situation where it is possible to perform switching in terms of time in response to a notification 30 seconds before the switching point because the driver originally looks forward. In this case, the time requested for the Take Over Request (TOR switching request information) generated in step S201a is referred on the basis of the determination information in step S202. In a case where it is found that the time requested for the Take Over Request (TOR switching request information) is shorter than the remaining budget in the return necessity grace time, the switching is started. That is, the procedure proceeds to step S203.

On the other hand, in a case where the remaining budget of the return necessity grace time that is recalculated according to the change is shorter than the time requested for the Take Over Request (TOR switching request information), the procedure proceeds to step S205 as a sequence for emergency.

In a case where there is a spare time even if these changes are additionally considered and there is room to continuously confirm whether or not the driver's state further changes, the procedure proceeds to step S201.

Note that the series of processing executed in step S201 is not simple biological information detection and includes the visual detection specific for the driver, the situation recognition, the determination corresponding to the recognized information, and conversion of the expected distribution of the delay time needed before the actual switching is completed. When the switching request from the automatic driving to the manual driving occurs in step S201a as the Take-over Request (TOR) due to a planned or an unexpected factor during this processing, the system needs to determine a timing to issue the notification to the driver or to perform a procedure such as the warning or emergency stop as necessary. In the determination procedure S203, it is determined whether or not the switching can be performed depending on whether or not the return is completed within the time from the return time characteristics of the driver that is constantly estimated in the previous stage.

In order to simplify the description in this flowchart, conditional branches in step S202 summarized into three branches are illustrated. However, the processing is more complicated in reality, and for example, a delay in reaching a switching point may be further generated by reducing the vehicle speed to minimize the occurrence of the switching failure (step S205). Here, in a case where the automatic driving is not normally switched to the manual driving, the learning processing unit stores the state data taken as the observable data log of the driver before start of the switching in the storage unit as the teacher data at the abnormal time L(F)424 in step S205.

Specific examples of three branch processing in step S202 are, for example, as follows.

### (1) Case of returning from step S202 to step S201

Although a state in which the driver has waked up and operated a terminal device such as a tablet terminal has been observed, the driver has fell asleep with time. Moreover, a section in which the manual driving is needed does not approach. In this case, it is possible to further continue to monitor the change observation. The procedure returns to step S201, and the monitoring is continued.

### (2) Case of proceeding from step S202 to step S203

Although a state in which the driver has waked up and operated a terminal device such as a tablet terminal has been observed, the driver has fell asleep with time. Moreover, a section in which the manual driving is needed is approaching. In this case, the procedure proceeds to step S203, and a wakefulness alarm and a wakefulness notification are issued to the driver to prompt to return to the driving.

### (3) Case of proceeding from step S202 to step S205

Although a state in which the driver has waked up and operated a terminal device such as a tablet terminal has been observed, the driver has fell asleep with time. Moreover, unexpected early manual driving return request is issued at a point before a switching start predicted time when the manual driving is needed due to an accident or a rapid change in the road environment (for example, change such as flood caused by rapid rain on planned traveling road). In this way, in a situation where it is too late for the driver to recover the wakefulness in response to an alarm due to the sudden return request at an earlier timing than the normal planned travel, the procedure proceeds to step S205 in order to execute evacuation processing for automatically performing an emergency speed reduction and evacuation procedure by the system.

### (Step S203)

Step S203 is executed in a case where the switching from the automatic driving to the manual driving is needed in the branch processing in step S202 and it is determined that the driver can safely start the manual driving.

The system (information processing apparatus) issues the wakefulness alarm or the wakefulness notification to the driver in step S203 and prompts the driver to return to the driving.

In step S203, the driving starts the switching work in response to the actual switching request from the system. Because the driver is in various states such as a case where the driver is taking a nap in response to the switching request or a case where the driver loses the sitting posture, the return sequence is observed. It is observed that whether or not the return sequence during this time is performed along a normal return procedure specific for the driver through the behavior evaluation of the posture fluctuation or the return procedure takes time. For example, a learning dictionary recorded value of distribution of the return time of the driver in response to the return request for the driver who rotates the seat and executes slip entry processing on the tablet terminal is about ten seconds. In a state where the return start is not detected even after about 20 seconds, it is determined that the return of the driver is obviously delayed. Furthermore, the driver who is lying and taking a nap does not get up within a normal learning history time, it is determined that the delay is caused.

Step S203 includes a series of procedures of the return to the manual driving by the driver. Therefore, for example, from the time when the driver is lying and taking a nap to the time when the switching is completely completed, a series of intermediate observation values can be obtained. The observation values include 1. getting up posture, 2. moving to the driver's seat, a forward confirmation operation, a sitting transition, wearing the seatbelt, eyeball detailed behavior analysis and facial expression analysis, and in addition, drivers actual steering device steering characteristics.

### (Step S204)

In step S204, upon the completion of the switching from the automatic driving to the manual driving, the quality is evaluated whether or not the switching operation is smoothly performed or the procedure delays, each time the switching is performed.

In a case where it is determined in step S204 that the switching fails, in order to avoid to trigger an accident or a traffic congestion by the switching failure, the speed of the vehicle may be reduced or the vehicle may be slowly driven and evacuated. Basically, a main cause of the switching failure is because the driver is unable to appropriately perform switching at a necessary timing or the driver has a poor situation detection and determination ability due to the autonomic ataxia or precursor symptoms. When a mechanism that applies some penalties to the driver in a case where the delay occurs with respect to the request to the driver from the system is in combination with the system using the automatic driving, the driver promptly normally starts the return work in response to the return request or the warning notification from the system.

However, both of a case where the wakefulness state of the driver is insufficient and a case where the driver's return is too late because the event suddenly occurs are similarly determined as the switching failure. Originally, it is expected for the driver to start to promptly recognize the situation and to perform the needed return recovery procedures in response to the notification in order to perform the expected return work by the driver by using the mechanism in which an operation that the driver promptly returns to the driving is established. As a result, the driver's recognition of the situation affects the cause of the delay. Therefore, regarding the same switching failures, in switching in a case where the switching request from the automatic driving to the manual driving is generated in step S201a as the Take-over Request (TOR) due to an unexpected, sudden, and early switching request or a planned or unexpected factor, exclusion processing is applied on a correlation of the acquired observable value log that is associated with the wakefulness degree and the switching quality.

Note that an observable evaluation value group of the driver that is constantly observed and acquired in step 201 is stored in the storage unit after being classified into the teacher data at the usual time L(N)422 that is a log of an observable evaluation value group of usual time behavior characteristics in a case where the driving switching is not particularly performed, the teacher data at the normal time L(T)423 that is a log of an observable evaluation value group at the time of the switching success, and the teacher data at the abnormal time L(F)424 that is a log of an observable evaluation value group at the time of the switching failure.

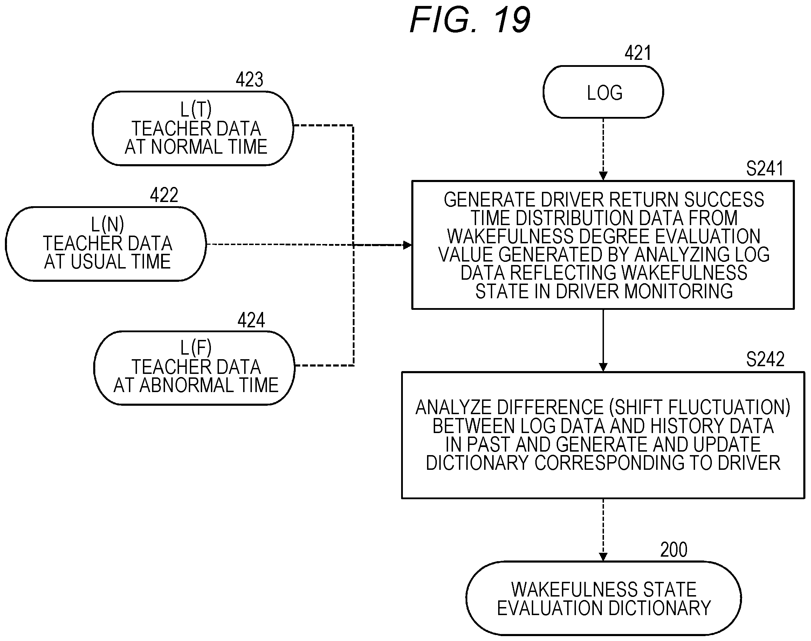

The learning processing unit executes the processing according to the flow illustrated in Fig. 19 by using these pieces of data and executes processing for updating the wakefulness state evaluation dictionary 200 indicated at the end of the flow in Fig. 19.

The wakefulness state evaluation dictionary 200 is a dictionary used to determine a degree of the deterioration in the wakefulness degree (consciousness level) of the driver.

Processing in each step in the flow in Fig. 19 will be described.

### (Step S241)

First, in step S241, the learning processing unit (learning device) of the information processing apparatus executes learning processing to generate and update the wakefulness state evaluation dictionary 200. Specifically, for example, by monitoring the driver, driver return success time distribution data is generated from the wakefulness degree evaluation value generated by analyzing the log data reflecting the wakefulness state.

By executing the learning processing, data can be obtained that is used to estimate whether or not the driver can normally start the manual driving on the basis of the driver's state information acquired by monitoring the driver and a time before the manual driving can be started (limit allowable delay time).

Step S241 is a learning step for creating the wakefulness state evaluation dictionary 200 used to execute the determination processing in step S202 in the flow described above with reference to Fig. 16. The learning device used for the determination processing is, for example, an evaluator that calculates a time until the completion of the actual return when the return notification is received, from the observable activity amount evaluation value of the driver. The calculated time is used to determine whether or not there is a grace time necessary for the return to the manual driving by the driver. However, the characteristics vary for each driver in reality. If this variation is not excluded, it is necessary to set the grace time with a margin that is applicable to a large number of drivers so as to issue a notification.

In this case, even when a large number of drivers receive the notification, the notification timing is too early, and the users neglect the notification in this use form. Therefore, as a result, there are many cases where it is not possible for the system user to perform normal switching. The measures against the above state are dictionary corresponding to each driver (wakefulness state evaluation dictionary 200) generated by the processing according to the present invention. By using the dictionary for each driver, it is possible to issue a notification in accordance with the return characteristics specific for each driver.

For more accurate estimation of the return delay time, for example, the state in the brain of the driver whether or not the perceptual determination can be made is detected. By executing such processing, it is possible to more accurately determine the wakefulness degree of the driver, and in addition, it is possible to improve the accuracy of the return time estimation.

### (Step S242)

In step 242, a difference (shift fluctuation) between the latest log data and the history data in the past is analyzed, and the wakefulness state evaluation dictionary 200 corresponding to the driver is generated and updated.

Specifically, by monitoring a history of a medium- and-long-term fluctuation amount in the past by using the driver log information of which the history is saved to obtain the return delay time distribution characteristics from the driver's observation value acquired in step S241, the characteristic fluctuations that are difficult to detect in the short-term observation are analyzed.

A wide variety of evaluation values of the observable evaluation value group may be used according to the mounted detection system, and the evaluation values include the heart rate, the heart rate variability, the blood flow, the blood flow fluctuation, the electrodermal activity, the pupil luminance response characteristics, the eye-opening time, the eye-closing behavior characteristics, the saccade, the visual fixation, the microsaccade, the breathing fluctuation, the blood pressure, the brain wave, the ocular potential, the breath, the facial expression evaluation, the direction of the head, the behavior, the gesture, the posture evaluation, the behavior evaluation of the posture fluctuation, the active gesture response characteristics, and the sitting posture fluctuation. In particular, regarding the eyeball behavior characteristics and the pupil behavior characteristics among the observable vital signals, it is possible to observe a response reaction in the wakefulness state in an extremely short time in response to a fluctuation in external information regarding the driving. In particular, the behavior of the eyeball such as the saccade operation, the microsaccade, the drift, the fixation, or convergence eyeball movement is perceptually and reflectively determined on the basis of a target recognition degree in the brain unconsciously for driver's recognition of the external world. Transition of the appearance of these behaviors and the behaviors is tracked at high speed, and multidimensional self-completed learning for each condition is performed on each event and the switching result, thereby the eyeball behavior characteristics and the learning dictionary specific for the driver are created. For example, in the fixational eye movement behavior for about 0.1 seconds observed at 1000 f/s illustrated in Fig. 9, in order to understand the details of the target captured by the central visual field in a state where the wakefulness degree of the driver is high, a search behavior appears in a range where the driver views substantially the same target in order to recognize and determine details of the destination of the line-of-sight. However, in a state of the lowered consciousness, the eyes do not have a target, and the behavior becomes the glance behavior. Therefore, the behaviors in both cases are different from each other. It has not been able to perform analysis due to a lack of a time resolution in conventional video observations at about 30 f/s. However, by analyzing the details of the eye behavior at high speed, it is possible to quantitatively evaluate a local behavior of eyes, which move at high speed, in a short time. Note that the observational evaluation includes individual evaluation of one eye or both eyes, correlation evaluation of both eyes, or the like. Because the behavior differs for each individual, it is desirable to fix a combination and perform the observational evaluation to enhance the accuracy.

In more detail, according to initial inputs that have been determined, by the driver to be important, auditorily (notification sound, alarm sound, horn of surrounding vehicle, or the like), visually (lamp display, notification display, alarm display, information from front side, flashlight of surrounding emergency vehicle, mobile and wearable device notification, or the like), and the driver visually searches for and acquires the information that is important for driving. The driver confirms primary input information first. Moreover, the driver starts to perform search necessary for determination regarding the next event that is continuously generated at the time when individual confirmation procedures of the perceptual determination in the brain are completed. If there is no information to be obtained next by the completely same line-of-sight, a dynamic behavior is observed such as the saccade operation of the eyeball is caused in a direction assumed to be confirmed by the peripheral visual field, smooth pursuit for locking the line-of-sight on a target on which the visual fixation has been executed and which has been recognized once and tracking the target, or the like.

Furthermore, the information determination is not completed instantly after the line-of-sight is directed from the experience history specific for the driver, more accurate evidence is continuously searched near that position. Therefore, an operation is waited in which the search visual fixation specific for the driver appears, understanding and the determination are completed or a saccade operation occurs for moving the line-of-sight to another determination item according to search necessity weighting on another important determination matter that may concurrently occur. In a case where the next target is confirmed in a state where the determination and the operation are not terminated, an operation for returning the line-of-sight to the same place and fixing the determination may be repeated.

Since these high-speed and small movements of the driver occur in association with the operation connected to a time necessary for recognizing and determining the target in the brain, when the balance between the sympathetic nerve that controls the autonomic nerves of the user and the parasympathetic nerve is lost, the imbalance affects the determination operation, and an effect appears in the detailed behavior of eyeballs that is unconsciously made by the user. The movement of the individual eyeball appears as an operation necessary for recognition based on the experience of the individual. Therefore, the movements of the eyeballs do not appear as uniform behaviors. For example, personal constitutional characteristics classified into a person who is more active in the morning and a person who is more active in the night are caused because the characteristics regarding time bands when the sympathetic nerve and the parasympathetic nerve become active are different between individuals.

The learning processing executed as the processing in step S241 is executed as learning processing to optimize the determination for the individual behavior characteristics of the observable evaluation value detected from the driver.

In step 242, the learning processing is executed for detecting an appearing behavior change as the disease or the precursor of the disease that appears as a gradual change in most cases by acquiring the learning data reflecting the individual characteristics in the medium and long term and continuously performing stationary observation on the behavior.

Note that it is preferable that an evaluation value that is calculated by using the wakefulness state evaluation dictionary 200 generated and updated as a result of the processing in steps S241 and S242 be not completed in one itinerary and be set to reflect the observation result of the changes in the behavior characteristics over a plurality of days. Unlike a use application of a short-term determination used in step S241, a user (driver) who constantly uses the vehicle on a daily basis can use the value to calculate an index in a long term such as one week or one month.

Note that, in the wakefulness state evaluation dictionary 200 generated and updated as the result of the processing in steps S241 and S242, each time one itinerary is made according to an object of the driver, a change in an observation value related to a wakefulness state during the travel that is recorded in the itinerary is recorded as a statistically processed value. The individual observable value that is observed each time the manual driving is performed is an observation value necessary for the determination on the return of the driver that is made each time. However, on the other hand, only by simply performing pinpoint observation on the evaluation value, it is difficult to determine the level of the wakefulness state of the driver. Therefore, each time the switching (switching from automatic driving to manual driving) determination procedure is performed, the processing in steps S241 and S242 is executed. Then, in accordance with the switching quality obtained from the result of the switching, the observable log acquired in step S201 in the flow in Fig. 16 described above is repeatedly classified and reevaluated. By repeatedly executing (learning) the series of classification evaluation for each situation, it is possible to improve the wakefulness state determination performance.

Furthermore, when the fatigue of the driver is accumulated, for example, it is not possible to smoothly steering the steering wheel. That is, a reflection operation of the driver is delayed, and the delay causes a small steering amplitude, disturbance, or a delay. With respect to information regarding these individual disturbances, for example, a frequency of the behavior is analyzed, and an occurrence frequency distribution and an amplitude distribution width are analyzed, and the result may be recorded in the dictionary. For example, when the driver's wakefulness state is analyzed on the basis of the eyeball behavior, it is effective to record a certain behavior analysis value such as a saccade occurrence frequency with respect to visual Saliency information regarding the front side on the road, a pursuit tracking behavior according to travel and its duration, a time period in which the driver continuously looking at the target by visual fixation (Fixation), direction stability and a flow of a peripheral small behavior that occurs during the fixation, an amplitude and a search range of a neighborhood search behavior performed to understand the target to which the line-of-sight is directed, staying time when the line-of-sight moves to another target, or the like. Since the eyeball behavior changes due to a different factor such as a visual acuity state including daytime, nighttime, and fatigue, an environment specific for each traveling state, accumulated driving fatigue, or the like, it is preferable to record and save a log as a multidimensionality dependent fluctuation value.

Moreover, by observing the change in the individual itinerary in a longer term, it is possible to observe a change in the situation reflection characteristics. For example, in the eyeball behavior analysis, when the situation response of the driver is not a temporary delay and is delayed in a medium and long term, this means deterioration in an information transmission function of the optic nerve. Alternatively, as a result of the delay in the situation determination, a rapid correction reflection reaction that compensates the delay starts to increase, and from the smooth steering, occurrence of under-correction and over-correction of a steering angle and an increase in the frequency of the occurrence are detected. The recorded data of the behavior characteristics for each itinerary is used to learn specific behaviors and can be used for processing for comparing a regular behavior of the driver with a medium- and-long-term behavior and detecting a change in the behavior.

In the second embodiment, in particular, the embodiment based on the invention using the level 3 or higher has been described. However, functions using another side effect of the present invention will be supplemented. In the today's society with too much stress, a large number of people have the autonomic ataxia and need to temporarily interrupt the social activity and receive treatment. On the other hand, it is difficult to grasp the state on the way to the autonomic ataxia. Moreover, even in a case where the person has the autonomic ataxia, the subjective symptoms do not clearly appear. Even in a case where a patient complains health problems and receives medical examination by a doctor, the patient receives the medical examination by a specialist other than a psychiatrist in many cases, and it takes time to be determined as a mental disorder. Therefore, an index that can recognize an imbalance between the sympathetic nerve and the parasympathetic nerve that causes the autonomic ataxia at an early stage or can indicate the precursor of the autonomic ataxia is a technique useful for estimating the disease condition of the driver. At the stage of a mild imbalance between the sympathetic nerve and the parasympathetic nerve, recovery without making the disease condition be severe is expected by reducing the stress at the time when a noticeable subjective symptom does not appear. In general, the imbalance between the sympathetic nerve and the parasympathetic nerve is largely different for each individual, and it is difficult to recognize the subjective symptom. Therefore, it has been difficult to prevent the imbalance. Originally, the sympathetic nerve and the parasympathetic nerve are adjusted in a balanced manner, and it is desirable that activities important for humans such as adjustment of sweat and a body temperature, the blood pressure, the breathing, the heartbeat, food digestion, or the like work with balance.

There is a case where an excessive load is applied in life to one of the nerves due to a specific factor such as stress, the balance of the autonomic nerves is lost for some reason, and it is not possible to adjust the balance. As a result, various physical and mental disorders may appear, and these disorders provoke diseases such as the autonomic ataxia. When one of the sympathetic nerve and the parasympathetic nerve continues to be in a tension state, frequent firing of the nerves excessively occurs, and the tension state is maintained. A large number of patients who suffer the autonomic ataxia feel an abnormal change as a result of advanced symptoms and visit a department related to the symptoms due to the disorders. The present invention relates to the determination on the driver's wakefulness that is essential when the driver uses an automatic driving function of a self-driving vehicle. By using the information acquired when the wakefulness of the driver is determined, a mental health index can be obtained. Therefore, the present invention also has an effect that it is possible to easily cope with the mental disorder of the driver such as the autonomic ataxia before the disorder becomes serious.

### [6. Exemplary Configuration of Information Processing Apparatus]

The processing described above can be executed by applying the configuration of the moving device described with reference to Fig. 3. However, a part of the processing can be executed, for example, by the information processing apparatus that is detachable from the moving device.

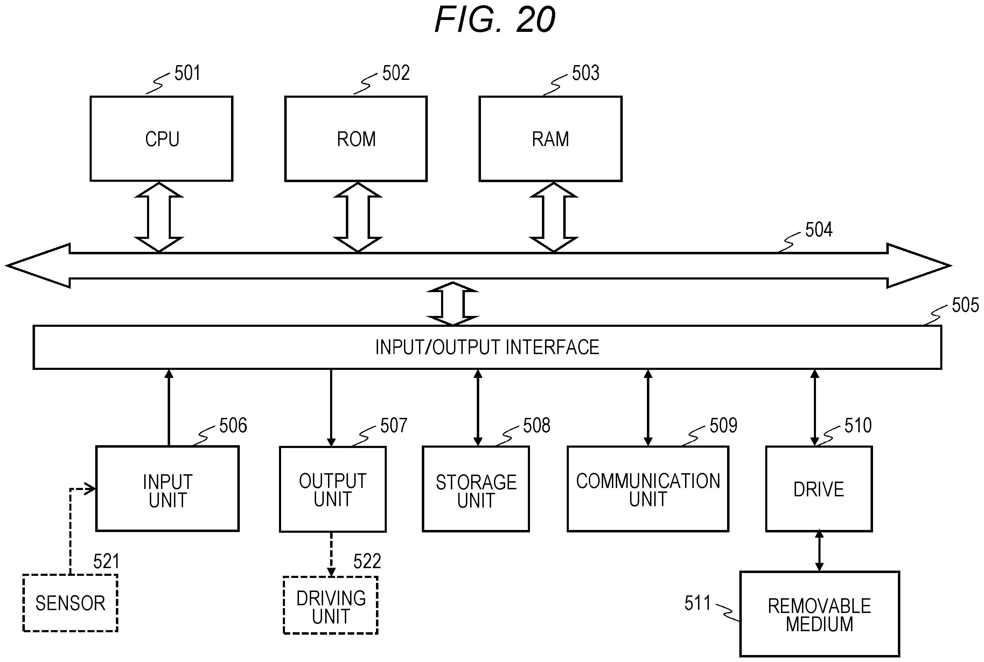

An exemplary hardware configuration of such an information processing apparatus will be described with reference to Fig. 20.

Fig. 20 is a diagram illustrating an exemplary hardware configuration of an information processing apparatus.

A central processing unit (CPU) 501 functions as a data processing unit which executes various processing according to a program stored in a read only memory (ROM) 502 or a storage unit 508. For example, processing according to the sequence described in the above embodiment is executed.

A random access memory (RAM) 503 stores the program executed by the CPU 501, data, and the like. The CPU 501, the ROM 502, and the RAM 503 are connected to each other by a bus 504.

The CPU 501 is connected to an input/output interface 505 via the bus 504, and the input/output interface 505 is connected to an input unit 506 including various switches, a keyboard, a touch panel, a mouse, a microphone, and a situation data acquisition unit such as a sensor, a camera, a GPS, or the like and an output unit 507 including a display, a speaker, or the like.

Note that input information from a sensor 521 is input to the input unit 506.

Furthermore, the output unit 507 outputs driving information with respect to a driving unit 522 of the moving device.

The CPU 501 inputs an instruction, situation data, or the like input from the input unit 506, executes various processing, and outputs the processing result to, for example, the output unit 507.

The storage unit 508 connected to the input/output interface 505 includes, for example, a hard disk and the like and stores the program executed by the CPU 501 and various data. A communication unit 509 functions as a transceiver for data communication via a network such as the Internet and a local area network and communicates with external devices.

A drive 510 connected to the input/output interface 505 drives a removable medium 511 such as a magnetic disk, an optical disk, a magneto-optical disk, or a semiconductor memory such as a memory card and records or reads data.

Furthermore, the series of processing described in the specification can be executed by hardware, software, or a composite configuration of the hardware and the software. In a case where the processing is executed by the software, it is possible that a program in which a processing sequence has been recorded is installed in a memory, which is built in dedicated hardware in a computer, and executed or it is possible to install the program in a general computer, which can execute various processing, and make the computer execute the program. For example, the program can be recorded in a recording medium in advance. In addition to installing the program from the recording medium to the computer, it is possible that the program is received via a network such as a local area network (LAN) or the Internet and installed to a recording medium such as a built-in hard disk.

Note that various processing described in the present specification is not only executed in time series according to the description, and may be executed in parallel or individually according to a processing ability of an apparatus for executing the processing or as necessary. Furthermore, in the present specification, the system is a logical group configuration of a plurality of devices, and the devices of the configuration are not limited to being housed in the same casing.

### INDUSTRIAL APPLICABILITY

As described above, according to the configuration of one embodiment of the present invention, the configuration in which the driver's biological information is input and which evaluates the wakefulness degree of the driver is realized.

Specifically, for example, a data processing unit that receives the driver's biological information and evaluates the wakefulness degree of the driver is included. The data processing unit analyzes a behavior of at least one of eyeballs or pupils of the driver and evaluates the driver's wakefulness degree by applying the behavior analysis result and a wakefulness state evaluation dictionary, which has been generated in advance, specific for the driver. The data processing unit evaluates the wakefulness degree of the driver by using the wakefulness state evaluation dictionary specific for the driver generated as a result of learning processing based on log data of the driver's biological information. The data processing unit further executes processing for estimating a return time until the driver can start safety manual driving.

Note that, by executing the processing according to the present invention, the data acquired by the observation is analyzed in the medium and long term, and continuous monitoring for the manual driving return request is performed in a self-completed manner when the automatic driving is used. This analysis information can be used at the same time as high-sensitivity mental health care monitoring data used to capture the precursor of the autonomic nerves diseases or the like and is expected to be used to prevent the disorder from being serious.

With this configuration, the configuration that receives the driver's biological information and evaluates the wakefulness degree of the driver is realized.

### REFERENCE SIGNS LIST

- 10: Automobile
- 11: Data processing unit
- 12: Driver biological information acquisition unit
- 13: Driver operation information acquisition unit
- 14: Environment information acquisition unit
- 15: Communication unit
- 16: Notification unit
- 20: Driver
- 30: Server
- 100: Moving device
- 101: Input unit
- 102: Data acquisition unit
- 103: Communication unit
- 104: In-vehicle device
- 105: Output control unit
- 106: Output unit
- 107: Driving system control unit
- 108: Driving system
- 109: Body system control unit
- 110: Body system
- 111: Storage unit
- 112: Automatic driving control unit
- 121: Communication network
- 131: Detection unit
- 132: Self-position estimation unit
- 133: Situation analysis unit
- 134: Planning unit
- 135: Operation control unit
- 141: Vehicle exterior information detection unit
- 142: In-vehicle information detection unit
- 143: Vehicle state detection unit
- 151: Map analysis unit
- 152: Traffic rule recognition unit
- 153: Situation recognition unit
- 154: Situation prediction unit
- 155: Safety determination unit
- 161: Route planning unit
- 162: Action planning unit
- 163: Operation planning unit
- 171: Emergency avoidance unit
- 172: Acceleration and deceleration control unit
- 173: Direction control unit
- 300: Driver's state information acquisition and analysis unit
- 501: CPU
- 502: ROM
- 503: RAM
- 504: Bus
- 505: Input/output interface
- 506: Input unit
- 507: Output unit
- 508: Storage unit
- 509: Communication unit
- 510: Drive
- 511: Removable medium
- 521: Sensor
- 522: Driving unit

## Claims

1. An information processing apparatus comprising:
a data processing unit (11) configured to receive driver's biological information and evaluate a wakefulness degree of a driver, wherein
the data processing unit (11)
is configured to analyze at least one of behaviors of an eyeball or a pupil of the driver and is configured to evaluate the wakefulness degree of the driver by applying the behavior analysis result and a wakefulness state evaluation dictionary that is specific for the driver and has been generated in advance, wherein the data processing unit (11) is configured to evaluate the wakefulness degree of the driver by using the wakefulness state evaluation dictionary specific for the driver that is generated as a result of learning processing based on log data of the driver's biological information, wherein
the data processing unit (11)
includes a learning processing unit that is configured to execute learning processing by analyzing a log obtained by monitoring processing for acquiring the driver's biological information, to evaluate the wakefulness degree of the driver, and to generate the wakefulness state evaluation dictionary specific for the driver, **characterized in that**
the learning processing unit
is configured to execute learning processing that acquires and uses teacher data at the normal time that is driver's state information when it is possible to normally start manual driving and teacher data at the abnormal time that is driver's state information when it is not possible to normally start manual driving on a basis of the operation information of the driver at the time of return from automatic driving to manual driving.

2. The information processing apparatus according to claim 1, wherein the driver includes a driver in a moving device that performs automatic driving and a driver that is completely separated from a driving operation or performs only a partial operation.

3. The information processing apparatus according to claim 1 or 2, wherein
the data processing unit (11)
is configured to evaluate the wakefulness degree of the driver by analyzing at least one of behaviors including saccade, microsaccade, drift, or fixation of the eyeballs.

4. The information processing apparatus according to any preceding claim, wherein the wakefulness state evaluation dictionary has a configuration that is configuered to store data used to calculate the wakefulness degree of the driver on a basis of a plurality of pieces of biological information that is able to be acquired from the driver.

5. The information processing apparatus according to any preceding claim, wherein
the data processing unit (11)
is configured to acquire the biological information and operation information of the driver and to evaluate the wakefulness degree of the driver on a basis of the acquired biological information and operation information of the driver.

6. The information processing apparatus according to any preceding claim, wherein
the data processing unit (11)
is configured to evaluate the wakefulness degree of the driver and to executes processing for estimating a return time before the driver is able to start safety manual driving.

7. The information processing apparatus according to any preceding claim, wherein
the data processing unit (11)
is configured to performs at least one of evaluation of the wakefulness degree of the driver by using medium-and-long-term data of the driver's state information including the biological information of the driver acquired from the driver or the calculation of a perceptual transmission index of the driver and/or on a basis of calculated difference data obtained by calculating a difference between the driver's state information including current biological information of the driver acquired from the driver and medium-and-long-term data of the acquired driver's state information or calculation of the perceptual transmission index of the driver, wherein
the perceptual transmission index is configured to indicate a mental state.

8. A moving device (10) comprising:
a biological information acquisition unit (12) configured to acquire biological information of a driver of the moving device; and
an information processing apparatus as claimed in claim 1.

9. The moving device (10) according to claim 8, wherein the driver includes a driver in the moving device that performs automatic driving and a driver that is completely separated from a driving operation or performs only a partial operation.

10. The moving device (10) according to claim 8 or 9, wherein
the data processing unit (11)
is configured to evaluate the wakefulness degree of the driver by analyzing at least one of behaviors including saccade, microsaccade, drift, or fixation of the eyeballs and/or by using the wakefulness state evaluation dictionary specific for the driver that is generated as a result of learning processing based on log data of the driver's biological information.

11. An information processing method executed by an information processing apparatus, wherein
the information processing apparatus includes a data processing unit (11) that is configured to receive driver's biological information and to evaluate a wakefulness degree of a driver, and
the data processing unit (11)
is configured to analyze at least one of behaviors of an eyeball or a pupil of the driver and to evaluate the wakefulness degree of the driver by applying the behavior analysis result and a wakefulness state evaluation dictionary that is specific for the driver and has been generated in advance, wherein
the data processing unit (11) is configured to evaluate the wakefulness degree of the driver by using the wakefulness state evaluation dictionary specific for the driver that is generated as a result of learning processing based on log data of the driver's biological information, wherein
the data processing unit (11)
includes a learning processing unit that is configured to execute learning processing by analyzing a log obtained by monitoring processing for acquiring the driver's biological information, to evaluate the wakefulness degree of the driver, and to generate the wakefulness state evaluation dictionary specific for the driver, **characterized in that**
the learning processing unit
is configured to execute learning processing that acquires and uses teacher data at the normal time that is driver's state information when it is possible to normally start manual driving and teacher data at the abnormal time that is driver's state information when it is not possible to normally start manual driving on a basis of the operation information of the driver at the time of return from automatic driving to manual driving.

12. An information processing method executed by a moving device, comprising:
a step of acquiring biological information of a driver of the moving device by a biological information acquisition unit (12); and
an information processing method as claimed in claim 11.

13. A computer program comprising program code means for causing a computer to carry out the steps of the method as claimed in claim 11 when said computer program is carried out on the computer.

## Patentansprüche

1. Informationsverarbeitungseinrichtung, umfassend:
eine Datenverarbeitungseinheit (11), die konfiguriert ist, um biologische Informationen eines Fahrers zu empfangen und einen Wachheitsgrad eines Fahrers zu bewerten, wobei
die Datenverarbeitungseinheit (11)
konfiguriert ist, um mindestens eine der Verhaltensweisen eines Augapfels oder einer Pupille des Fahrers zu analysieren, und konfiguriert ist, um den Wachheitsgrad des Fahrers durch Anwenden des Verhaltensanalyseergebnisses und eines Wachheitszustandsbewertungswörterbuchs, das für den Fahrer spezifisch ist und im Voraus generiert wird, zu bewerten, wobei die Datenverarbeitungseinheit (11) konfiguriert ist, um den Wachheitsgrad des Fahrers unter Verwendung des für den Fahrer spezifischen Wachheitszustandsbewertungswörterbuchs zu bewerten, das als Ergebnis einer Lernverarbeitung basierend auf Protokolldaten der biologischen Informationen des Fahrers generiert wird, wobei
die Datenverarbeitungseinheit (11)
eine lernende Verarbeitungseinheit einschließt, die konfiguriert ist, um Lernverarbeitung durch Analysieren eines Protokolls auszuführen, das durch Überwachungsverarbeitung zum Erfassen der biologischen Informationen des Fahrers erhalten wird, um den Wachheitsgrad des Fahrers zu bewerten und das für den Fahrer spezifische Wachheitszustandsbewertungswörterbuch zu generieren, **dadurch gekennzeichnet, dass**
die lernende Verarbeitungseinheit
konfiguriert ist, um einen Lernprozess auszuführen, der zum normalen Zeitpunkt Lehrerdaten, d. h. Informationen zum Zustand des Fahrers, wenn ein normaler Start des manuellen Fahrens möglich ist, und zum anormalen Zeitpunkt Lehrerdaten, d. h. Informationen zum Zustand des Fahrers, wenn ein normaler Start des manuellen Fahrens nicht möglich ist, auf Grundlage der Betriebsinformationen des Fahrers zum Zeitpunkt der Rückkehr vom automatischen Fahren zum manuellen Fahren erfasst und verwendet.

2. Informationsverarbeitungseinrichtung nach Anspruch 1, wobei der Fahrer einen Fahrer in einer sich bewegenden Vorrichtung, die automatisches Fahren durchführt, und einen Fahrer, der vollständig von einem Fahrvorgang getrennt ist oder nur einen Teilvorgang durchführt, einschließt.

3. Informationsverarbeitungseinrichtung nach Anspruch 1 oder 2, wobei
die Datenverarbeitungseinheit (11)
konfiguriert ist, um den Wachheitsgrad des Fahrers durch Analysieren von mindestens einer der Verhaltensweisen einschließend Sakkade, Mikrosakkade, Drift oder Fixierung der Augäpfel zu bewerten.

4. Informationsverarbeitungseinrichtung nach einem der vorstehenden Ansprüche, wobei das Wachheitszustandsbewertungswörterbuch eine Konfiguration aufweist, die konfiguriert ist, um Daten zu speichern, die zum Berechnen des Wachheitsgrads des Fahrers auf der Grundlage einer Vielzahl von biologischen Informationen verwendet werden, die vom Fahrer erfasst werden können.

5. Informationsverarbeitungseinrichtung nach einem der vorstehenden Ansprüche, wobei
die Datenverarbeitungseinheit (11)
konfiguriert ist, um die biologischen Informationen und Betriebsinformationen des Fahrers zu erfassen und den Wachheitsgrad des Fahrers auf Grundlage der erfassten biologischen Informationen und Betriebsinformationen des Fahrers zu bewerten.

6. Informationsverarbeitungseinrichtung nach einem der vorstehenden Ansprüche, wobei
die Datenverarbeitungseinheit (11)
konfiguriert ist, um den Wachheitsgrad des Fahrers zu bewerten und eine Verarbeitung zum Schätzen einer Rückkehrzeit auszuführen, bevor der Fahrer mit dem sicheren manuellen Fahren beginnen kann.

7. Informationsverarbeitungseinrichtung nach einem der vorstehenden Ansprüche, wobei
die Datenverarbeitungseinheit (11)
konfiguriert ist, um mindestens eine der Bewertung des Wachheitsgrads des Fahrers unter Verwendung von mittel- und langfristigen Daten der Zustandsinformationen des Fahrers, einschließlich der vom Fahrer erfassten biologischen Informationen des Fahrers, oder der Berechnung eines perzeptuellen Übertragungsindex des Fahrers und/oder auf der Grundlage berechneter Differenzdaten durchzuführen, die durch Berechnen einer Differenz zwischen den Zustandsinformationen des Fahrers, einschließlich aktueller biologischer Informationen des Fahrers, die vom Fahrer erfasst werden, und mittel- und langfristigen Daten der erfassten Zustandsinformationen des Fahrers, oder der Berechnung des perzeptuellen Übertragungsindex des Fahrers erhalten werden, wobei
der Wahrnehmungsübertragungsindex konfiguriert ist, um einen mentalen Zustand anzugeben.

8. Sich bewegende Vorrichtung (10), umfassend:
eine Einheit (12) zum Erfassen biologischer Informationen, die konfiguriert ist, um biologische Informationen eines Fahrers der sich bewegenden Vorrichtung zu erfassen; und
eine Informationsverarbeitungseinrichtung, wie in Anspruch 1 beansprucht.

9. Sich bewegende Vorrichtung (10) nach Anspruch 8, wobei der Fahrer einen Fahrer in einer sich bewegenden Vorrichtung, die automatisches Fahren durchführt, und einen Fahrer, der vollständig von einem Fahrvorgang getrennt ist oder nur einen Teilvorgang durchführt, einschließt.

10. Sich bewegende Vorrichtung (10) nach Anspruch 8 oder 9, wobei
die Datenverarbeitungseinheit (11)
konfiguriert ist, um den Wachheitsgrad des Fahrers durch Analysieren von mindestens einer der Verhaltensweisen einschließend Sakkade, Mikrosakkade, Drift oder Fixierung der Augäpfel, und/oder unter Verwendung des für den Fahrer spezifischen Wachheitszustandsbewertungswörterbuchs, das als Ergebnis eines Lernprozesses auf Grundlage von Protokolldaten der biologischen Informationen des Fahrers generiert wird, zu bewerten.

11. Informationsverarbeitungsverfahren, das durch eine Informationsverarbeitungseinrichtung ausgeführt wird, wobei
die Informationsverarbeitungseinrichtung eine Datenverarbeitungseinheit (11) einschließt, die konfiguriert ist, um biologische Informationen des Fahrers zu empfangen und einen Wachheitsgrad eines Fahrers zu bewerten, und
die Datenverarbeitungseinheit (11)
konfiguriert ist, um mindestens eine der Verhaltensweisen eines Augapfels oder einer Pupille des Fahrers zu analysieren und den Wachheitsgrad des Fahrers durch Anwenden des Verhaltensanalyseergebnisses und eines Wachheitszustandsbewertungswörterbuchs, das für den Fahrer spezifisch ist und im Voraus generiert wird, zu bewerten, wobei
die Datenverarbeitungseinheit (11) konfiguriert ist, um den Wachheitsgrad des Fahrers unter Verwendung des für den Fahrer spezifischen Wachheitszustandsbewertungswörterbuchs, das als Ergebnis einer Lernverarbeitung auf der Grundlage von Protokolldaten der biologischen Informationen des Fahrers generiert wird, zu bewerten, wobei
die Datenverarbeitungseinheit (11)
eine lernende Verarbeitungseinheit einschließt, die konfiguriert ist, um Lernverarbeitung durch Analysieren eines Protokolls auszuführen, das durch Überwachungsverarbeitung zum Erfassen der biologischen Informationen des Fahrers erhalten wird, um den Wachheitsgrad des Fahrers zu bewerten und das für den Fahrer spezifische Wachheitszustandsbewertungswörterbuch zu generieren, **dadurch gekennzeichnet, dass**
die lernende Verarbeitungseinheit
konfiguriert ist, um einen Lernprozess auszuführen, der zum normalen Zeitpunkt Lehrerdaten, d. h. Informationen zum Zustand des Fahrers, wenn ein normaler Start des manuellen Fahrens möglich ist, und zum anormalen Zeitpunkt Lehrerdaten, d. h. Informationen zum Zustand des Fahrers, wenn ein normaler Start des manuellen Fahrens nicht möglich ist, auf Grundlage der Betriebsinformationen des Fahrers zum Zeitpunkt der Rückkehr vom automatischen Fahren zum manuellen Fahren erfasst und verwendet.

12. Informationsverarbeitungsverfahren, das durch eine sich bewegende Vorrichtung ausgeführt wird, umfassend:
einen Schritt zum Erfassen biologischer Informationen eines Fahrers der sich bewegenden Vorrichtung durch eine Einheit zum Erfassen biologischer Informationen (12); und
ein Informationsverarbeitungsverfahren wie in Anspruch 11 beansprucht.

13. Computerprogramm, umfassend Programmcodemittel zum Veranlassen eines Computers, die Schritte des Verfahrens wie in Anspruch 11 beansprucht auszuführen, wenn das Computerprogramm auf einem Computer ausgeführt wird.

## Revendications

1. Appareil de traitement d'informations comprenant :
une unité de traitement de données (11) configurée pour recevoir les informations biologiques du conducteur et évaluer un degré d'éveil d'un conducteur, dans lequel
l'unité de traitement de données (11)
est configurée pour analyser au moins l'un des comportements d'un globe oculaire ou d'une pupille du conducteur et est configurée pour évaluer le degré d'éveil du conducteur en appliquant le résultat de l'analyse du comportement et un dictionnaire d'évaluation de l'état d'éveil qui est spécifique au conducteur et qui a été généré à l'avance, dans lequel l'unité de traitement de données (11) est configurée pour évaluer le degré d'éveil du conducteur en utilisant le dictionnaire d'évaluation de l'état d'éveil spécifique au conducteur qui est généré à la suite d'un traitement d'apprentissage basé sur des données de journal des informations biologiques du conducteur, dans lequel
l'unité de traitement de données (11)
comporte une unité de traitement d'apprentissage qui est configurée pour exécuter un traitement d'apprentissage en analysant un journal obtenu par la surveillance du traitement destiné à acquérir les informations biologiques du conducteur, à évaluer le degré d'éveil du conducteur et à générer le dictionnaire d'évaluation de l'état d'éveil spécifique au conducteur, **caractérisé en ce que**
l'unité de traitement d'apprentissage
est configurée pour exécuter un traitement d'apprentissage qui acquiert et utilise des données d'enseignement au moment normal, qui sont les informations d'état du conducteur lorsqu'il est possible de commencer normalement la conduite manuelle, et des données d'enseignement au moment anormal, qui sont les informations d'état du conducteur lorsqu'il n'est pas possible de commencer normalement la conduite manuelle, sur une base des informations de fonctionnement du conducteur au moment du retour de la conduite automatique à la conduite manuelle.

2. Appareil de traitement d'informations selon la revendication 1, dans lequel le conducteur comporte un conducteur dans un dispositif mobile qui effectue une conduite automatique et un conducteur qui est complètement séparé d'une opération de conduite ou qui n'effectue qu'une opération partielle.

3. Appareil de traitement d'informations selon la revendication 1 ou 2, dans lequel
l'unité de traitement de données (11)
est configurée pour évaluer le degré d'éveil du conducteur en analysant au moins l'un des comportements comportant : saccade, microsaccade, dérive ou fixation des globes oculaires.

4. Appareil de traitement d'informations selon l'une quelconque revendication précédente, dans lequel le dictionnaire d'évaluation de l'état d'éveil a une configuration qui est configurée pour stocker des données utilisées pour calculer le degré d'éveil du conducteur sur une base d'une pluralité de parties d'informations biologiques qui peuvent être acquises auprès du conducteur.

5. Appareil de traitement d'informations selon l'une quelconque revendication précédente, dans lequel
l'unité de traitement de données (11)
est configurée pour acquérir les informations biologiques et les informations de fonctionnement du conducteur et pour évaluer le degré d'éveil du conducteur sur une base des informations biologiques acquises et des informations de fonctionnement du conducteur.

6. Appareil de traitement d'informations selon l'une quelconque revendication précédente, dans lequel
l'unité de traitement de données (11)
est configurée pour évaluer le degré d'éveil du conducteur et pour exécuter un traitement destiné à estimer un temps de retour avant que le conducteur ne soit en mesure de commencer la conduite manuelle en sécurité.

7. Appareil de traitement d'informations selon l'une quelconque revendication précédente, dans lequel
l'unité de traitement de données (11)
est configurée pour effectuer au moins l'un parmi l'évaluation du degré d'éveil du conducteur en utilisant des données à moyen et long terme des informations d'état du conducteur, y compris les informations biologiques du conducteur acquises auprès du conducteur, ou le calcul d'un indice de transmission perceptive du conducteur et/ou sur une base de données de différence calculée obtenues par le calcul d'une différence entre les informations d'état du conducteur, y compris les informations biologiques actuelles du conducteur acquises auprès du conducteur, et des données à moyen et long terme des informations d'état du conducteur acquises ou le calcul de l'indice de transmission perceptive du conducteur, dans lequel
l'indice de transmission perceptive est configuré pour indiquer un état mental.

8. Dispositif mobile (10) comprenant :
une unité d'acquisition d'informations biologiques (12) configurée pour acquérir des informations biologiques sur un conducteur du dispositif mobile ; et
un système de traitement d'informations selon la revendication 1.

9. Dispositif mobile (10) selon la revendication 8, dans lequel le conducteur comporte un conducteur dans le dispositif mobile qui effectue une conduite automatique et un conducteur qui est complètement séparé d'une opération de conduite ou qui n'effectue qu'une opération partielle.

10. Dispositif mobile (10) selon la revendication 8 ou 9, dans lequel
l'unité de traitement de données (11)
est configurée pour évaluer le degré d'éveil du conducteur en analysant au moins l'un parmi les comportements comportant : saccade, microsaccade, dérive ou fixation des globes oculaires et/ou en utilisant le dictionnaire d'évaluation de l'état d'éveil spécifique au conducteur qui est généré à la suite d'un traitement d'apprentissage basé sur des données de journal des informations biologiques du conducteur.

11. Procédé de traitement d'informations exécuté par un dispositif de traitement d'informations, dans lequel
l'appareil de traitement d'informations comporte une unité de traitement de données (11) qui est configurée pour recevoir les informations biologiques du conducteur et pour évaluer un degré d'éveil d'un conducteur et
l'unité de traitement de données (11)
est configurée pour analyser au moins l'un des comportements d'un globe oculaire ou d'une pupille du conducteur et pour évaluer le degré d'éveil du conducteur en appliquant le résultat de l'analyse du comportement et un dictionnaire d'évaluation de l'état d'éveil qui est spécifique au conducteur et qui a été généré à l'avance, dans lequel
l'unité de traitement de données (11) est configurée pour évaluer le degré d'éveil du conducteur en utilisant le dictionnaire d'évaluation de l'état d'éveil spécifique au conducteur qui est généré à la suite d'un traitement d'apprentissage basé sur les données de journal des informations biologiques du conducteur, dans lequel
l'unité de traitement de données (11)
comporte une unité de traitement d'apprentissage qui est configurée pour exécuter un traitement d'apprentissage en analysant un journal obtenu par la surveillance du traitement destiné à acquérir les informations biologiques du conducteur, à évaluer le degré d'éveil du conducteur et à générer le dictionnaire d'évaluation de l'état d'éveil spécifique au conducteur, **caractérisé en ce que**
l'unité de traitement d'apprentissage
est configurée pour exécuter un traitement d'apprentissage qui acquiert et utilise des données d'enseignement au moment normal, qui sont les informations d'état du conducteur lorsqu'il est possible de commencer normalement la conduite manuelle, et des données d'enseignement au moment anormal, qui sont les informations d'état du conducteur lorsqu'il n'est pas possible de commencer normalement la conduite manuelle, sur une base des informations de fonctionnement du conducteur au moment du retour de la conduite automatique à la conduite manuelle.

12. Procédé de traitement d'informations exécuté par un dispositif mobile, comprenant :
une étape consistant à acquérir des informations biologiques d'un conducteur du dispositif mobile par une unité d'acquisition d'informations biologiques (12) ; et
un système de traitement d'informations selon la revendication 11.

13. Programme informatique comprenant un moyen de code de programme destiné à amener un ordinateur à mettre en œuvre les étapes du procédé selon la revendication 11 lorsque ledit programme informatique est mis en œuvre sur un ordinateur.
